# EUROPÄISCHE PATENTSCHRIFT

(11) **EP 0 987 246 B1**
(45) Veröffentlichungstag und Bekanntmachung des Hinweises auf die Patenterteilung: **08.09.2004**
(21) Anmeldenummer: 99123926.0
(22) Anmeldetag: 26.06.1995
(51) Int. Cl.: C07C 69/013, C07C 69/96, A01N 35/06, A01N 37/02, C07C 49/747, C07C 309/65, C07C 271/32, C07C 59/84, C07C 329/06

(54) **2-Arylcyclopentan-1,3-dion-Derivate**
Derivatives of 2-arylcyclopentan-1,3-dion
Dérivés de la 2-arylecyclopentane-1,3-dione

(30) Priorität: 07.07.1994 DE 4423943; 30.01.1995 DE 19502815; 23.05.1995 DE 19518962
(43) Veröffentlichungstag der Anmeldung: 22.03.2000
(62) Teilanmeldung aus: 95924938.4
(73) Patentinhaber: Bayer CropScience AG, 40789 Monheim (DE)
(72) Erfinder: Fischer, Reiner, Dr., 40789 Monheim (DE); Dumas, Jacques, Dr., Orange, CT 06477 (US); Bretschneider, Thomas, Dr., 53797 Lohmar (DE); Erdelen, Christoph, Dr., 42799 Leichlingen (DE); Wachendorff-Neumann, Ulrike, Dr., 56566 Neuwied (DE); Santel, Hans-Joachim, Dr., Leawood, KS 66209 (US); Dollinger, Markus, Dr., Overland Park, Kansas 66213 (US); Turberg, Andreas, 42781 Haan (DE); Mencke, Norbert, Dr., 51381 Leverkusen (DE)

(56) Entgegenhaltungen:
- US-A- 4 283 348
- US-A- 4 436 666
- US-A- 4 551 547

## Beschreibung

Die vorliegende Erfindung betrifft neue 2-Aryl-3-hydroxy-Δ²-cyclopenten-1-on-Derivate, Verfahren zu ihrer Herstellung und ihre Verwendung als Herbizide und Schädlingsbekämpfungsmittel.

Es ist bekannt, daß bestimmte substituierte 2-Arylcyclopentandione herbizide und akarizide Eigenschaften besitzen (vgl. z.B. US 4 283 348; 4 338 122; 4 436 666; 4 526 723; 4 551 547 und 4 632 698). Außerdem sind ähnlich substituierte Verbindungen bekannt; 3-Hydroxy-5,5-dimethyl-2-phenylcyclopent-2-en-1-on aus der Publikation Micklefield et al., Tetrahedron, (1992), 7519-26 sowie der Naturstoff Involutin (-)-cis-5-(3,4-dihydroxyphenyl)-3,4-dihydroxy-2-(4-hydroxyphenyl)-cyclopent-2-enone aus der Publikation Edwards et al., J. Chem. Soc. S, (1967), 405-9. Eine insektizide oder akarizide Wirkung wird nicht beschrieben.

Es wurden nun neue Verbindungen der Formel (I) 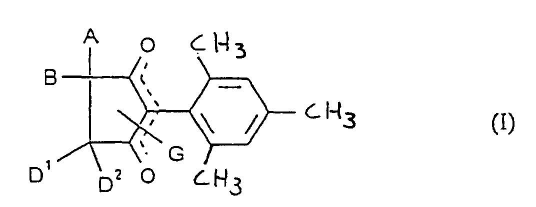 gefunden,
in welcher
- A und B: unabhängig voneinander für jeweils gegebenenfalls einfach oder mehrfach gleich oder verschieden durch Halogen substituiertes Alkyl,
- D¹ und D²: unabhängig voneinander für Wasserstoff, Halogen, gegebenenfalls durch Halogen substituiertes Alkyl oder gegebenenfalls substituiertes Phenyl stehen,
- G: für Wasserstoff (a) oder für eine der Gruppen

-CO-R¹ (b)

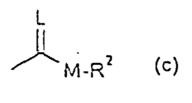

-SO₂-R³ (d)

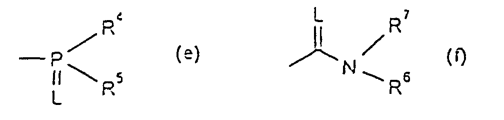

oder

E (g)
- E: für ein Metallionäquivalent oder ein Ammoniumion steht,
- L: für Sauerstoff oder Schwefel steht,
- M: für Sauerstoff oder Schwefel steht,
- R¹: für jeweils gegebenenfalls einfach oder mehrfach gleich oder verschieden durch Halogen substituiertes Alkyl, Alkenyl, Alkoxyalkyl, Alkylthioalkyl, Polyalkoxyalkyl oder gegebenenfalls substituiertes, gesättigtes oder ungesättigtes Cycloalkyl steht, das durch mindestens ein Heteroatom unterbrochen sein kann oder für jeweils gegebenenfalls substituiertes Phenyl, Phenylalkyl, Hetaryl, Phenoxyalkyl oder Hetaryloxyalkyl steht,
- R²: für jeweils gegebenenfalls einfach oder mehrfach gleich oder verschieden durch Halogen substituiertes Alkyl, Alkenyl, Alkoxyalkyl, Polyalkoxyalkyl oder jeweils gegebenenfalls substituiertes Cycloalkyl, Phenyl oder Benzyl steht,
- R³: für jeweils gegebenenfalls einfach oder mehrfach, gleich oder verschieden durch Halogen substituiertes Alkyl oder für jeweils gegebenenfalls substituiertes Phenyl oder Phenylalkyl steht,
- R⁴ und R⁵: unabhängig voneinander für jeweils gegebenenfalls einfach oder mehrfach gleich oder verschieden durch Halogen substituiertes Alkyl, Alkoxy, Alkylamino, Alkenylamino, Dialkylamino, Dialkenylamino, Alkylthio, Alkenylthio, Alkinylthio, Cycloalkylthio oder für jeweils gegebenenfalls substituiertes Phenyl, Phenoxy oder Phenylthio stehen,
- R⁶ und R⁷: unabhängig voneinander für Wasserstoff, jeweils gegebenenfalls einfach oder mehrfach gleich oder verschieden durch Halogen substituiertes Alkyl, Alkenyl, Alkoxy, Alkoxyalkyl, für gegebenenfalls substituiertes Phenyl oder für gegebenenfalls substituiertes Benzyl stehen,
oder
- R⁶ und R⁷: zusammen für einen gegebenenfalls durch Sauerstoff oder Schwefel unterbrochenen Alkylenrest stehen,
sowie die enantiomerenreinen Formen von Verbindungen der Formel (I).

Die Verbindungen der Formel (I) können in Abhängigkeit von der Stellung des Substituenten G in den zwei isomeren Formen der Formeln I-A und I-B vorliegen, 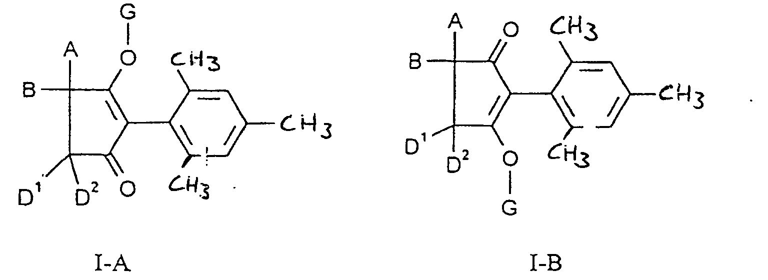 was durch die gestrichelte Linie in der Formel (I) zum Ausdruck gebracht werden soll.

Die Verbindungen der Formeln I-A und I-B können sowohl als Gemische als auch in Form ihrer reinen Isomeren vorliegen. Gemische der Verbindungen der Formeln I-A und I-B lassen sich gegebenenfalls durch physikalische Methoden trennen, beispielsweise durch chromatographische Methoden.

Aus Gründen der besseren Übersichtlichkeit wird im folgenden jeweils nur eines der möglichen Isomeren aufgeführt. Das schließt nicht aus, daß die Verbindungen gegebenenfalls in Form der Isomerengemische oder in der jeweils anderen isomeren Form vorliegen können.

Unter Einbeziehung der verschiedenen Bedeutungen (a), (b), (c), (d), (e), (f) und (g) der Gruppe G ergeben sich folgende hauptsächlichen Strukturen (Ia) bis (Ig): 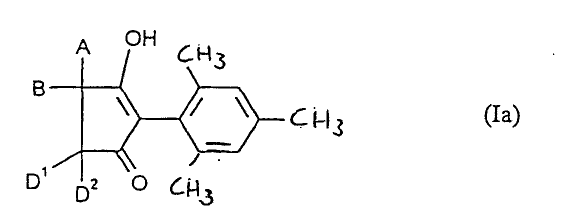 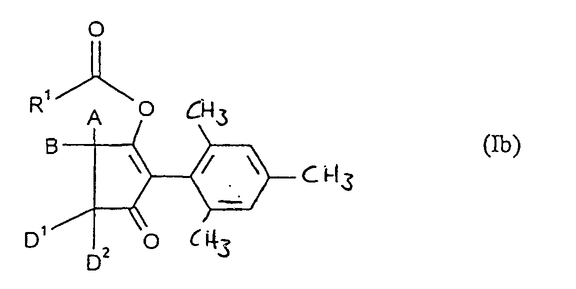 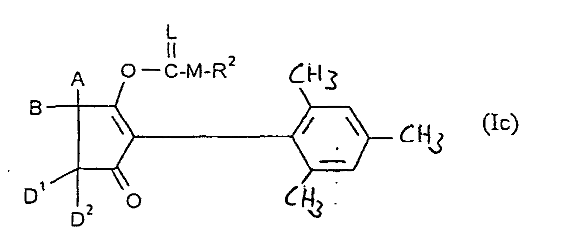 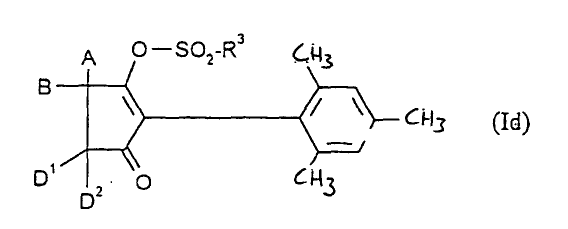 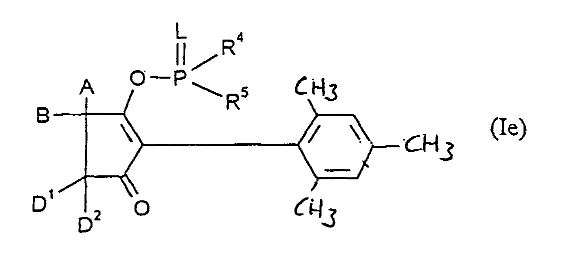 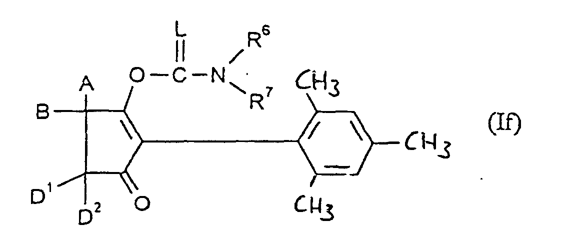 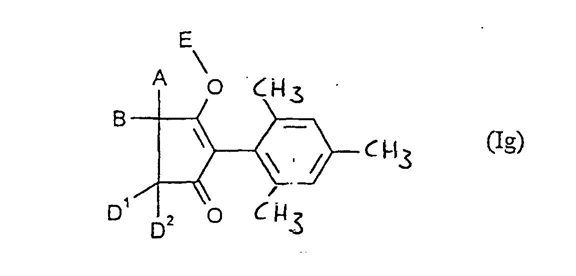 worin
- A, B, D¹, D², E, L, M, R¹, R², R³, R⁴, R⁵, R⁶ und R⁷: die oben angegebenen Bedeutungen besitzen.
Weiterhin wurde gefunden,
(A)
   daß man Verbindungen der Formel (Ia) 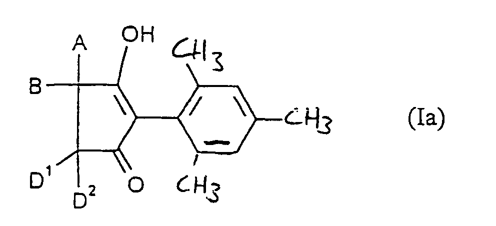 in welcher
   - A, B, D¹, D²: die oben angegebene Bedeutung haben,
   erhält, wenn man
   Ketocarbonsäureester der Formel (II) 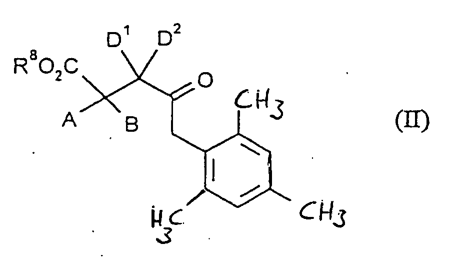 in welcher
   - A, B, D¹, D²: die oben angegebene Bedeutung haben,
   und
   - R⁸: für Alkyl (insbesondere C₁-C₈-Alkyl) steht,
   gegebenenfalls in Gegenwart eines Verdünnungsmittels und in Gegenwart einer Base intramolekular cyclisiert und
(B)
   daß man Verbindungen der Formel (Ib) 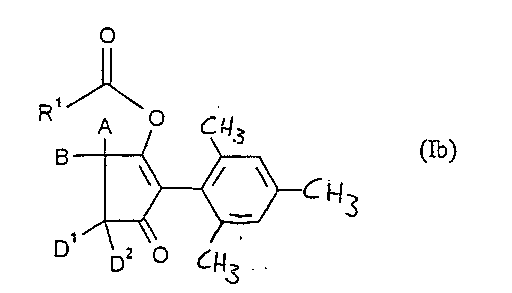 in welcher
   - A, B, D¹, D², R¹: die oben angegebene Bedeutung haben,
   erhält, wenn man Verbindungen der Formel (Ia) 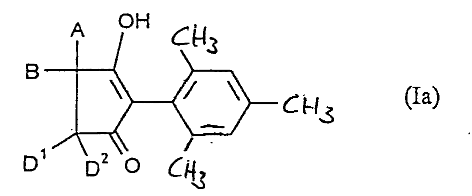 in welcher
   - A, B, D¹, D²,: die oben angegebene Bedeutung haben,

   α) mit Säurehalogeniden der Formel (III) 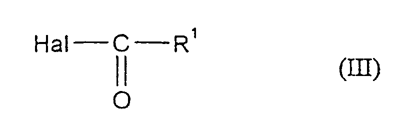 in welcher
      - R¹: die oben angegebene Bedeutung hat
      und
      - Hal: für Halogen (insbesondere Chlor oder Brom) steht,
      gegebenenfalls in Gegenwart eines Verdünnungsmittels und gegebenenfalls in Gegenwart eines Säurebindemittels umsetzt
      oder
   β) mit Carbonsäureanhydriden der Formel (IV)

      R¹-CO-O-CO-R¹ (IV)

      in welcher
      - R¹: die oben angegebene Bedeutung hat,
      gegebenenfalls in Gegenwart eines Verdünnungsmittels und gegebenenfalls in Gegenwart eines Säurebindemittels,
   umsetzt, und
(C)
   daß man Verbindungen der Formel (Ic) 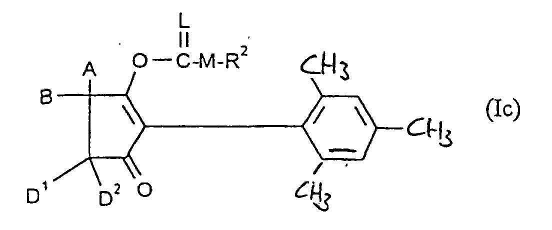 in welcher
   - A, B, D¹, D², R²: die oben angegebene Bedeutung haben,
   - L: für Sauerstoff und
   - M: für Sauerstoff oder Schwefel steht,
   erhält, wenn man Verbindungen der Formel (Ia) 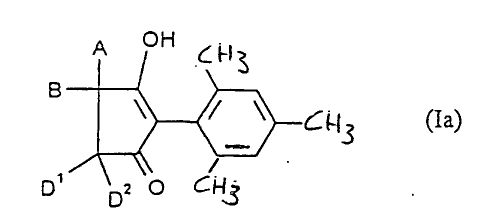 in welcher
   - A, B, D¹, D²: die oben angegebene Bedeutung haben,
   mit einem Chlorameisensäureester oder Chlorameisensäurethiolester der Formel (V)

   R²-M-CO-Cl (V)

   in welcher
   - R² und M: die oben angegebene Bedeutung haben,
   gegebenenfalls in Gegenwart eines Verdünnungsmittels und gegebenenfalls in Gegenwart eines Säurebindemittels umsetzt, und
(D)
   daß man Verbindungen der Formel (Ic) 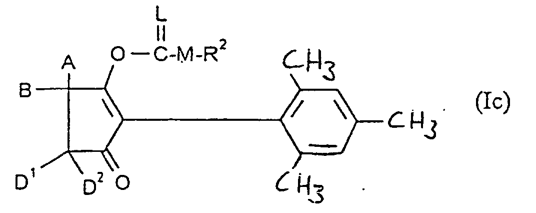 in welcher
   - A, B, D¹, D², R²: die oben angegebene Bedeutung haben,
   - L: für Schwefel und

   - M: für Sauerstoff oder Schwefel steht,
   erhält, wenn man Verbindungen der Formel (Ia) 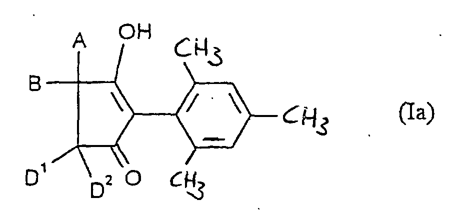 in welcher
   - A, B, D¹, D²,: die oben angegebene Bedeutung haben,

   α) mit einem Chlormonothioameisensäureester oder Chlordithioameisensäureester der Formel (VI) 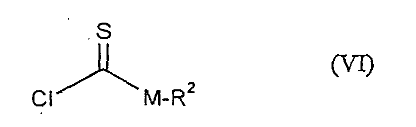 in welcher
      - M und R²: die oben angegebene Bedeutung haben
      gegebenenfalls in Gegenwart eines Verdünnungsmittels und gegebenenfalls in Gegenwart eines Säurebindemittels umsetzt, oder
   β) mit Schwefelkohlenstoff und anschließend mit Alkylhalogeniden der Formel (VII)

      R²-Hal (VII)
   in welcher
   - R²: die oben angegebene Bedeutung hat
   und
   - Hal: für Chlor, Brom oder Iod steht,
   gegebenenfalls in Gegenwart eines Verdünnungsmittels und gegebenenfalls in Gegenwart einer Base umsetzt und
B)
   daß man Verbindungen der Formel (Id) 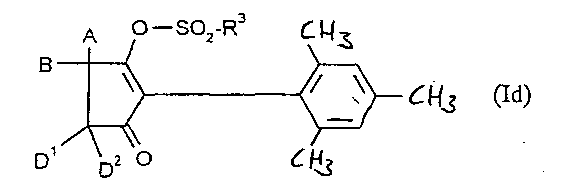 in welcher
   - A, B, D¹, D², R³: die oben angegebene Bedeutung haben,
   erhält, wenn man Verbindungen der Formel (Ia) 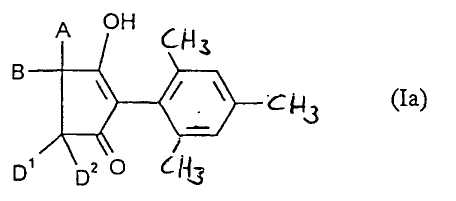 in welcher
   - A, B, D¹, D²,: die oben angegebene Bedeutung haben,
   mit Sulfonsäurechloriden der Formel (VIII)

   R³-SO₂-Cl (VIII)

   in welcher
   - R³: die oben angegebene Bedeutung hat,
   gegebenenfalls in Gegenwart eines Verdünnungsmittels und gegebenenfalls in Gegenwart eines Säurebindemittels umsetzt, und
(F)
   daß man Verbindungen der Formel (Ie) 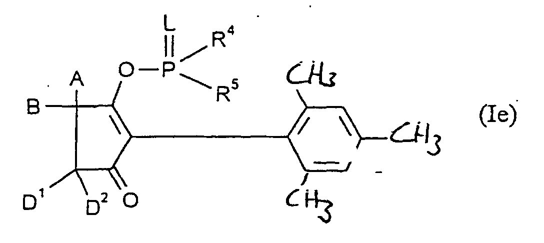 in welcher
   - A, B, D¹, D², L, R⁴, R⁵: die oben angegebene Bedeutung haben,
   erhält, wenn man Verbindungen der Formel (Ia) 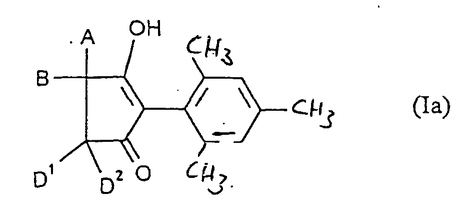 in welcher
   - A, B, D¹, D²,: die oben angegebene Bedeutung haben,
   mit Phosphorverbindungen der Formel (IX) 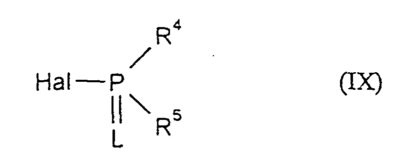 in welcher
   - L, R⁴ und R⁵: die oben angegebene Bedeutung haben und
   - Hal: für Halogen (insbesondere Chlor oder Brom) steht,
   gegebenenfalls in Gegenwart eines Verdünnungsmittels und gegebenenfalls in Gegenwart eines Säurebindemittels umsetzt, und
(G)
   daß man Verbindungen der Formel (If) 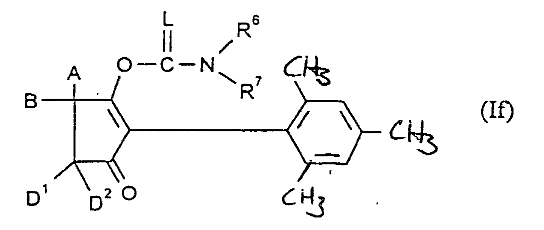 in welcher
   - A, B, D¹, D², L, R⁶, R⁷: die oben angegebene Bedeutung haben,
   erhält, wenn man Verbindungen der Formel (Ia) 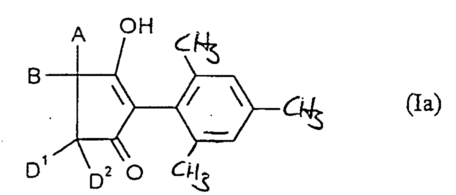 in welcher
   - A, B, D¹, D²: die oben angegebene Bedeutung haben,

   α) mit Isocyanaten oder Isothiocyanaten der Formel (X)

      R⁶-N=C=L (X)

      in welcher
      - R⁶ und L: die oben angegebene Bedeutung haben,
      gegebenenfalls in Gegenwart eines Verdünnungsmittels und gegebenenfalls in Gegenwart eines Katalysators umsetzt, oder
   β) mit Carbamidsäurechloriden oder Thiocarbamidsäurechloriden der Formel (XI) 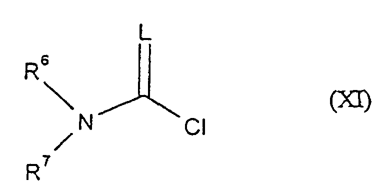
   in welcher
   - L, R⁶ und R⁷: die oben angegebene Bedeutung haben,
   gegebenenfalls in Gegenwart eines Verdünnungsmittels und gegebenenfalls in Gegenwart eines Säurebindemittels umsetzt, und

(H)
   daß man Verbindungen der Formel (Ig) 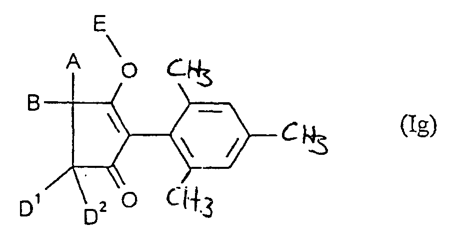 in welcher
   - D¹, D², A, B: die oben angegebene Bedeutung haben,
   und
   - E: für ein Metallionäquivalent (insbesondere für ein Alkali- oder Erdalkalimetall wie Lithium, Natrium, Kalium, Magnesium oder Calcium) oder ein Ammoniumion steht,
   erhält, wenn man Verbindungen der Formel (Ia) 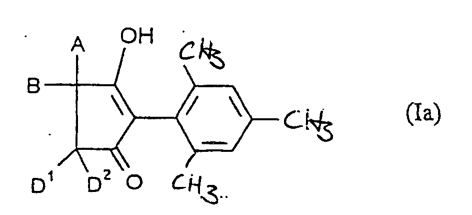 in welcher
   - A, B, D¹, D²: die oben angegebene Bedeutung haben,
   mit Metallverbindungen der Formel (XII) oder Aminen der Formel (XIII)

   MeR⁹ₜ (XII)

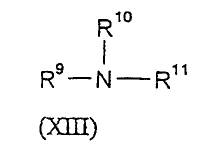

   in welchen
   - Me: für ein- oder zweiwertige Metallionen (wie oben für E genannt) und
   - t: für die Zahl 1 oder 2 steht und
   - R⁹, R¹⁰ und R¹¹: unabhängig voneinander für Wasserstoff, Alkyl (bevorzugt C₁-C₈-Alkyl), Alkoxy (bevorzugt C₁-C₈-Alkoxy) oder Hydroxy stehen,
   gegebenenfalls in Gegenwart eines Verdünnungsmittels, umsetzt.
   Weiterhin wurde gefunden, daß sich die neuen Verbindungen der Formel (I) durch hervorragende herbizide, akarizide und insektizide Wirkungen auszeichnen.

Die erfindungsgemäßen Verbindungen sind durch die Formel (I) allgemein definiert.

Bevorzugte Substituenten bzw. Bereiche der in den oben und nachstehend erwähnten Formeln aufgeführten Reste werden im folgenden erläutert.
- A und B: stehen unabhängig voneinander bevorzugt für jeweils gegebenenfalls einfach oder mehrfach, gleich oder verschieden durch Halogen substituiertes C₁-C₁₂-Alkyl,

- D¹ und D²: stehen unabhängig voneinander bevorzugt für Wasserstoff, Halogen, gegebenenfalls einfach oder mehrfach, gleich oder verschieden durch Halogen substituiertes C₁-C₆-Alkyl oder für gegebenenfalls einfach oder mehrfach, gleich oder verschieden durch Halogen, C₁-C₆-Alkyl, C₁-C₆-Alkoxy, Nitro, C₁-C₆-Halogenalkyl oder C₁-C₆-Halogenalkoxy substituiertes Phenyl ,
- G: steht bevorzugt für Wasserstoff (a) oder für eine der Gruppen

-CO-R¹ (b)

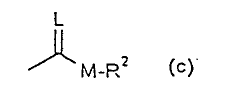

- SO₂-R³ (d)

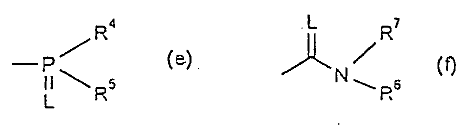

oder

E (g)

in welchen
E für ein Metallionäquivalent oder ein Ammoniumion steht,
L für Sauerstoff oder Schwefel steht,
M für Sauerstoff oder Schwefel steht,
- R¹: steht bevorzugt für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, Poly-C₁-C₈-alkoxy-C₂-C₈-alkyl oder gegebenenfalls einfach oder mehrfach, gleich oder verschieden durch Halogen, C₁-C₆-Alkyl oder C₁-C₆-Alkoxy substituiertes, gesättigtes oder ungesättigtes Cycloalkyl mit 3 bis 8 Ringatomen, in welchem mindestens eine Methylen-gruppe durch ein Sauerstoff- und/oder Schwefelatom ersetzt sein kann,
für gegebenenfalls einfach oder mehrfach, gleich oder verschieden durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl,
für gegebenenfalls einfach oder mehrfach, gleich oder verschieden durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach oder mehrfach, gleich oder verschieden durch Halogen oder C₁-C₆-Alkyl substituiertes fünf- oder sechsgliedriges Hetaryl,
für gegebenenfalls einfach oder mehrfach, gleich oder verschieden durch Halogen oder C₁-C₆-Alkyl substituiertes Phenoxy-C₁-C₆-alkyl oder
für gegebenenfalls einfach oder mehrfach, gleich oder verschieden durch Halogen, Amino oder C₁-C₆-Alkyl substituiertes fünf- oder sechsgliedriges Hetaryloxy-C₁-C₆-alkyl ,
- R²: steht bevorzugt für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl oder für gegebenenfalls einfach oder mehrfach, gleich oder verschieden durch Halogen, C₁-C₆-Alkyl oder C₁-C₆-Alkoxy substituiertes C₃-C₈-Cycloalkyl oder
für jeweils gegebenenfalls einfach oder mehrfach, gleich oder verschieden durch Halogen, Nitro, Cyano, C₁-C₆-Alkyl, C₁-C₆-Alkoxy oder C₁-C₆-Halogenalkyl substituiertes Phenyl oder Benzyl,
- R³: steht bevorzugt für jeweils gegebenenfalls einfach oder mehrfach, gleich oder verschieden durch Halogen substituiertes C₁-C₁₂-Alkyl oder für jeweils gegebenenfalls einfach oder mehrfach, gleich oder verschieden durch Halogen, C₁-C₆-Alkyl, C₁-C₆-Alkoxy C₁-C₃-Halogenalkyl, C₁-C₃-Halogenalkoxy, Cyano oder Nitro substituiertes Phenyl oder Phenyl-C₁-C₄-alkyl
- R⁴ und R⁵: stehen unabhängig voneinander bevorzugt für jeweils gegebenenfalls einfach oder mehrfach, gleich oder verschieden durch Halogen substituiertes C₁-C₈-Alkyl, C₁-C₈-Alkoxy, C₁-C₈-Alkylamino, C₃-C₈-Alkenylamino, Di-(C₁-C₈-alkyl)-amino, Di-(C₃-C₈-alkenyl)-amino, C₁-C₈-Alkylthio, C₃-C₅-Alkenylthio, C₃-C₅-Alkinylthio, C₃-C₇-Cycloalkylthio oder für jeweils gegebenenfalls einfach oder mehrfach, gleich oder verschieden durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio und
- R⁶: und R⁷ stehen unabhängig voneinander bevorzugt für Wasserstoff oder für jeweils gegebenenfalls einfach oder mehrfach, gleich oder verschieden durch Halogen substituiertes C₁-C₁₀-Alkyl, C₁-C₁₀-Alkoxy, C₃-C₈-Alkenyl oder C₁-C₁₀-Alkoxy-C₁-C₁₀-alkyl, für gegebenenfalls einfach oder mehrfach, gleich oder verschieden durch Halogen, C₁-C₃-Halogenalkyl, C₁-C₈-Alkyl oder C₁-C₈-Alkoxy substituiertes Phenyl, für gegebenenfalls einfach oder mehrfach, gleich oder verschieden durch Halogen, C₁-C₈-Alkyl, C₁-C₈-Halogenalkyl oder C₁-C₈-Alkoxy substituiertes Benzyl oder zusammen für einen C₂-C₆-Alkylenrest in welchem eine Methylengruppe durch Sauerstoff oder Schwefel ersetzt sein kann.
- A und B: stehen unabhängig voneinander besonders bevorzugt für jeweils gegebenenfalls einfach bis fünffach, gleich oder verschieden durch Fluor oder Chlor substituiertes C₁-C₁₀-Alkyl,
- D¹ und D²: stehen unabhängig voneinander besonders bevorzugt für Wasserstoff, Fluor, Chlor, gegebenenfalls einfach bis fünffach, gleich oder verschieden durch Fluor oder Chlor substituiertes C₁-C₄-Alkyl oder für gegebenenfalls einfach bis dreifach, gleich oder verschieden durch Fluor, Chlor, Brom, C₁-C₄-Alkyl, C₁-C₄-Alkoxy, Nitro, C₁-C₂-Halogenalkyl oder C₁-C₂-Halogenalkoxy substituiertes Phenyl,
- G: steht besonders bevorzugt für Wasserstoff (a) oder für eine der Gruppen

-CO-R¹ (b)

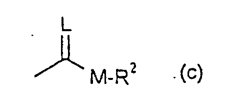

-SO₂-R³ (d)

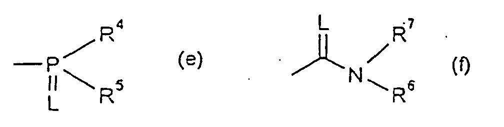

oder

E (g)

steht,
in welchen
E für ein Metallionäquivalent oder ein Ammoniumion steht,
L für Sauerstoff oder Schwefel steht,
M für Sauerstoff oder Schwefel steht,
- R¹ steht besonders bevorzugt: für jeweils gegebenenfalls einfach bis neunfach, gleich oder verschieden durch Halogen substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, Poly-C₁-C₆-alkoxy-C₂-C₆-alkyl oder gegebenenfalls einfach bis sechsfach, gleich oder verschieden durch Fluor, Chlor, C₁-C₄-Alkyl oder C₁-C₄-Alkoxy substituiertes, gesättigtes oder ungesättigtes Cycloalkyl mit 3 bis 7 Ringatomen, in welchem gegebenenfalls eine oder zwei Methylengruppen durch Sauerstoff- und/oder Schwefelatome ersetzt sind,
für gegebenenfalls einfach bis dreifach gleich oder verschieden durch Fluor, Chlor, Brom, Nitro, Cyano, C₁-C₄-Alkyl, C₁-C₄-Alkoxy, C₁-C₃-Halogenalkyl oder C₁-C₃-Halogenalkoxy substituiertes Phenyl,
für gegebenenfalls einfach bis dreifach, gleich oder verschieden durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach oder zweifach, gleich oder verschieden durch Fluor, Chlor oder C₁-C₆-Alkyl substituiertes Pyridyl, Thienyl, Furanyl; Pyrimidyl, Thiazolyl oder Pyrazolyl,
für gegebenenfalls einfach bis dreifach, gleich oder verschieden durch Fluor, Chlor, Brom oder C₁-C₄-Alkyl substituiertes Phenoxy-C₁-C₅-alkyl oder
für jeweils gegebenenfalls einfach oder zweifach, gleich oder verschieden durch Fluor, Chlor, Brom, Amino oder C₁-C₄-Alkyl substituiertes Pyridyloxy-, Pyrimidyloxy- oder Thiazolyloxy-C₁-C₅-alkyl,
- R²: steht besonders bevorzugt für jeweils gegebenenfalls einfach bis neunfach, gleich oder verschieden durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl, für gegebenenfalls einfach bis fünffach, gleich oder verschieden durch Fluor, Chlor, C₁-C₄-Alkyl oder C₁-C₄-Alkoxy substituiertes C₃-C₇-Cycloalkyl oder
für jeweils gegebenenfalls einfach bis dreifach, gleich oder verschieden durch Fluor, Chlor, Brom, Nitro, Cyano, C₁-C₄-Alkyl, C₁-C₃-Alkoxy oder C₁-C₃-Halogenalkyl substituiertes Phenyl oder Benzyl
- R³: steht besonders bevorzugt für jeweils gegebenenfalls einfach bis fünffach, gleich oder verschieden durch Fluor oder Chlor substituiertes C₁-C₈-Alkyl oder für jeweils gegebenenfalls einfach bis dreifach, gleich oder verschieden durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Phenyl-C₁-C₂-alkyl,
- R⁴ und R⁵: stehen unabhängig voneinander besonders bevorzugt für jeweils gegebenenfalls einfach bis fünffach, gleich oder verschieden durch Fluor oder Chlor substituiertes C₁-C₆-Alkyl, C₁-C₆-Alkoxy, C₁-C₆-Alkylamino, C₃-C₆-Alkenylamino, Di-(C₁-C₆-alkyl)-amino, Di-(C₃-C₆-alkenyl)-amino, C₁-C₆-Alkylthio, C₃-C₄-Alkenylthio, C₃-C₄-Alkinylthio, C₃-C₆-Cycloalkylthio oder für jeweils gegebenenfalls einfach bis dreifach, gleich oder verschieden durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio und
- R⁶ und R⁷: stehen unabhängig voneinander besonders bevorzugt für Wasserstoff oder für jeweils gegebenenfalls einfach bis fünffach, gleich oder verschieden durch Fluor oder Chlor substituiertes C₁-C₆-Alkyl, C₁-C₆-Alkoxy, C₃-C₆-Alkenyl oder C₁-C₆-Alkoxy-C₁-C₆-alkyl, für gegebenenfalls einfach bis dreifach, gleich oder verschieden durch Fluor, Chlor, C₁-C₃-Halogenalkyl, C₁-C₃-Alkyl oder C₁-C₃-Alkoxy substituiertes Phenyl, für gegebenenfalls einfach bis fünffach, gleich oder verschieden durch Fluor, Chlor, C₁-C₃-Alkyl, C₁-C₃-Halogenalkyl oder C₁-C₃-Alkoxy substituiertes Benzyl oder zusammen für einen C₄-C₆-Alkylenrest , in welchem eine Methylengruppe durch Sauerstoff oder Schwefel ersetzt sein kann.
- A und B: stehen unabhängig voneinander ganz besonders bevorzugt für jeweils gegebenenfalls einfach bis dreifach, gleich oder verschieden durch Fluor oder Chlor substituiertes C₁-C₈-Alkyl,
- D¹ und D²: stehen unabhängig voneinander ganze besonders bevorzugt für Wasserstoff, Fluor, Chlor, Methyl, Ethyl oder für gegebenenfalls einfach oder zweifach, gleich oder verschieden durch Fluor, Chlor, Methyl, Methoxy oder Trifluormethyl substituiertes Phenyl,
- G: steht ganz besonders bevorzugt für Wasserstoff (a) oder für eine der Gruppen

-CO-R¹ (b)

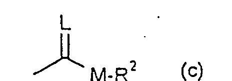

-SO₂-R³ (d)

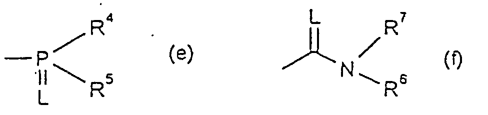

oder

E (g)

steht,
in welchen
E für ein Metallionäquivalent oder ein Ammoniumion steht,
L für Sauerstoff oder Schwefel steht,
M für Sauerstoff oder Schwefel steht,
- R¹: steht ganz besonders bevorzugt für jeweils gegebenenfalls einfach bis dreifach, gleich oder verschieden durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₂-C₄-alkyl oder gegebenenfalls einfach oder zweifach, gleich oder verschieden durch Fluor, Chlor, Methyl, Ethyl, Methoxy oder Ethoxy substituiertes Cycloalkyl mit 3 bis 6 Ringatomen, in welchem gegebenenfalls eine oder zwei Methylengruppen durch Sauerstoff- und/oder Schwefelatome ersetzt sind,
für gegebenenfalls einfach oder-zweifach, gleich oder verschieden durch Fluor, Chlor, Brom, Methyl, Ethyl, Propyl, i-Propyl, Methoxy, Ethoxy, Trifluormethyl, Trifluormethoxy, Cyano oder Nitro substituiertes Phenyl,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl oder Trifluormethoxy substituiertes Phenyl-C₁-C₃-alkyl, insbesondere Benzyl,
für jeweils gegebenenfalls einfach oder zweifach, gleich oder verschieden durch Fluor, Chlor, Brom, Methyl oder Ethyl substituiertes Pyridyl, Thienyl oder Furanyl,
für gegebenenfalls einfach oder zweifach, gleich oder verschieden durch Fluor, Chlor, Methyl oder Ethyl substituiertes PhenoxyC₁-C₄-alkyl oder
für jeweils gegebenenfalls einfach oder zweifach, gleich oder verschieden durch Fluor, Chlor, Amino, Methyl oder Ethyl substituiertes Pyridyloxy-C₁-C₃-alkyl, Pyrimidyloxy-C₁-C₃-alkyl oder Thiazolyloxy-C₁-C₃-alkyl ,
- R²: steht ganz besonders bevorzugt für jeweils gegebenenfalls einfach bis fünffach, gleich oder verschieden durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl, für gegebenenfalls einfach bis dreifach, gleich oder verschieden durch Fluor, Chlor, Methyl, Ethyl, Methoxy oder Ethoxy substituiertes C₃-C₆-Cycloalkyl,
oder für jeweils gegebenenfalls einfach oder zweifach, gleich oder verschieden durch Fluor, Chlor, Nitro, Cyano, Methyl, Ethyl, Propyl, i-Propyl, Methoxy, Ethoxy oder Trifluormethyl substituiertes Phenyl oder Benzyl ,
- R³: steht ganz besonders bevorzugt für gegebenenfalls einfach bis dreifach, gleich oder verschieden durch Fluor oder Chlor substituiertes C₁-C₆-Alkyl oder für jeweils gegebenenfalls einfach oder zweifach, gleich oder verschieden durch Fluor, Chlor, Brom, Methyl, Methoxy, Trifluormethyl, Trifluormethoxy, Cyano oder Nitro substituiertes Phenyl oder Benzyl,
- R⁴ und R⁵: stehen unabhängig voneinander ganz besonders bevorzugt für jeweils gegebenenfalls einfach bis dreifach, gleich oder verschieden durch Fluor oder Chlor substituiertes C₁-C₄-Alkyl, C₁-C₄-Alkoxy, C₁-C₄-Alkylamino, C₃-C₄-Alkenylamino, Di-(C₁-C₄-alkyl)-amino, Di-(C₃-C₄-alkenyl)-amino, C₁-C₄-Alkylthio, für jeweils gegebenenfalls einfach oder zweifach, gleich oder verschieden durch Fluor, Chlor, Brom, Nitro, Cyano, C₁-C₂-Alkoxy, C₁-C₂-Fluoralkoxy, C₁-C₂-Chloralkoxy, C₁-C₂-Alkylthio, C₁-C₂-Fluoralkylthio, C₁-C₂-Chloralkylthio oder C₁-C₃-Alkyl substituiertes Phenyl, Phenoxy oder Phenylthio und
- R⁶ und R⁷: stehen unabhängig voneinander ganz besonders bevorzugt für Wasserstoff oder für jeweils gegebenenfalls einfach bis dreifach, gleich oder verschieden durch Fluor oder Chlor substituiertes C₁-C₄-Alkyl, C₁-C₄-Alkoxy oder C₁-C₄-Alkoxy-C₁-C₄-alkyl, für gegebenenfalls einfach oder zweifach, gleich oder verschieden durch Fluor, Chlor, C₁-C₂-Halogenalkyl, C₁-C₂-Alkyl oder C₁-C₂-Alkoxy substituiertes Phenyl, für gegebenenfalls einfach oder zweifach, gleich oder verschieden durch Fluor, Chlor, Brom, C₁-C₂-Alkyl, C₁-C₂-Halogenalkyl oder C₁-C₂-Alkoxy substituiertes Benzyl oder zusammen für einen C₄-C₆-Alkylenrest , in welchem eine Methylengruppe durch Sauerstoff oder Schwefel ersetzt sein kann.

Dabei sind die enantiomerenreinen Formen von Verbindungen der Formel (I) jeweils eingeschlossen.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden. Sie gelten für die Endprodukte sowie für die Vor- und Zwischenprodukte entsprechend.

Erfindungsgemäß bevorzugt werden die Verbindungen der Formel (I), in welchen eine Kombination der vorstehend als bevorzugt (vorzugsweise) aufgeführten Bedeutungen vorliegt.

Erfindungsgemäß besonders bevorzugt werden die Verbindungen der Formel (I), in welchen eine Kombination der vorstehend als besonders bevorzugt aufgeführten Bedeutungen vorliegt.

Erfindungsgemäß ganz besonders bevorzugt werden die Verbindungen der Formel (I), in welchen eine Kombination der vorstehend als ganz besonders bevorzugt aufgeführten Bedeutungen vorliegt.

Im einzelnen seien außer den bei den Herstellungsbeispielen genannten Verbindungen die folgenden Verbindungen der Formel (Ia) genannt:

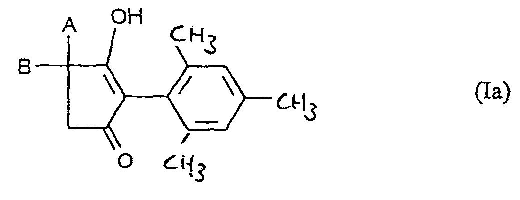

**Tabelle 1**

| A | B |
|---|---|
| -CH₃ | -CH₃ |
| -C₂H₅ | -CH₃ |
| -CH(CH₃)₂ | -CH₃ |
| -CH₂-CH(CH₃)₂ | -CH₃ |
| -C₂H₅ | -C₂H₅ |

Im einzelnen seien außer den bei den Herstellungsbeispielen genannten Verbindungen die folgenden Verbindungen der Formel (Ib) genannt (Tabelle 2):

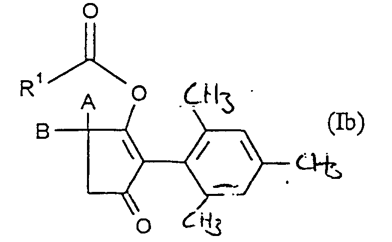

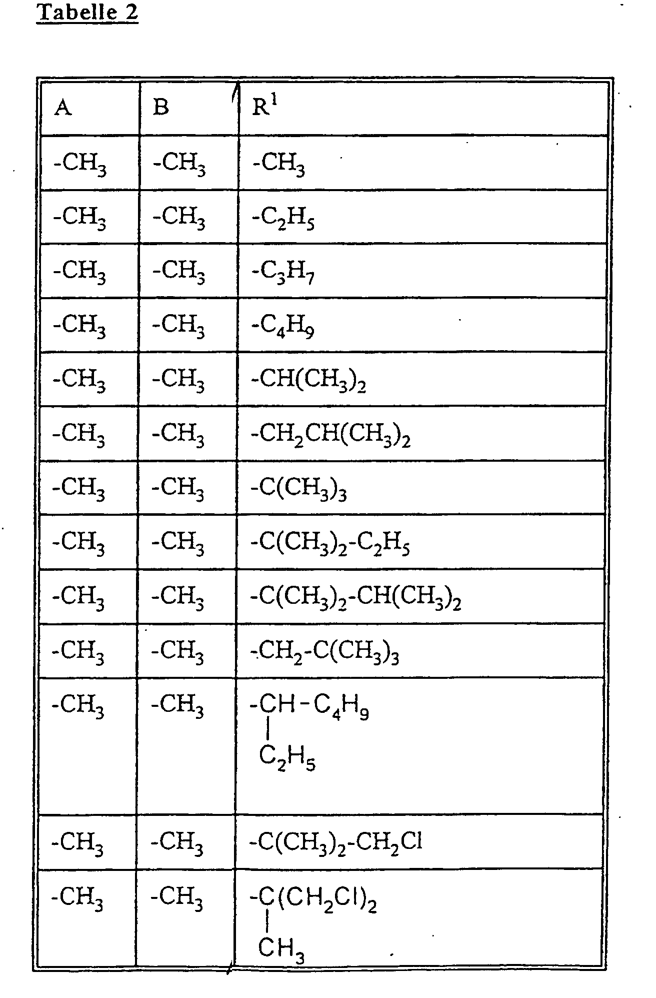

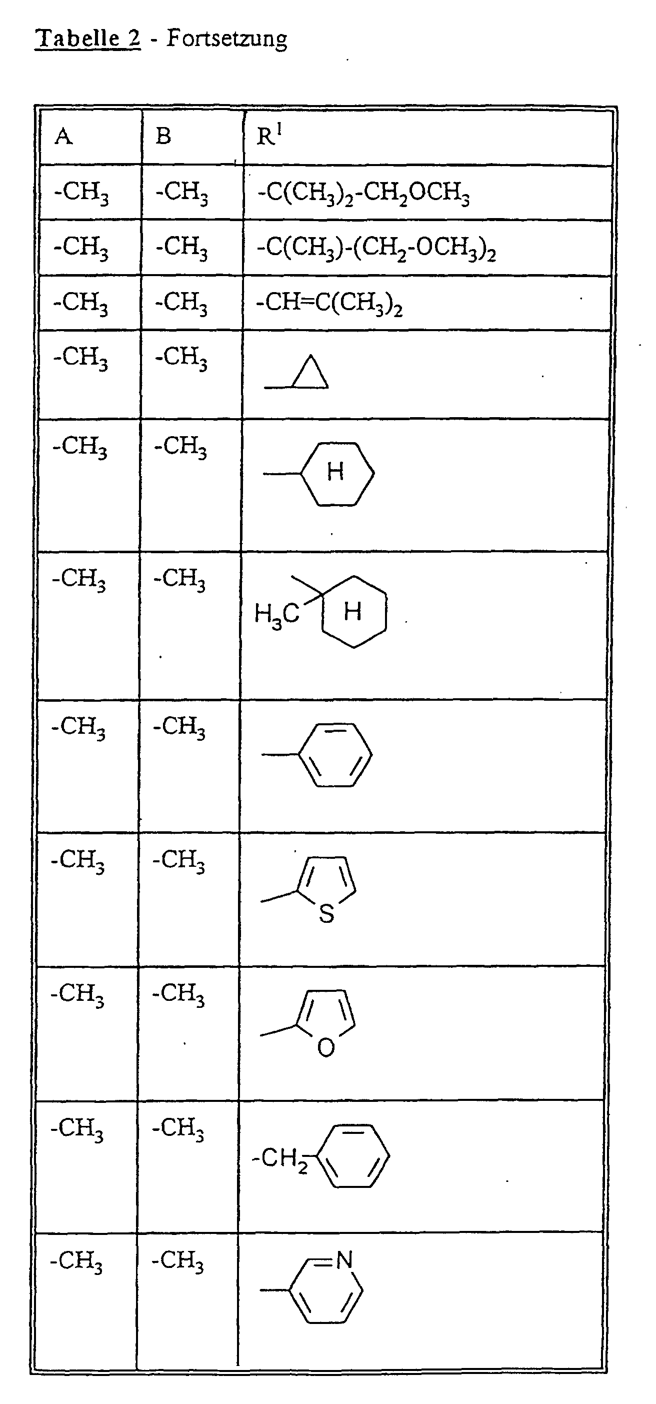

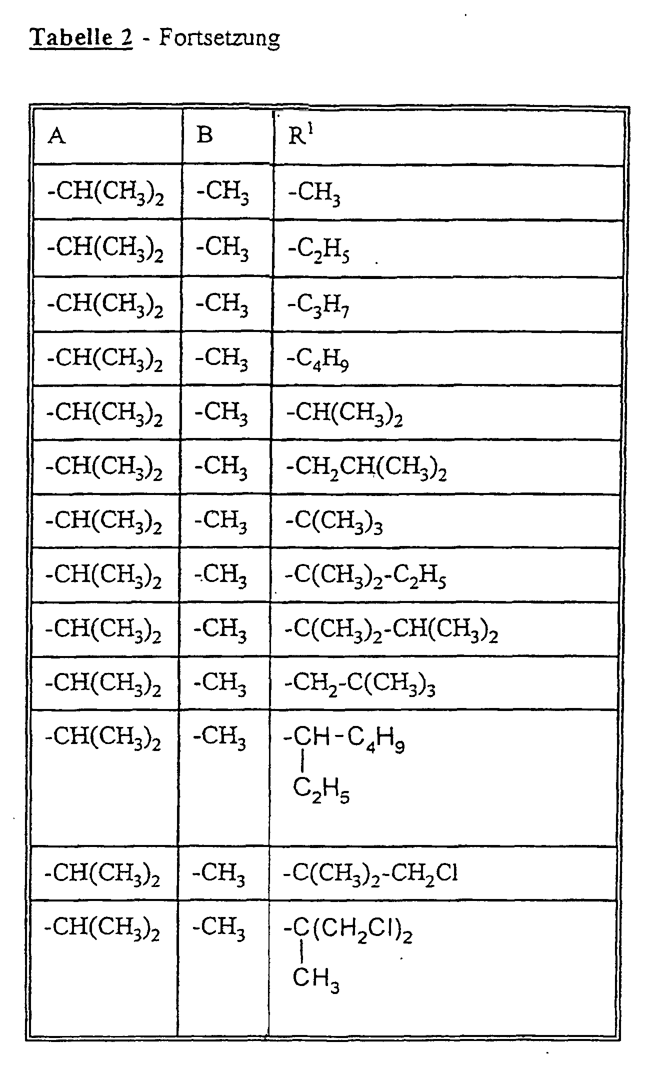

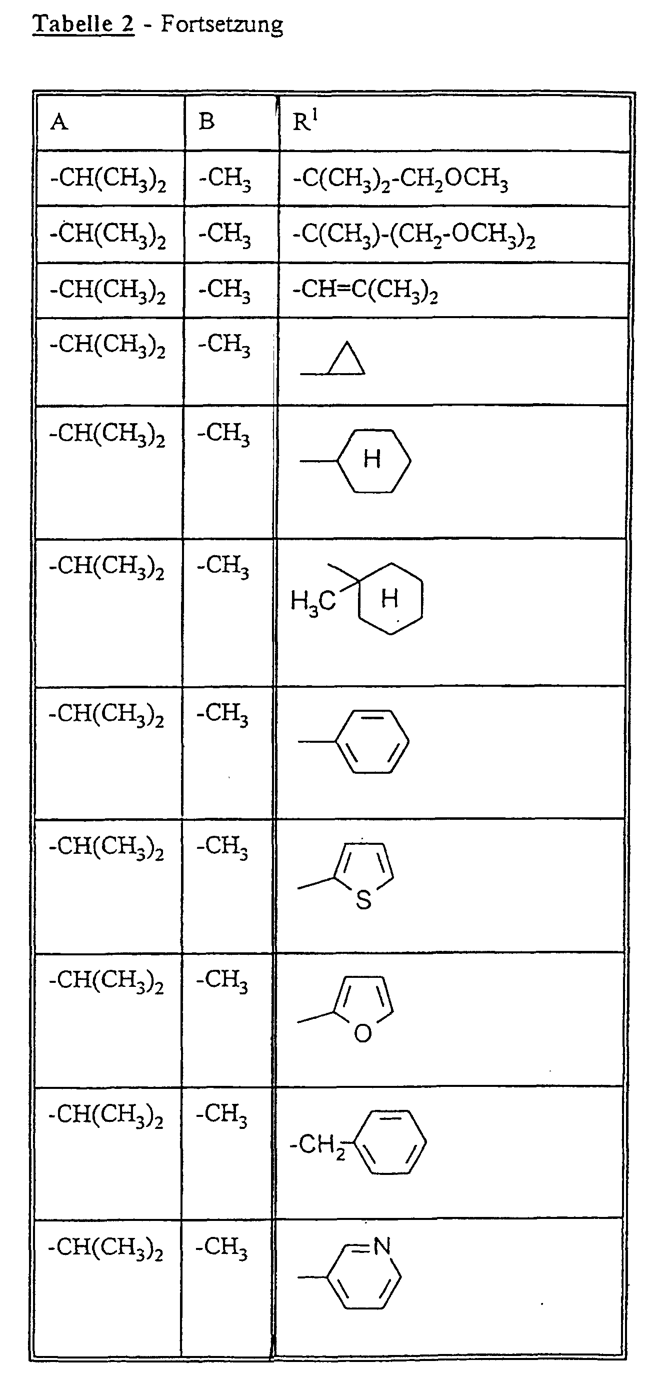

Im einzelnen seien außer den bei den Herstellungsbeispielen genannten Verbindungen die folgenden Verbindungen der Formel (Ic) genannt (Tabelle 5):

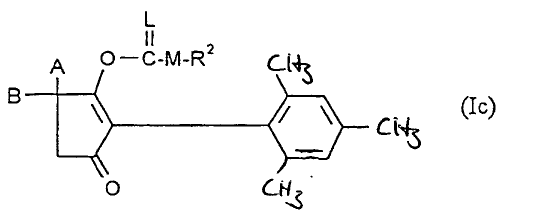

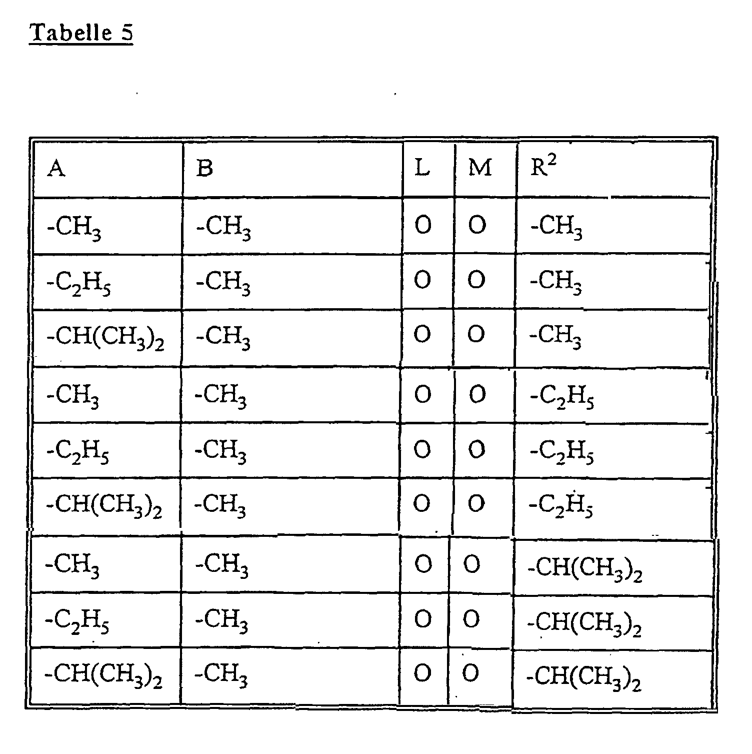

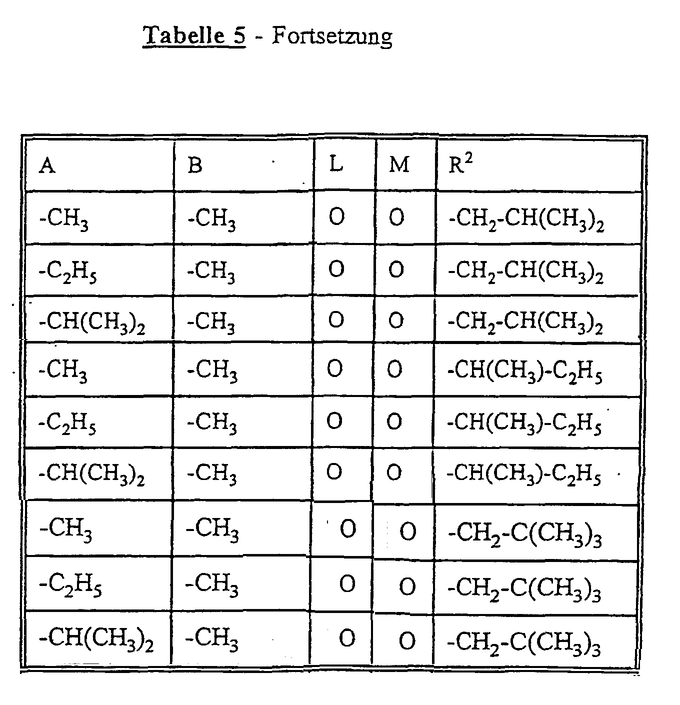

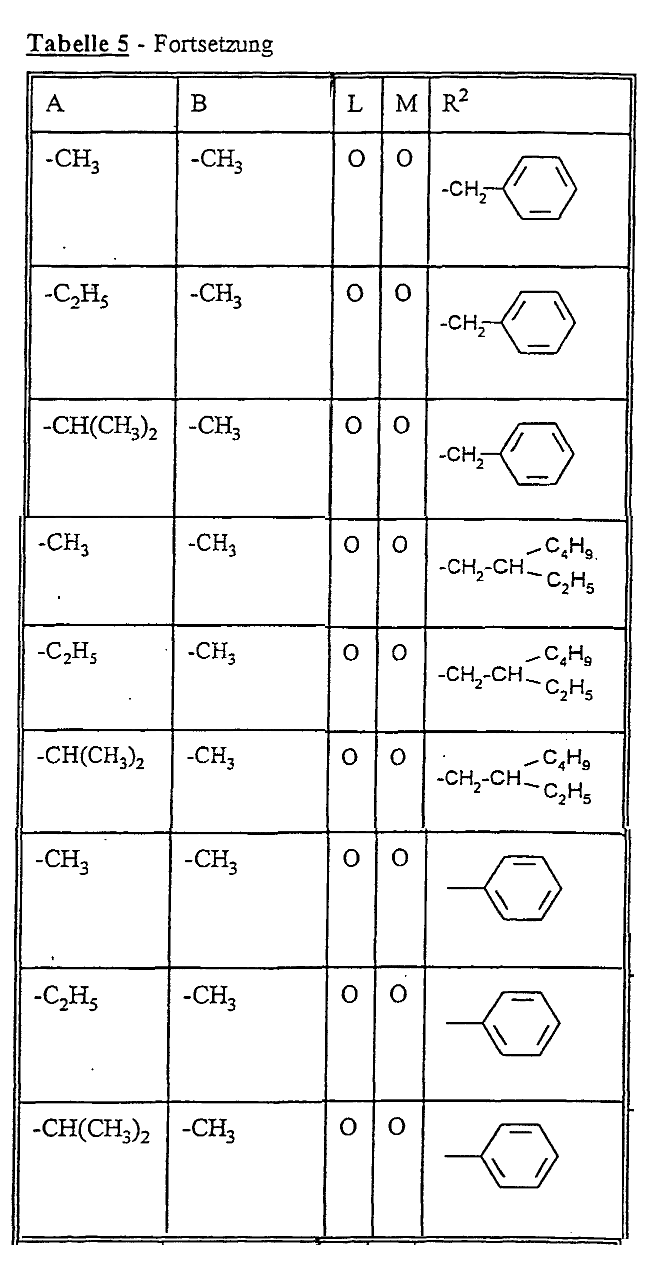

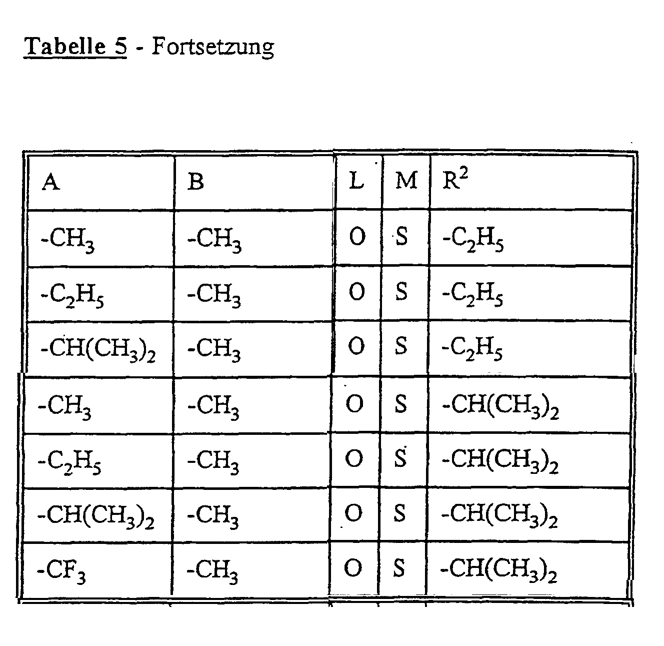

Im einzelnen seien außer den bei den Herstellungsbeispielen genannten Verbindungen die folgenden Verbindungen der Formel (Ig) genannt (Tabelle 11):

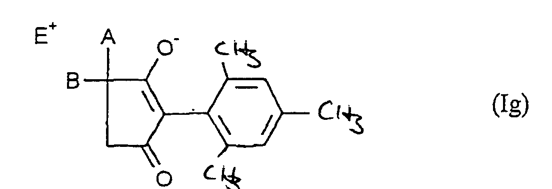

**Tabelle 11**

| A | B | E |
|---|---|---|
| - CH₃ | CH₃ | NH₄ |
| - CH₃ | -CH₃ | Na |
| - C₂H₅ | -CH₃ | Na |
| - CH(CH₃)₂ | -CH₃ | Na |
| - CF₃ | -CH₃ | Na |
| - CH₃ | -CH₃ | i-C₃H₇NH₃ |
| -C₂H₅ | -CH₃ | i-C₃H₇NH₃ |
| -CH(CH₃)₂ | -CH₃ | i-C₃H₇NH₃ |
| -CF₃ | -CH₃ | i-C₃H₇NH₃ |

Verwendet man gemäß Verfahren (A) 5-(2,6-Dichlorphenyl)-2,2-dimethyl-4-oxovaleriansäure-ethylester, so kann der Verlauf des erfindungsgemäßen Verfahrens durch folgendes Reaktionsschema wiedergegeben werden:

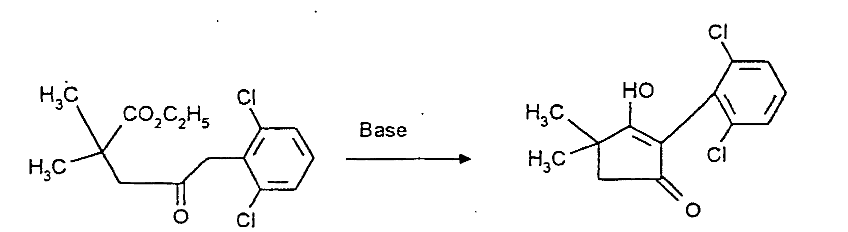

Verwendet man gemäß Verfahren (B) (Variante α) 2-(2,4,6-Trimethylphenyl)-3-hydroxy-4,4-dimethyl-Δ²-cyclopentenon und Pivaloylchlorid als Ausgangsstoff, so kann der Verlauf des erfindungsgemäßen Verfahrens durch folgendes Reaktionsschema wiedergegeben werden.

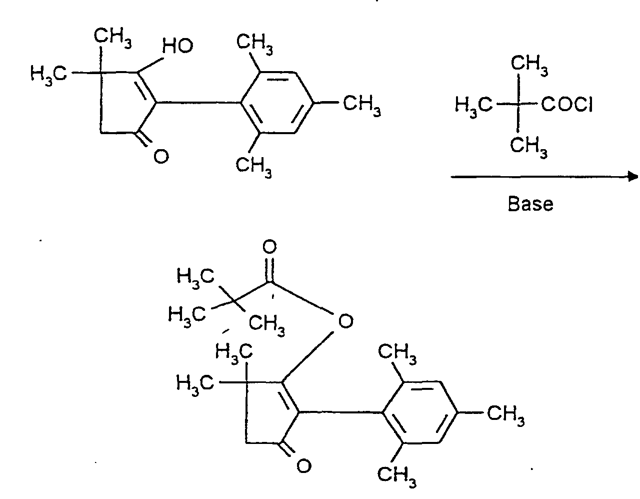

Verwendet man gemäß Verfahren (B) (Variante β) 2-(2,4-Dimethylphenyl)-3-hydroxy-4-methyl-4-phenyl-Δ²-cyclopentenon und Acetanhydrid als Ausgangsverbindungen, so kann der Verlauf des erfindungsgemäßen Verfahrens durch folgendes Reaktionsschema wiedergegeben werden.

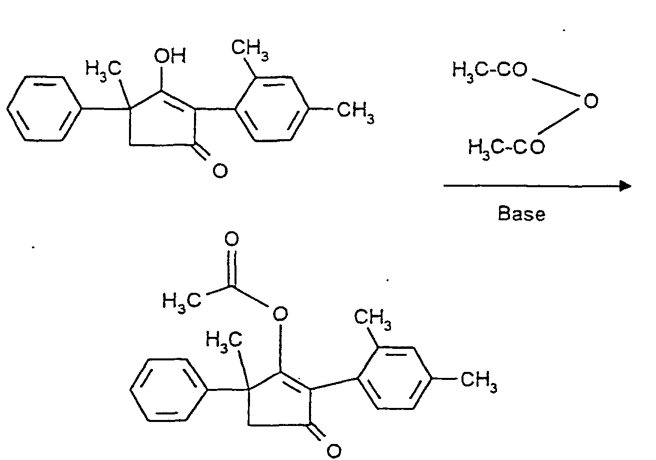

Verwendet man gemäß Verfahren (C) 2-(2,4-Dichlorphenyl)-3-hydroxy-4-isoproisopropyl-4-methyl-Δ²-cyclopentenon und Chlorameisensäureethoxyethylester als Ausgangsverbindungen, so kann der Verlauf des erfindungsgemäßen Verfahrens durch folgendes Reaktionsschema wiedergegeben werden.

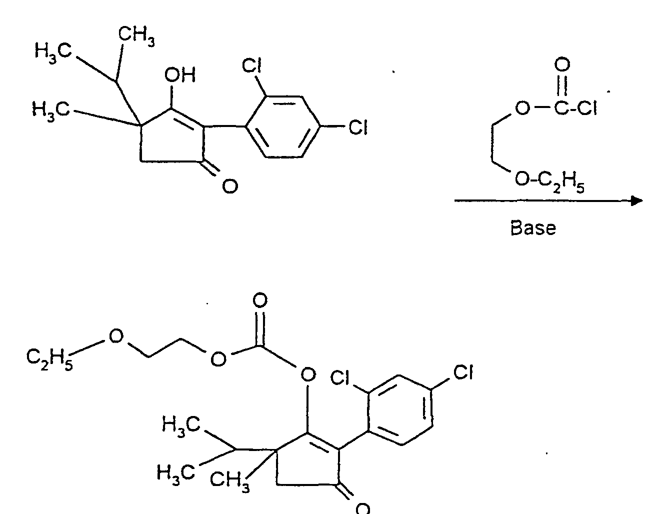

Verwendet man gemäß Verfahren (D_{α}) 2-(2,4,6-Trimethylphenyl)-3-hydroxy-4-ethyl-4-methyl-Δ²-cyclopentenon und Chlormonothioameisensäuremethylester als Ausgangsprodukte, so kann der Reaktionsverlauf wie folgt wiedergegeben werden:

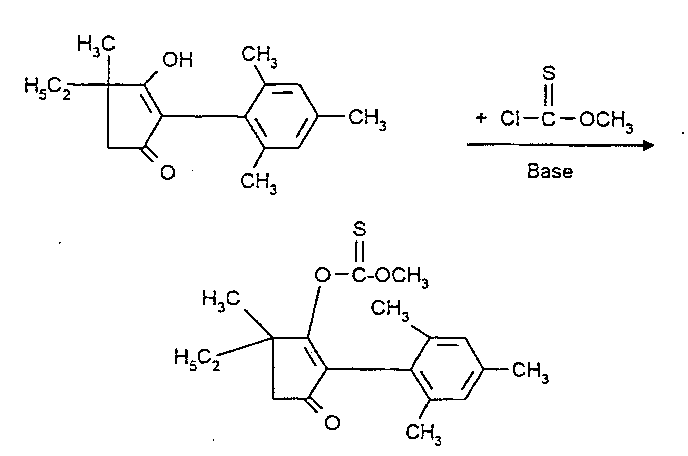

Verwendet man gemäß Verfahren (D_{β}) 2-(2,4,6-Trimethylphenyl)-3-hydroxy-4,4-pentamethylen-Δ²-cyclopentenon, Schwefelkohlenstoff und Methyliodid als Ausgangskomponenten, so kann der Reaktionsverlauf wie folgt wiedergegeben werden:

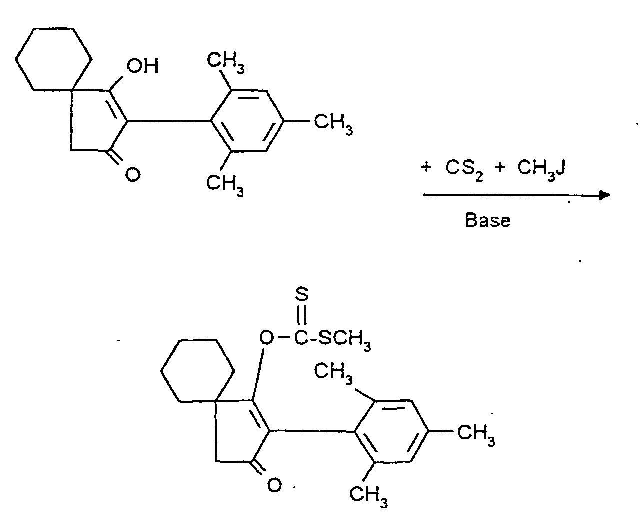

Verwendet man gemäß Verfahren (E) 2-(2,4,6-Trimethylphenyl)-3-hydroxy-4,4-(3-methoxy)-pentamethylen-Δ²-cyclopentenon und Methansulfonsäurechlorid als Ausgangsprodukt, so kann der Reaktionsverlauf durch folgendes Reaktionsschema wiedergegeben werden:

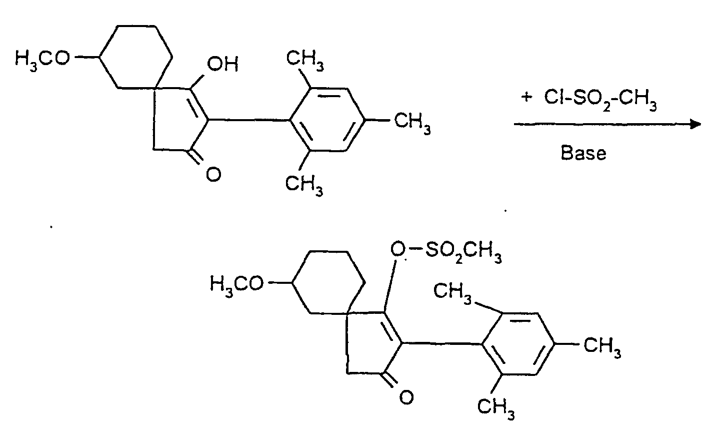

Verwendet man gemäß Verfahren (F) 2-(2,4,6-Trimethylphenyl)-3-hydroxy-4,4-dimethyl-Δ²-cyclopentenon und Methanthio-phosphonsäurechlorid-(2,2,2-trifluorethylester) als Ausgangsprodukte, so kann der Reaktionsverlauf durch folgendes Reaktionsschema wiedergegeben werden:

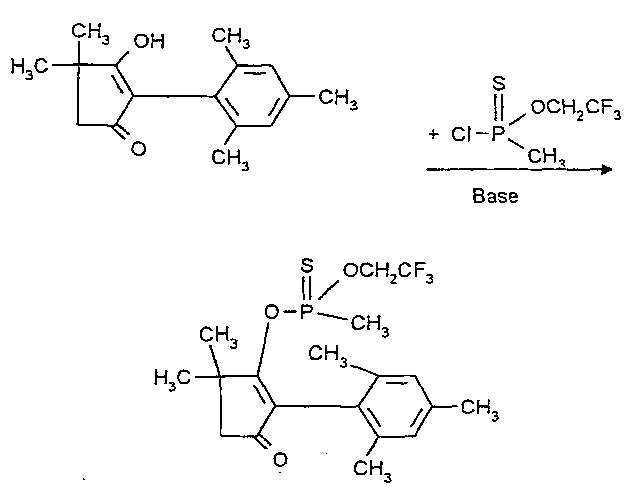

Verwendet man gemäß Verfahren (G_{α}) 2-(2,4,6-Trimethylphenyl)-3-hydroxy-4,4-tetramethylen-Δ²-cyclopentenon und Ethylisocyanat als Ausgangsprodukte, so kann der Reaktionsverlauf durch folgendes Reaktionsschema wiedergegeben werden:

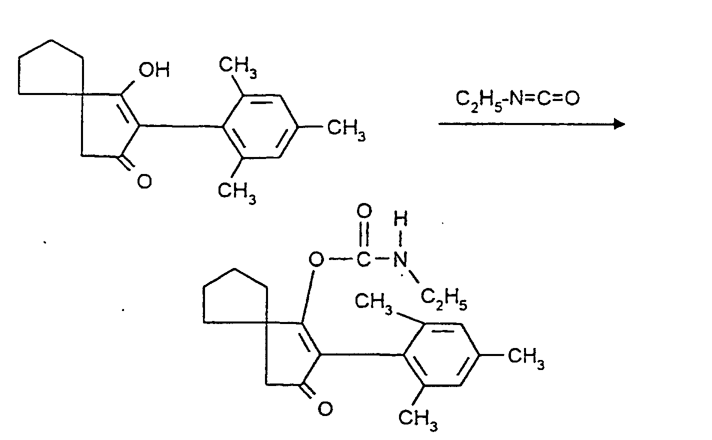

Verwendet man gemäß Verfahren (G_{β}) 2-(2,4,6-Trimethylphenyl)-3-hydroxy-4-tritrifluormethyl-4-methyl-Δ²-cyclopentenon und Dimethylcarbamidsäurechlorid als Ausgangsprodukte, so kann der Reaktionsverlauf durch folgendes Schema wiedergegeben werden:

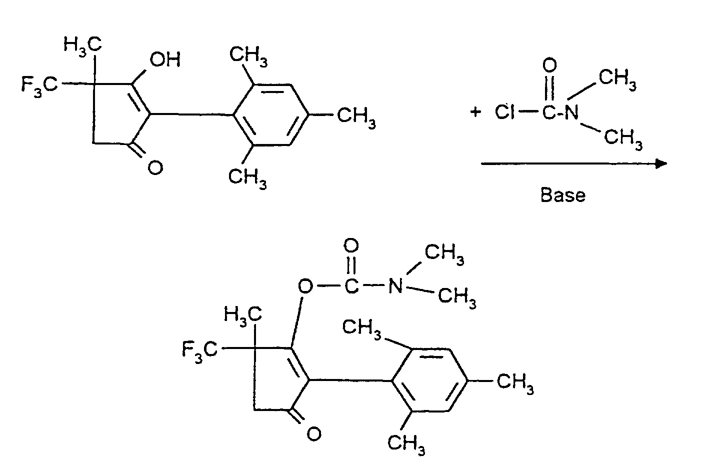

Verwendet man gemäß Verfahren (H) 2-(2,4,6-Trimethylphenyl)-3-hydroxy-4,4-didimethyl-Δ²-cyclopentenon und NaOH als Komponenten, so kann der Verlauf des erfindungsgemäßen Verfahrens durch folgendes Reaktionsschema wiedergegeben werden:

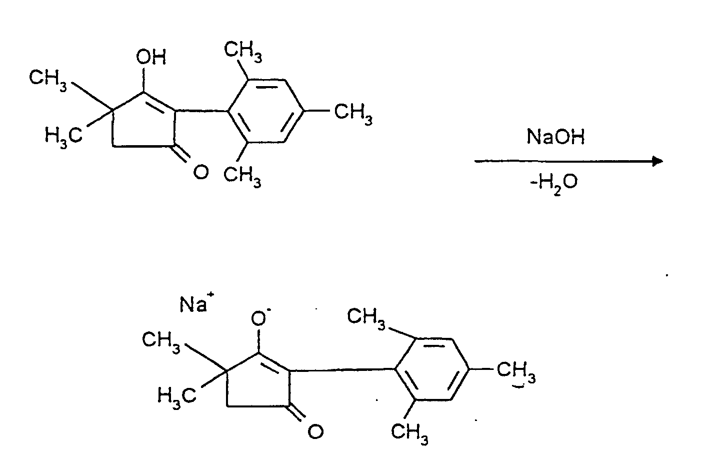

Die bei dem obigen Verfahren (A) als Ausgangsstoffe benötigten Verbindungen der Formel (II) 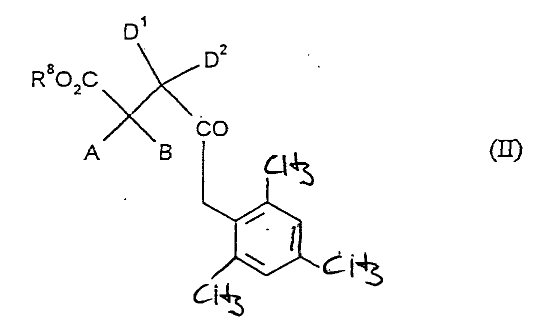 in welcher
- A, B, D¹, D², und R⁸: die oben angegebene Bedeutung haben,
sind neu.

Sie lassen sich nach im Prinzip bekannten Methoden herstellen.

Man erhält die 5-Aryl-4-ketocarbonsäureester der Formel (II) beispielsweise, wenn man 5-Aryl-4-ketocarbonsäuren der Formel (XIV) 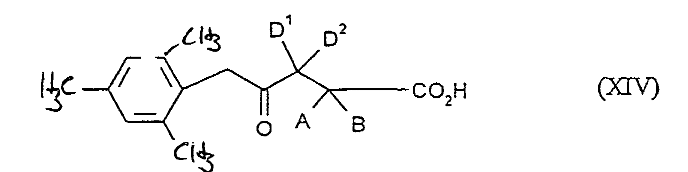 in welcher
- A, B, D¹, D²: die oben angegebene Bedeutung haben,
verestert (vgl. z.B. Organikum, 15. Auflage, Berlin, 1977, Seite 499).

Die 5-Aryl-4-ketocarbonsäuren der Formel (XIV) 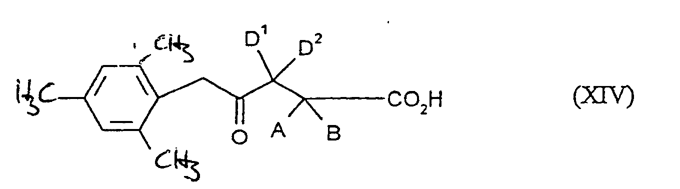 in welcher
- A, B, D¹, D²: die oben angegebene Bedeutung haben,
sind neu, lassen sich aber nach im Prinzip bekannten Methoden herstellen.

Man erhält die 5-Aryl-4-ketocarbonsäuren der Formel (XIV) beispielsweise, wenn man Carbonsäureanhydride der Formel (XV) 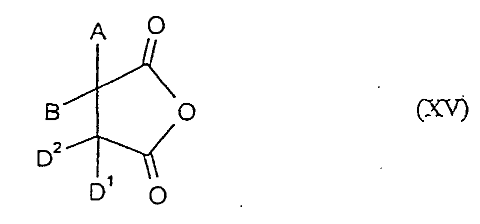 in welcher
- A, B, D¹ und D²: die oben angegebene Bedeutung haben,
mit metallorganischen Verbindungen der Formel (XVI) 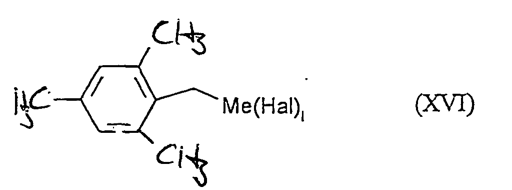 in welcher
- Me: für ein- oder zweiwertige Metallionen, beispielsweise des Lithiums oder Magnesiums,
- Hal: für Chlor oder Brom
und
- l: für eine Zahl 0 oder 1 steht,
in Gegenwart eines Verdünnungsmittels umsetzt (vgl. z.B. Organikum, 15. Auflage, Berlin, 1977, Seite 623).

Die Verbindungen (XV) und (XVI) sind teilweise bekannt und/oder lassen sich nach bekannten Verfahren in einfacher Weise darstellen (vgl. z.B. Organikum, 15. Auflage, Berlin, 1977, Seiten 525, 526 und 623).

Weiterhin erhält man 5-Aryl-4-ketocarbonsäuren der Formel (XIV) 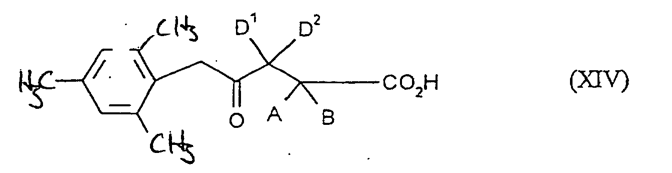 in welcher
- A, B, D¹, D²: die oben angegebene Bedeutung haben,
wenn man 2-Phenyl-3-oxo-adipinsäureester der Formel (XVII) 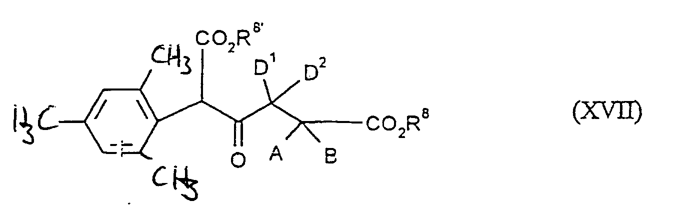 in welcher
- A, B, D¹, D²: die oben angegebene Bedeutung haben und
- R⁸ und R^{8'}: für Alkyl (insbesondere C₁-C₈-Alkyl) stehen,
gegebenenfalls in Gegenwart eines Verdünnungsmittels und gegebenenfalls in Gegenwart einer Base oder Säure decarboxyliert (vgl. z.B. Organikum, 15. Auflage, Berlin, 1977, Seite 519 bis 521).

Die Verbindungen der Formel (XVII) 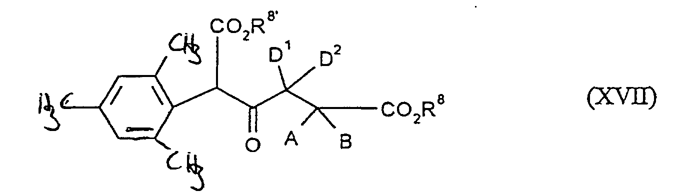 in welcher
- A, B, D¹, D², R⁸, R^{8'}: die oben angegebene Bedeutung haben,
sind neu.

Man erhält die Verbindungen der Formel (XVII) beispielsweise,
wenn man Dicarbonsäurehalbesterchloride der Formel (XVIII), 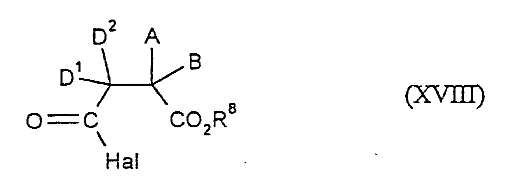 in welcher
- A, B, D¹, D² und R⁸: die oben angegebene Bedeutung haben und
- Hal: für Chlor oder Brom steht,
mit einem Phenylessigsäureester der Formel (XIX) 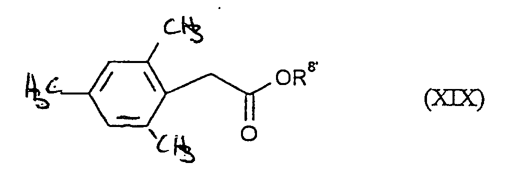 in welcher
- R^{8'}: die oben angegebene Bedeutung hat,
in Gegenwart eines Verdünnungsmittels und in Gegenwart einer Base acyliert (vgl. z.B. M.S. Chambers, E. J. Thomas, D.J. Williams, J. Chem. Soc. Chem. Commun., (1987), 1228).

Die Verbindungen der Formeln (XVIII) und (XIX) sind allgemein bekannte Verbindungen der Organischen Chemie und/oder lassen sich nach im Prinzip bekannten Methoden in einfacher Weise herstellen.

Das Verfahren (A) ist dadurch gekennzeichnet, daß man Verbindungen der Formel (II), in welcher A, B, D¹, D², und R⁸ die oben angegebene Bedeutung haben, in Gegenwart einer Base einer intramolekularen Kondensation unterwirft.

Als Verdünnungsmittel können bei dem erfindungsgemäßen Verfahren (A) alle gegenüber den Reaktionsteilnehmern inerten organischen Solventien eingesetzt werden. Vorzugsweise verwendbar sind Kohlenwasserstoffe, wie Toluol und Xylol, ferner Ether, wie Dibutylether, Tetrahydrofuran, Dioxan, Glykoldimethylether und Diglykoldimethylether, außerdem polare Lösungsmittel, wie Dimethylsulfoxid, Sulfolan, Dimethylformamid und N-Methyl-pyrrolidon. Weiterhin können Alkohole wie Methanol, Ethanol, Propanol, iso-Propanol, Butanol, Isobutanol, tert.-Butanol eingesetzt werden.

Als Basen (Deprotonierungsmittel) können bei der Durchführung des erfindungsgemäßen Verfahrens (A) alle üblichen Protonenakzeptoren eingesetzt werden. Vorzugsweise verwendbar sind Alkalimetall- und Erdalkalimetalloxide, -hydroxide und -carbonate, wie Natriumhydroxid, Kaliumhydroxid, Magnesiumoxid, Calciumoxid, Natriumcarbonat, Kaliumcarbonat und Calciumcarbonat, die auch in Gegenwart von Phasentransferkatalysatoren wie z.B. Triethylbenzylammoniumchlorid, Tetrabutylammoniumbromid, Adogen 464 (Methyltrialkyl(C₈-C₁₀)ammoniumchlorid) oder TDA 1 (Tris-(methoxyethoxyethyl)-amin) eingesetzt werden können. Weiterhin können Alkalimetalle wie Natrium oder Kalium verwendet werden. Ferner sind Alkalimetall- und Erdalkalimetallamide und -hydride, wie Natriumamid, Natriumhydrid und Calciumhydrid, und außerdem auch Alkalimetall-alkoholate, wie Natrium-methylat, Natrium-ethylat und Kalium-tert.-butylat einsetzbar.

Die Reaktionstemperaturen können bei der Durchführung des erfindungsgemäßen Verfahrens (A) innerhalb eines größeren Bereiches variiert werden. Im allgemeinen arbeitet man bei Temperaturen zwischen 0°C und 250°C, vorzugsweise zwischen 50°C und 150°C.

Das erfindungsgemäße Verfahren (A) wird im allgemeinen unter Normaldruck durchgeführt.

Bei der Durchführung des erfindungsgemäßen Verfahrens (A) setzt man die Reaktionskomponenten der Formel (II) und die deprotonierenden Basen im allgemeinen in etwa äquimolaren Mengen ein. Es ist jedoch auch möglich, die eine oder andere Komponente in einem größeren Überschuß (bis zu 3 Mol) zu verwenden.

Das Verfahren (Bα) ist dadurch gekennzeichnet, daß man Verbindungen der Formel (Ia) mit Carbonsäurehalogeniden der Formel (III) umsetzt.

Als Verdünnungsmittel können bei dem erfindungsgemäßen Verfahren (Bα) alle gegenüber den Säurehalogeniden inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenstoff, Chlorbenzol und o-Dichlorbenzol, außerdem Ketone, wie Aceton und Methylisopropylketon, weiterhin Ether, wie Diethylether, Methyl-tert.butylether, Tetrahydrofuran und Dioxan, darüber hinaus Carbonsäureester, wie Ethylacetat, und auch stark polare Solventien, wie Dimethylsulfoxid und Sulfolan. Wenn die Hydrolysestabilität des Säurehalogenids es zuläßt, kann die Umsetzung auch in Gegenwart von Wasser durchgeführt werden.

Als Säurebindemittel kommen bei der Umsetzung nach dem erfindungsgemäßen Verfahren (Bα) alle üblichen Säureakzeptoren in Betracht. Vorzugsweise verwendbar sind tertiäre Amine, wie Triethylamin, Pyridin, Diazabiyclooctan (DABCO), Diazabicycloundecan (DBU), Diazabicyclononen (DBN), Hünig-Base und N,N-Dimethyl-anilin, ferner Erdalkalimetalloxide, wie Magnesium- und Calciumoxid, außerdem Alkali- und Erdalkali-metall-carbonate, wie Natriumcarbonat, Kaliumcarbönat und Calciumcarbonat.

Die Reaktionstemperaturen können bei dem erfindungsgemäßen Verfahren (Bα) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens (Bα) werden die Ausgangsstoffe der Formel (Ia) und das Carbonsäurehalogenid der Formel (III) im allgemeinen in angenähert äquivalenten Mengen verwendet. Es ist jedoch auch möglich, das Carbonsäurehalogenid in einem größeren Überschuß (bis zu 5 Mol) einzusetzen. Die Aufarbeitung erfolgt nach üblichen Methoden.

Das Verfahren (Bβ) ist dadurch gekennzeichnet, daß man Verbindungen der Formel (Ia) mit Carbonsäureanhydriden der Formel (IV) umsetzt.

Bei dem erfindungsgemäßen Verfahren (Bβ) können als Verdünnungsmittel vorzugsweise diejenigen Verdünnungsmittel verwendet werden, die auch bei der Verwendung von Säurehalogeniden vorzugsweise in Betracht kommen. Im übrigen kann auch ein im Überschuß eingesetztes Carbonsäureanhydrid gleichzeitig als Verdünnungsmittel fungieren.

Die Reaktionstemperaturen können bei dem erfindungsgemäßen Verfahren (Bβ) innerhalb eines größeren Bereiches variiert werden. Im allgemeinen arbeitet man bei Temperaturen zwischen -20°C und +150°C, vorzugsweise zwischen 0°C und 100°C.

Bei der Durchführung des erfindungsgemäßen Verfahrens werden die Ausgangsstoffe der Formel (Ia) und das Carbonsäureanhydrid der Formel (IV) im allgemeinen in angenähert äquivalenten Mengen verwendet. Es ist jedoch auch möglich, das Carbönsäureanhydrid in einem größeren Überschuß (bis zu 5 Mol) einzusetzen. Die Aufarbeitung erfolgt nach üblichen Methoden.

Im allgemeinen geht man so vor, daß man Verdünnungsmittel und im Überschuß vorhandenes Carbonsäureanhydrid sowie die entstehende Carbonsäure durch Destillation oder durch Waschen mit einem organischen Lösungsmittel oder mit Wasser entfernt.

Das Verfahren (C) ist dadurch gekennzeichnet, daß man Verbindungen der Formel (Ia) mit Chlorameisensäureestern oder Chlorameisensäurethiolestern der Formel (V) umsetzt.

Als Säurebindemittel kommen bei der Umsetzung nach dem erfindungsgemäßen Verfahren (C) alle üblichen Säureakzeptoren in Betracht. Vorzugsweise verwendbar sind tertiäre Amine, wie Triethylamin, Pyridin, DABCO, DBN, DBU, Hünig-Base und N,N-Dimethyl-anilin, ferner Erdalkalimetalloxide, wie Magnesium- und Calcium-oxid, außerdem Alkali- und Erdalkali-metall-carbonate, wie Natriumcarbonat, Kaliumcarbonat und Calciumcarbonat.

Als Verdünnungsmittel können bei dem erfindungsgemäßen Verfahren (C) alle gegenüber den Chlorameisensäureestern bzw. Chlorameisensäurethiolestern inerten Solventien eingesetzt werden. Vorzugsweise verwendbar sind Kohlenwasserstoffe, wie Benzin, Benzol, Toluol, Xylol und Tetralin, ferner Halogenkohlenwasserstoffe, wie Methylenchlorid, Chloroform, Tetrachlorkohlenwasserstoff, Chlorbenzol und o-Dichlorbenzol, außerdem Ketone, wie Aceton und Methylisopropylketon, weiterhin Ether, wie Diethylether, Tetrahydrofuran und Dioxan, darüber hinaus Carbonsäureester, wie Ethylacetat, und auch stark polare Solventien, wie Dimethylsulfoxid und Sulfolan.

Die Reaktionstemperaturen können bei der Durchführung des erfindungsgemäßen Verfahrens (C) innerhalb eines größeren Bereiches variiert werden. Arbeitet man in Gegenwart eines Verdünnungsmittels und eines Säurebindemittels, so liegen die Reaktionstemperaturen im allgemeinen zwischen -20°C und +100°C, vorzugsweise zwischen 0°C und 50°C.

Das erfindungsgemäße Verfahren (C) wird im allgemeinen unter Normaldruck durchgeführt.

Bei der Durchführung des erfindungsgemäßen Verfahrens (C) werden die Ausgangsstoffe der Formel (Ia) und der entsprechende Chlorameisensäureester bzw. Chlorameisensäurethiolester der Formel (V) im allgemeinen in angenähert äquivalenten Mengen verwendet. Es ist jedoch auch möglich, die eine oder andere Komponente in einem größeren Überschuß (bis zu 2 Mol) einzusetzen. Die Aufarbeitung erfolgt dann nach üblichen Methoden. Im allgemeinen geht man so vor, daß man ausgefallene Salze entfernt und das verbleibende Reaktionsgemisch durch Abziehen des Verdünnungsmittels einengt.

Beim Herstellungsverfahren (Dα) setzt man pro Mol Ausgangsverbindung der Formel (Ia) ca. 1 Mol Chlormonothioameisensäureester bzw. Chlordithioameisensäureester der Formel (VI) bei 0 bis 120°C, vorzugsweise bei 20 bis 60°C um.

Als gegebenenfalls zugesetzte Verdünnungsmittel kommen alle inerten polaren organischen Lösungsmittel in Frage, wie Halogenkohlenwasserstoffe, Nitrile, Carbonsäureester, Ether, Amide, Sulfone, Sulfoxide.

Vorzugsweise werden Acetonitril, Ethylacetat, Dimethylsulfoxid, Tetrahydrofuran, Dimethylformamid oder Dichlormethan eingesetzt.

Stellt man in einer bevorzugten Ausführungsform durch Zusatz von starken Deprotonierungsmitteln wie z.B. Natriumhydrid oder Kaliumtertiärbutylat das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (Dß) setzt man pro Mol Ausgangsverbindung der Formel (II) die äquimolare Menge bzw. einen Überschuß Schwefelkohlenstoff zu. Man arbeitet hierbei vorzugsweise bei Temperaturen von 0 bis 50°C und insbesondere bei 20 bis 30°C.

Das Verfahren (Dβ) wird gegebenenfalls in Gegenwart einer Base durchgeführt.

Als Basen können beim Verfahren (Dβ) alle üblichen Protonenakzeptoren eingesetzt werden. Vorzugsweise verwendbar sind Alkalimetallhydride, Alkalimetall-alkoholate, Alkali- oder Erdalkalimetallcarbonate oder -hydrogencarbonate oder Stickstoffbasen. Genannt seien beispielsweise Natriumhydrid, Natriummethanolat, Natriumhydroxid, Calciumhydroxid, Kaliumcarbonat, Natriumhydrogencarbonat, Triethylamin, Dibenzylamin, Diispropylamin, Pyridin, Chinolin, Diazabicyclooctan (DABCO), Diazabicyclononen (DBN) und Diazabicycloundecen (DBU).

Als Verdünnungsmittel können bei diesem Verfahren alle üblichen Lösungsmittel verwendet werden.

Vorzugsweise verwendbar sind aromatische Kohlenwasserstoffe wie Benzol oder Toluol, Alkohole wie Methanol, Ethanol, Isopropanol oder Ethylenglykol, Nitrile wie Acetonitril, Ether wie Tetrahydrofuran oder Dioxan, Amide wie Dimethylformamid oder andere polare Lösungsmittel wie Dimethylsulfoxid oder Sulfolan.

Oft ist es zweckmäßig zunächst aus der Verbindung der Formel (Ia) durch Zusatz eines Deprotonierungsmittels (wie z.B. Kaliumtertiärbutylat oder Natriumhydrid) das entsprechende Salz herzustellen. Man setzt die Verbindung der Formel (Ia) so lange mit Schwefelkohlenstoff um, bis die Bildung der Zwischenverbindung abgeschlossen ist, z.B. nach mehrstündigem Rühren bei Raumtemperatur.

Die weitere Umsetzung mit dem Alkylhalogenid der Formel (VII) erfolgt vorzugsweise bei 0 bis 70°C und insbesondere bei 20 bis 50°C. Hierbei wird mindestens die äquimolare Menge Alkylhalogenid eingesetzt.

Man arbeitet bei Normaldruck oder unter erhöhtem Druck, vorzugsweise bei Normaldruck.

Die Aufarbeitung erfolgt wiederum nach üblichen Methoden.

Beim Herstellungsverfahren (E) setzt man pro Mol Ausgangsverbindung der Formel (Ia) ca. 1 Mol Sulfonsäurechlorid der Formel (VIII) bei 0 bis 150°C, vorzugsweise bei 20 bis 70°C um.

Als gegebenenfalls zugesetzte Verdünnungsmittel kommen alle inerten polaren organischen Lösungsmittel in Frage wie Halogenkohlenwasserstoffe, Carbonsäureester, Ether, Amide, Nitrile, Sulfone, Sulfoxide.

Vorzugsweise werden Acetonitril, Ethylacetat, Dimethylsulfoxid, Tetrahydrofuran, Dimethylformamid oder Dichlormethan eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin und Triethylamin aufgeführt.

Die Reaktion kann bei Normaldruck oder unter erhöhtem Druck durchgeführt werden, vorzugsweise wird bei Normaldruck gearbeitet. Die Aufarbeitung geschieht nach üblichen Methoden.

Beim Herstellungsverfahren (E) kann gegebenenfalls unter Phasen-Transfer-Bedingungen gearbeitet werden (W.J. Spillane et. al.; J. Chem. Soc., Perkin Trans I, (3) 677-9 (1982)). In diesem Fall setzt man pro Mol Ausgangsverbindung der Formel Ia) 0,3 bis 5 Mol Sulfonsäurechlorid der Formel (VIII), bevorzugt 1 Mol bei 0° bis 150°C, vorzugsweise bei 20 bis 70°C um.

Als Phasen-Transfer-Katalysatoren können alle quartären Ammoniumsalze verwendet werden, vorzugsweise Tetraoctylammoniumbromid und Benzyltriethylammoniumchlorid. Als organische Lösungsmittel können in diesem Fall alle unpolaren inerten Lösungsmittel dienen, bevorzugt werden Benzol und Toluol eingesetzt.

Beim Herstellungsverfahren (F) setzt man zum Erhalt von Verbindungen der Formel (Ie) auf 1 Mol der Verbindung (Ia), 1 bis 2, vorzugsweise 1 bis 1,3 Mol der Phosphorverbindung der Formel (IX) bei Temperaturen zwischen -40°C und 150°C, vorzugsweise zwischen -10 und 110°C, ein.

Als gegebenenfalls zugesetzte Verdünnungsmittel kommen aller inerten, polaren organischen Lösungsmittel in Frage wie Halogenkohlenwasserstoffe, Ether, Amide, Nitrile, Sulfide, Sulfone, Sulfoxide etc.

Vorzugsweise werden Acetonitril, Ethylacetat, Dimethylsulfoxid, Tetrahydrofuran, Dimethylformamid oder Dichlormethan eingesetzt.

Als gegebenenfalls zugesetzte Säurebindemittel kommen übliche anorganische oder organische Basen in Frage wie Hydroxide, Carbonate. Beispielhaft seien Natriumhydroxid, Natriumcarbonat, Kaliumcarbonat, Pyridin, Triethylamin und DABCO aufgeführt.

Die Umsetzung kann bei Normaldruck oder unter erhöhtem Druck durchgeführt werden, vorzugsweise wird bei Normaldruck gearbeitet. Die Aufarbeitung geschieht nach üblichen Methoden der Organischen Chemie. Die Reinigung der anfallenden Endprodukte geschieht vorzugsweise durch Kristallisation, chromatographische Reinigung oder durch sogenanntes "Andestillieren", d.h. Entfernung der flüchtigen Bestandteile im Vakuum.

Beim Herstellungsverfahren (Gα) setzt man pro Mol Ausgangsverbindung der Formel (Ia) ca. 1 Mol Isocyanat bzw. Isothiocyanat der Formel (X) bei 0 bis 100°C, vorzugsweise bei 20 bis 50°C um.

Als gegebenenfalls zugesetzte Verdünnungsmittel kommen alle inerten organischen Lösungsmittel in Frage, wie Kohlenwasserstoffe, Halogenkohlenwasserstoffe, Carbonsäureester, Ether, Amide, Nitrile, Sulfone, Sulfoxide. Vorzugsweise werden Toluol, Methylenchlorid, Tetrahydrofuran, Ethylacetat, Dimethylformamid oder Dimethylsulfoxid eingesetzt.

Gegebenenfalls können Katalysatoren zur Beschleunigung der Reaktion zugesetzt werden. Als Katalysatoren können sehr vorteilhaft zinnorganische Verbindungen, wie z.B. Dibutylzinndilaurat eingesetzt werden. Es wird vorzugsweise bei Normaldruck gearbeitet.

Beim Herstellungsverfahren (Gβ) setzt man pro Mol Ausgangsverbindung der Formel (Ia) ca. 1 Mol Carbamidsäurechlorid bzw. Thiocarbamidsäurechlorid der Formel (XI) bei 0 bis 150°C, vorzugsweise bei 20 bis 70°C um.

Als gegebenenfalls zugesetzte Verdünnungsmittel kommen aller inerten polaren organischen Lösungsmittel in Frage wie Halogenkohlenwasserstoffe, Carbonsäureester, Ether, Amide, Sulfone, Sulfoxide.

Vorzugsweise werden Acetonitril, Ethylacetat, Dimethylsulfoxid, Tetrahydrofuran, Dimethylformamid oder Dichlormethan eingesetzt.

Stellt man in einer bevorzugten Ausführungsform durch Zusatz von starken Deprotonierungsmitteln (wie z.B. Natriumhydrid oder Kaliumtertiärbutylat) das Enolatsalz der Verbindung der Formel (Ia) dar, kann auf den weiteren Zusatz von Säurebindemitteln verzichtet werden.

Werden Säurebindemittel eingesetzt, so kommen übliche anorganische oder organische Basen in Frage, beispielhaft seien Natriumhydroxid, Natriumcarbonat, Kaliumcarbonat, Pyridin, Triethylamin und DABCO aufgeführt.

Die Reaktion kann bei Normaldruck oder unter erhöhtem Druck durchgeführt werden, vorzugswiese wird bei Normaldruck gearbeitet. Die Aufarbeitung geschieht nach üblichen Methoden.

Das Verfahren (H) ist dadurch gekennzeichnet, daß man Verbindungen der Formel (Ia) mit Metallverbindungen der Formel (XII) oder Aminen der Formel (XIII) umsetzt.

Als Verdünnungsmittel können bei dem erfindungsgemäßen Verfahren vorzugsweise Ether wie Tetrahydrofuran, Dioxan, Diethylether oder aber Alkohole wie Methanol, Ethanol, Isopropanol, aber auch Wasser eingesetzt werden. Das erfindungsgemäße Verfahren (H) wird im allgemeinen unter Normaldruck durchberührt. Die Reaktionstemperaturen liegen im allgemeinen zwischen -20°C und 100°C, vorzugsweise zwischen 0°C und 50°C.

Bei der Durchführung des erfindungsgemäßen Verfahrens (H) werden die Ausgangsstoffe der Formeln (Ia) bzw. (XII) oder (XIII) im allgemeinen in angenähert äquimolaren Mengen verwendet. Es ist jedoch auch möglich, die eine oder andere Komponente in einem größeren Überschuß (bis zu 2 Mol) einzusetzen. Im allgemeinen geht man so vor, daß man das Reaktionsgemisch durch Abziehen des Verdünnungsmittel einengt.

Beispielhaft seien folgende Verbindungen der Formel (II) genannt:
5-(2,4,6-Trimethylphenyl)-4-oxo-2,2-dimethyl-pentancarbonsäuremethylester
5-(2,4,6-Trimethylphenyl)-4-oxo-2-methyl-2-ethyl-pentancarbonsäuremethylester
5-(2,4,6-Trimethylphenyl)-4-oxo-2-methyl-2-isopropyl-pentancarbonsäuremethylester
5-(2,4,6-Trimethylphenyl)-4-oxo-2,2-pentamethylen-pentancarbonsäuremethylester
5-(2,4,6-Trimethylphenyl)-4-oxo-2,2-tetramethylen-pentancarbonsäuremethylester
5-(2,4,6-Trimethylphenyl)-4-oxo-2,2-(2,3-benzotetramethylen)-pentancarbonsäuremethylester
5-(2,4,6-Trimethylphenyl)-4-oxo-2,2-hexamethylen-pentancarbonsäuremethylester
5-(2,4,6-Trimethylphenyl)-4-oxo-2,2-(2-methylpentamethylen)-pentancarbonsäuremethylester
5-(2,4,6-Trimethylphenyl)-4-oxo-2,2-(3-methylpentamethylen)-pentancarbonsäuremethylester
5-(2,4,6-Trimethylphenyl)-4-oxo-2,2-(3-ethylpentamethylen)-pentancarbonsäuremethylester
5-(2,4,6-Trimethylphenyl)-4-oxo-2,2-(3-(1-methylethyl)pentamethylen)-pentancarbonsäuremethylester
5-(2,4,6-Trimethylphenyl)-4-oxo-2,2-(3-methoxypentamethylen)-pentancarbonsäuremethylester
5-(2,4,6-Trimethylphenyl)-4-oxo-2,2-(3-ethoxypentamethylen)-pentancarbonsäuremethylester
5-(2,4,6-Trimethylphenyl)-4-oxo-2,2-(2,3-dimethylpentamethylen)-pentancarbonsäuremethylester

Die erfindungsgemäßen Wirkstoffe eignen sich zur Bekämpfung von tierischen Schädlingen, vorzugsweise Arthropoden und Nematoden, insbesondere Insekten und Spinnentieren, die in der Landwirtschaft, in Forsten; im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:
Aus der Ordnung der Isopoda z.B. Oniscus asellus, Armadillidium vulgare, Porcellio scaber.
Aus der Ordnung der Diplopoda z.B. Blaniulus guttulatus
   Aus der Ordnung der Chilopoda z.B. Geophilus carpophagus, Scutigera spec.
   Aus der Ordnung der Symphyla z.B. Scutigerella immaculata.
   Aus der Ordnung der Thysanura z.B. Lepisma saccharina.
   Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria, Supella spp..
Aus der Ordnung der Dermaptera z.B. Forficula auricularia.
   Aus der Ordnung der Isoptera z.B. Reticulitermes spp..
Aus der Ordnung der Anoplura z.B. Phylloxera vastatrix, Pemphigus spp., Phthirus spp., Pediculus spp., Haematopinus spp., Linognathus spp., Solenopotes spp.. Aus der Ordnung der Mallophaga z.B. Trichodectes spp., Damalinea spp., Trimenopon spp., Monopon spp., Trinoton spp., Bovicola spp., Werneckiella spp., Lepikentron spp., Felicola spp..
Aus der Ordnung der Thysanoptera z.B. Hercinothrips femoralis, Thrips tabaci. Aus der Ordnung der Heteroptera z.B. Eurygaster spp., Dysdercus intermedius, Piesma quadrata, Cimex lectularius, Rhodnius prolixus, Triatoma spp., Panstrongylus spp..
Aus der Ordnung der Homoptera z.B. Aleurodes brassicae, Bemisia tabaci, Trialeurodes vaporariorum, Aphis gossypii, Brevicoryne brassicae, Cryptomyzüs ribis, Aphis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp., Earias insulana, Heliothis spp., Spodoptera exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Acanthoscelides obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varive stis, Atomaria spp., Oryzaephilus surinamensis, Antho nomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Cono derus spp., Melolontha melolontha, Amphimallon solsti tialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora spp., Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa, Simulium spp., Eusimulium spp., Phlebotomus spp., Lutzomyia spp., Culicoides spp., Chrysops spp., Hybomitra spp., Atylotus spp., Tabanus spp., Haematopota spp., Philipomyia spp., Braula spp., Hydrotaea spp., Stomoxys spp., Haematobia spp., Morellia spp., Fannia spp., Glossina spp., Calliphora spp., Wohlfahrtia spp., Sarcophaga spp., Oestrus spp., Hypoderma spp., Gasterophilus spp., Hippobosca spp., Lipoptena spp., Melophagus spp., Muscina spp..
Aus der Ordnung der Siphonapterida z.B. Xenopsylla spp., Ceratophyllus spp., Pulex spp., Ctenocephalides spp..
Aus der Ordnung der Arachnida z.B. Scorpio maurus, Latrodectus mactans.
Aus der Ordnung der Acarina z.B. Myocoptes spp., Otodectes spp., Acarus siro, Argas spp., Ornithodoros spp., Ornithonyssus spp., Dermanyssus spp., Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Dermacentor spp., Haemaphysalis spp., Raillietia spp., Pneumonyssus spp., Sternostorma spp., Varroa spp..
Aus der Ordnung der Actinedida (Prostigmata) und Acaridida (Astigmata) z.B.: Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Die erfindungsgemäßen Wirkstoffe zeichnen sich durch eine hohe insektizide und akarizide Wirksamkeit aus.

Sie lassen sich mit besonders gutem Erfolg zur Bekämpfung von pflanzenschädigenden Insekten verwenden, wie beispielsweise gegen die Larven des Meerrettichblattkäfers (Phaedon cochleariae) oder gegen die Larven der grünen Reiszikade (Nephotettix cincticeps) oder gegen die Raupen der Kohlschabe (Plutella maculipennis).

Die erfindungsgemäßen Wirkstoffe können weiterhin als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotola, Lindernia, Lamium, Veronica, Abutilon, Emex, Datura, Viola, Galeopsis, Papaver, Centaurea, Trifolium, Ranunculus, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cycnodon, Monochoria, Fimbristylis, Sagittaria, Eleocharis, Scirpus, Paspalum, Ischaemum, Sphenoclea, Dactyloctenium, Agrostis, Alopecurus, Apera.
Monokotyle Kulturen der Gattungen: Oryza, Zea, Triticum, Hordeum, Avena, Secale, Sorghum, Panicum, Sachharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindunngen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst, Wein-, Citrus-, Nuß-, Bananen-, Kaffee-, Tee-, Gummi-, Ölpalm-, Kakao-, Beerenfruchtund Hopfenanlagen, auf Zier- und Sportrasen und Weideflächen und zur selektiven Unkrautbekämpfung in einjährigen Kulturen eingesetzt werden.

Die erfindungsgemäßen Wirkstoffe eignen sich sehr gut zur selektiven Bekämpfung monokotyler Unkräuter in dikotylen Kulturen im Vor- und Nachlaufverfahren. Sie können beispielsweise in Baumwolle oder Zuckerrüben mit sehr gutem Erfolg zur Bekämpfung von Schadgräser eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Einweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoff, vorzugsweise zwischen 0,5 und 90 %.

Der erfindungsgemäße Wirkstoff kann in seinen handelsüblichen Formulierungen sowie in den aus diesen Formulierungen bereiteten Anwendungsformen in Mischung mit anderen Wirkstoffen, wie Insektiziden, Lockstoffen, Sterilantien, Bakteriziden, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Besonders günstige Mischpartner sind z.B. die folgenden:
**Fungizide:**
   2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxyquinolinsulfat; Methyl-(E)-2-{2-[6-(2-cyanophenoxy)-pyrimidin-4-yloxy]-phenyl}-3-methoxyacrylat; Methyl-(E)-methoximino-[alpha-(o-tolyloxy)-o-tolyl]acetat; 2-Phenylphenol (OPP), Aldimorph, Ampropylfos, Anilazin, Azaconazol,
   Benalaxyl, Benodanil, Benomyl, Binapacryl, Biphenyl, Bitertanol, Blasticidin-S, Bromuconazole, Bupirimate, Buthiobate,
   Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
   Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
   Edifenphos, Epoxyconazole, Ethirimol, Etridiazol,
   Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
   Guazatine,
   Hexachlorobenzol, Hexaconazol, Hymexazol,
   Imazalil, Imibenconazol, Iminoctadin, Iprobenfos (IBP), Iprodion, Isoprothiolan,
   Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Mepanipyrim, Mepronil, Metalaxyl, Metconazol, Methasulfocarb, Methfuroxam, Metiram, Metsulfovax, Myclobutanil,
   Nickel-dimethyldithiocarbamat, Nitrothal-isopropyl, Nuarimol,
   Ofurace, Oxadixyl, Oxamocarb, Oxycarboxin,
   Pefurazoat, Penconazol, Pencycuron, Phosdiphen, Phthalid, Pimaricin, Piperalin, Polycarbamate, Polyoxin, Probenazol, Prochloraz, Procymidon, Propamocarb, Propiconazole, Propineb, Pyrazophos, Pyrifenox, Pyrimethanil, Pyroquilon,
   Quintozen (PCNB),
   Schwefel und Schwefel-Zubereitungen,
   Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
   Validamycin A, Vinclozolin,
   Zineb, Ziram
**Bakterizide:**
   Bronopol, Dichlorophen, Nitrapyrin, Nickel-Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
**Insektizide / Akarizide / Nematizide:**
   Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
   Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
   Cadusafos, Carbaryl, Carbofuran, Carbophenothion, Carbosulfan, Cartap, CGA 157 419, CGA 184699, Chloethocarb, Chlorethoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
   Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
   Edifenphos, Emamectin, Esfenvalerat, Ethiofencarb, Ethion, Ethofenprox, Ethoprophos, Etrimphos,
   Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonöphos, Formothion, Fosthiazat, Fubfenprox, Furathiocarb,
   HCH, Heptenophos, Hexaflumuron, Hexythiazox,
   Imidacloprid, Iprobenfos, Isazophos, Isofenphos, Isoprocarb, Isoxathion, Ivemectin, Lamda-cyhalothrin, Lufenuron,
   Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
   Naled, NC 184, NI 25, Nitenpyram
   Omethoat, Oxamyl, Oxydemethon M, Oxydeprofos,
   Parathion A, Parathion M, Permethrin, Phenthoat, Phorat, Phosalon, Phosmet, Phosphamdon, Phoxim, Pirimicarb, Pirimiphos M, Primiphos A, Profenofos, Promecarb, Propaphos, Propoxur, Prothiofos, Prothoat, Pymetrozin, Pyrachlophos, Pyradaphenthion, Pyresmethrin, Pyrethrum, Pyridaben, Pyrimidifen, Pyriproxifen,
   Quinalphos,
   RH 5992,
   Salithion, Sebufos, Silafluofen, Sulfotep, Sulprofos,
   Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
   Vamidothion, XMC, Xylylcarb, YI 5301 / 5302, Zetamethrin.
**Herbizide:**
   beispielsweise Anilide, wie z.B. Diflufenican und Propanil; Arylcarbonsäuren, wie z.B. Dichlorpicolinsäure, Dicamba und Picloram; Aryloxyalkansäuren, wie z.B. 2,4 D, 2,4 DB, 2,4 DP, Fluroxypyr, MCPA, MCPP und Triclopyr; Aryloxy-phenoxyalkansäureester, wie z.B. Diclofop-methyl, Fenoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z.B. Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin, Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Der erfindungsgemäße Wirkstoff kann ferner in seinen handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 95 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnet sich der Wirkstoff durch eine hervorragende Residualwirkung auf Holz und Ton sowie durch eine gute Alkalistabilität auf gekälkten Unterlagen aus.

Die erfindungsgemäßen Wirkstoffe wirken nicht nur gegen Pflanzen-, Hygieneund Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läuse, Haarlinge, Federlinge und Flöhe. Beispielsweise zeigen sie eine hervorragende Wirksamkeit gegen Zecken, wie beispielsweise Boophilus microplus.

Die erfindungsgemäßen Wirkstoffe der Formel (I) eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist.

Die Anwendung der erfindungsgemäßen Wirkstoffe geschieht im Veterinärsektor in bekannter Weise durch enterale Verabreichung in Form von beispielsweise Tabletten, Kapseln, Tränken, Drenchen, Granulaten, Pasten, Boli, des feed-through-Verfahrens, von Zäpfchen, durch parenterale Verabreichung, wie zum Beispiel durch Injektion (intramuskulär, subcutan, intravenös, intraperitonal u.a.), Implantate, durch nasale Applikation, durch dermale Anwendung in Form beispielsweise des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Haltern, Markierungsvorrichtungen usw.

Bei der Anwendung für Vieh, Geflügel, Haustiere etc. kann man die Wirkstoffe der Formel (I) als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäßen Wirkstoffe geht aus den nachfolgenden Beispielen hervor.

### Herstellungsbeispiele

### Beispiel Ia-1:

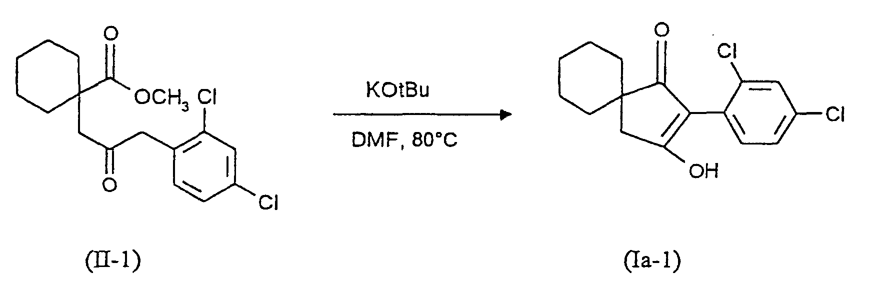

Zu einer Lösung der Verbindung gemäß Beispiel II-1 (5.19 g, 15,13 mmol) in DMF (25 ml) gibt man 1,85 g Kaliumtertbutylat und rührt 2 Stunden bei 80°C. Man gibt 5 ml Essigsäure zu, engt ein und chromatographiert an Kieselgel (2:1 Ethylacetat:Hexan). Man isoliert 3,07 g (65 %) der oben gezeigten Verbindung. Weißer Feststoff, Fp. 219°C.

Beispiel Ia-1 ist nicht Gegenstand des Ansprüche, dient aber zur Veranscharrung des Herstellungsprozesses.

Analog bzw. gemäß den allgemeinen Angaben zur Herstellung wurden die in der Tabelle 14 aufgeführten Verbindungen der Formel (Ia) hergestellt, die in Form eines der möglichen Isomeren dargestellt sind.

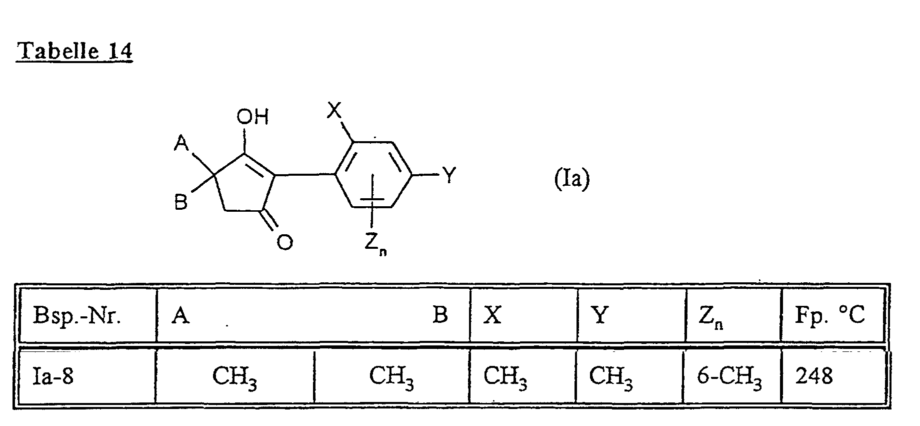

### Beispiel Ib-1

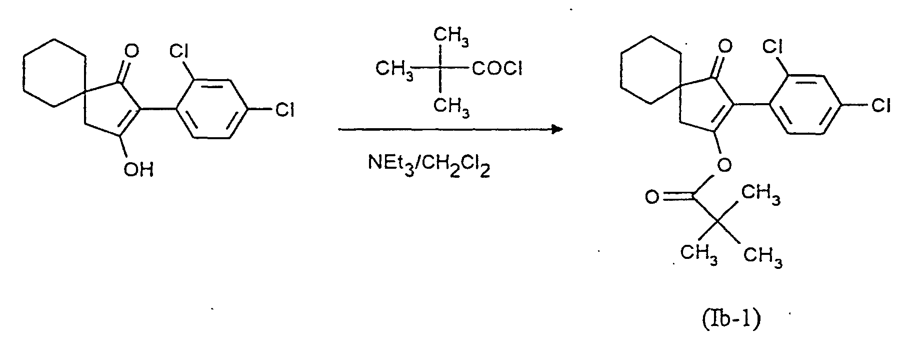

Zu einer Lösung der Verbindung gemäß Beispiel Ia-1 (936 mg, 3 mmol) in Dichlormethan (10 ml) und Triethylamin (1 ml) gibt man Pivaloylchlorid (0,7 ml). Nach einer Stunde bei Raumtemperatur wird das Reaktionsgemisch direkt über Kieselgel filtriert (Laufmittel 1:4, Ethylether:Petrolether). Man erhält 1,1 g (97 %) der oben gezeigten Verbindung. Weißer Feststoff, Fp. 93°C.

Beispiel Ib-1 ist nicht Gegenstand der Ansprüche, dient aber zur Veranchaltung des Herstellungsprozesses.

Analog und gemäß den allgemeinen Angaben zur Herstellung wurden die in der Tabelle 15 aufgeführten Verbindungen der Formel (Ib) hergestellt, die in Form eines der möglichen Isomeren dargestellt sind.

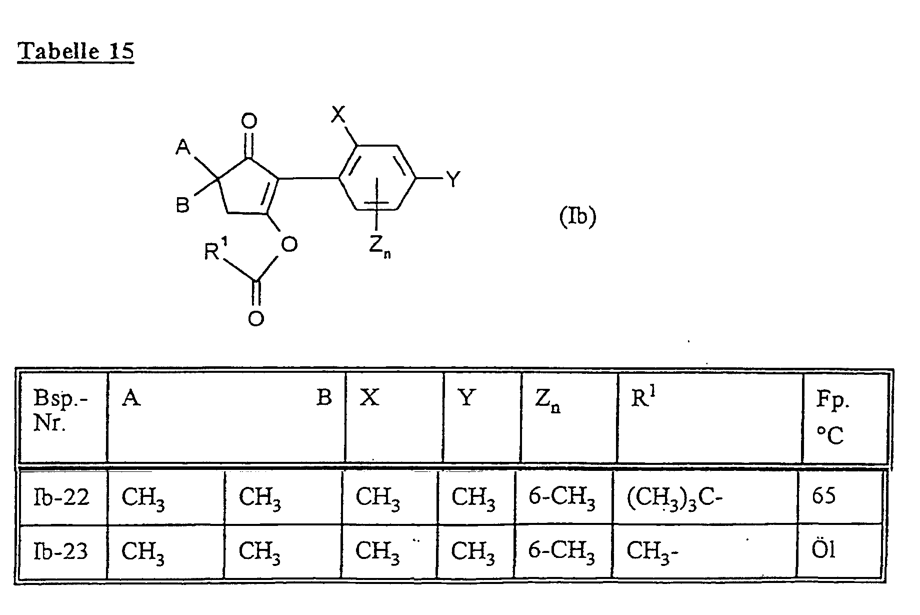

### Beispiel Ic-1:

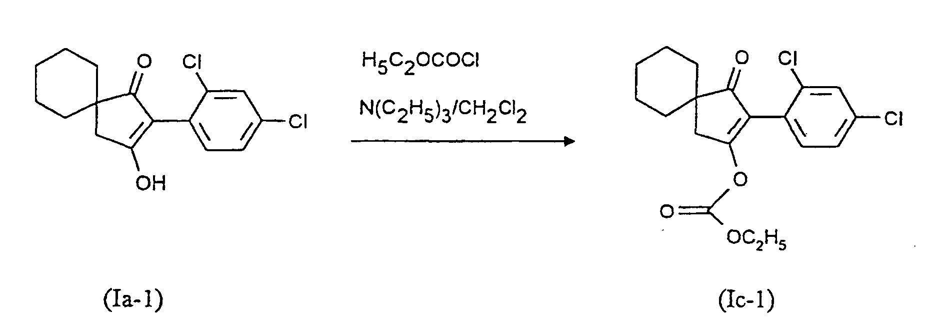

Zu einer Lösung der Verbindung gemäß Beispiel Ia-1 (1,43 g, 4.58 mmol) in CH₂Cl₂ (10 ml) und Triethylamin (2 ml) gibt man 0,8 ml Ethylchloroformiat bei Raumtemperatur. Nach 2,5 Stunden wird das Reaktionsgemisch direkt über Kieselgel filtriert (Laufmittel 4:1, CH₂Cl₂:Petrolether). Man erhält 1,37 g (77 %) der oben gezeigten Verbindung als Öl.
¹HNMR (CDCl₃, 200 MHz, δ ppm): 3.00 (bs, 1H); 4,28 (q, 2H, J = 7 Hz)

Beispiel Ic-1 ist nicht Gegenstand der Ansprüche, dient aber zur Veranchaltung des Herstellungsprozesses.

Analog und gemäß den allgemeinen Angaben zur Herstellung wurden die in der Tabelle 16 aufgeführten Verbindungen der Formel (Ic) hergestellt, die in Form eines der möglichen Isomeren dargestellt sind.

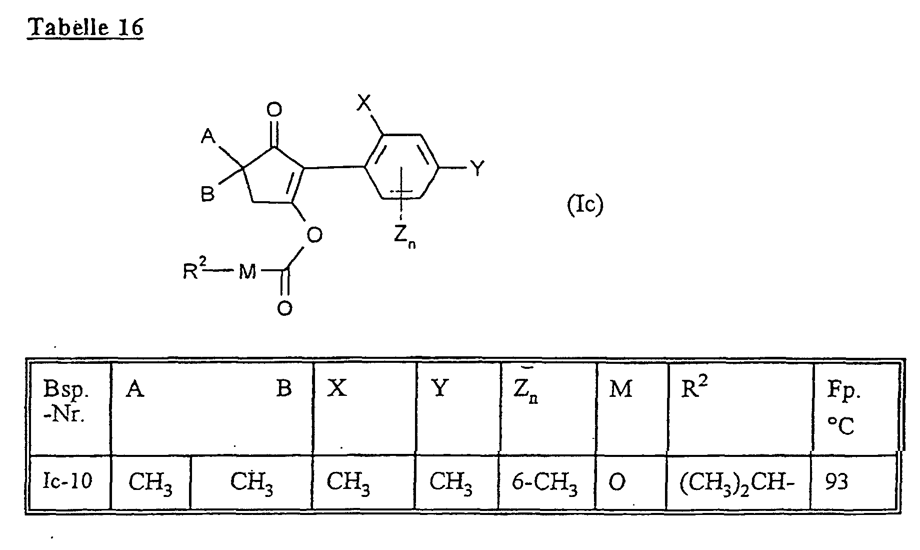

### Herstellung der Ausgangsverbindungen

### Beispiel II-1

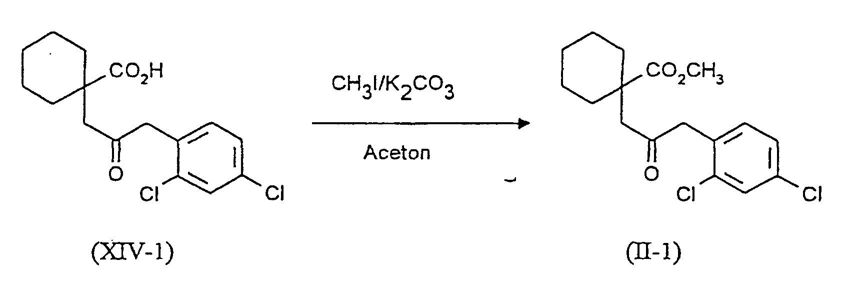

Eine Mischung der Verbindung gemäß Beispiel XIV-1 (1.95 g; 5.93 mmol), Kaliumcarbonat (0,82 g), Aceton (30 ml) und Jodmethan (2 ml) wird 5 Stunden unter Rückfluß gekocht, eingeengt und direkt an Kieselgel chromatographiert (Laufmittel 15 % Ethylacetat, 85 % Petrolether). Man erhält 1,89 g der oben gezeigten Verbindung als Öl (93 %).
¹HNMR (CDCl₃, 200 MHz, δ ppm): 2.86 (s, 2H); 3,65 (s, 3H); 3,78 (s 2H).

Beispiel II-1 ist nicht Gegenstand der Ansprüche, dient aber zur Veranchaltung des Herstellungsprozesses.

Analog wurden die in Tabelle 17 aufgeführten Beispiele der Formel (II) hergestellt.

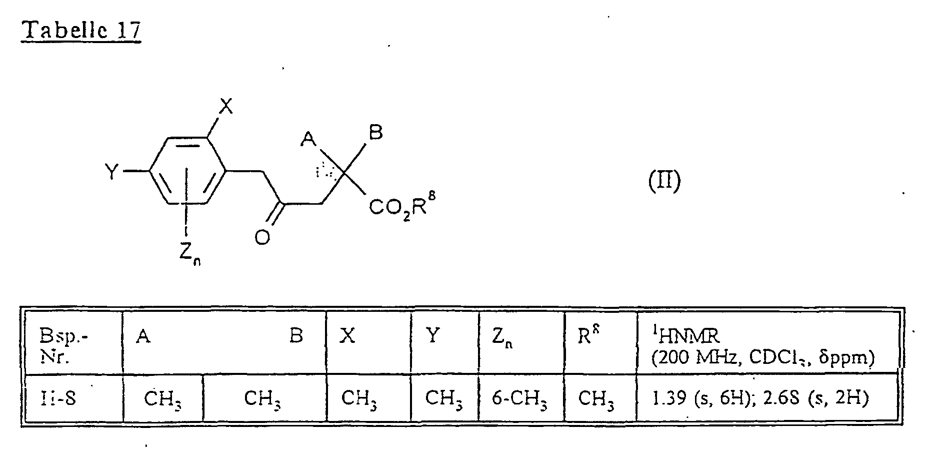

### Beispiel XIV-1

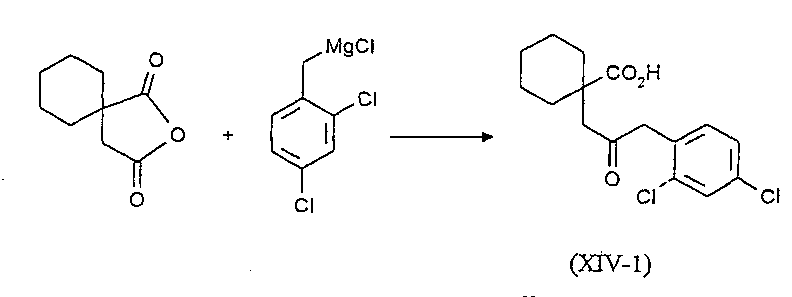

Zu einer Lösung von 10 g 2,2-Pentamethylenbernsteinsäureanhydrid (vgl.: M. Qudrat-Khuda et al., J. Indian Chem. Soc., (1947), 24, 15) in Ethylether (100 ml) gibt man bei Raumtemperatur eine Lösung von 2,4-Dichlorbenzylmagnesiumchlorid (hergestellt aus 12,6 g 2,4-Dichlorbenzylchlorid und 3 g Magnesium in 100 ml Ethylether). Nach 30 Minuten bei Raumtemperatur gibt man 100 ml Wasser und 20 g Ammoniumchlorid zu. Das Produkt wird mit Ethylether extrahiert. Chromatographie an Kieselgel (Laufmittel 30 % Ethylacetat:70 % Petrolether) ergibt 3.95 g (22 %) der oben gezeigten Verbindung als weißen Feststoff vom Fp. 115°C.

Beispiel XIV-1 ist nicht Gegenstand der Ansprüche, dient aber zur Veranchaltung des Herstellungsprozesses.

### Beispiel XIV-2

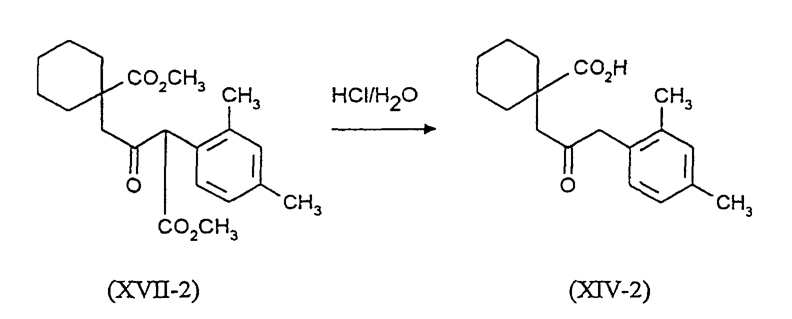

Ein Suspension der Verbindung gemäß Beispiel (XVII-2) (18,2 g mol) in 20 %iger wässriger HCl (300 ml) wird 50 Stunden unter Rückfluß erhitzt, abgekühlt und mit Ethylether extrahiert. Nach Einengen wird der Rückstand mit Petrolether gewaschen. Man erhält 9,5 g (65 %) der oben gezeigten Verbindung als Öl,

Beispiel XIV-2 ist nicht Gegenstand der Ansprüche, dient aber zur Veranchaltung des Herstellungsprozesses.

Analog den unter Beispiel XIV-1 und XIV-2 beschriebenen Verfahren wurden folgende Verbindungen hergestellt:

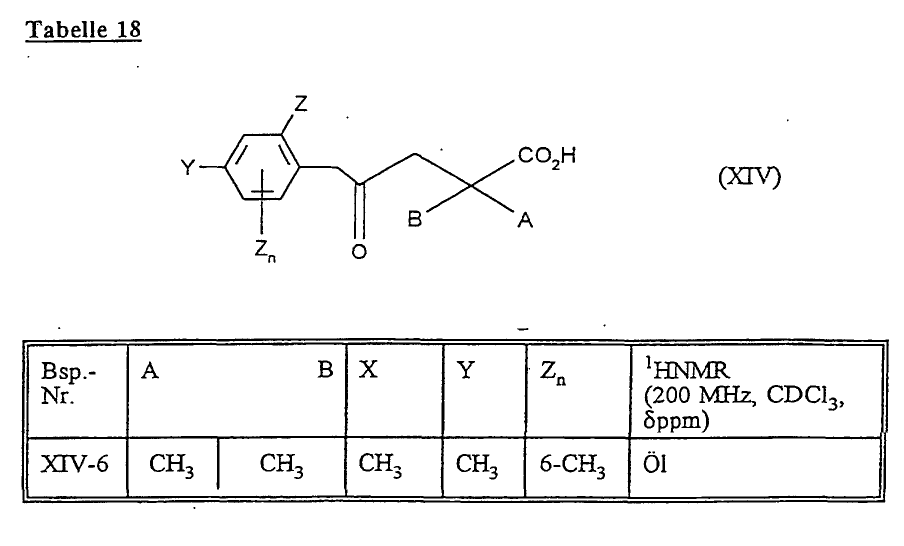

### Beispiel XVII-1

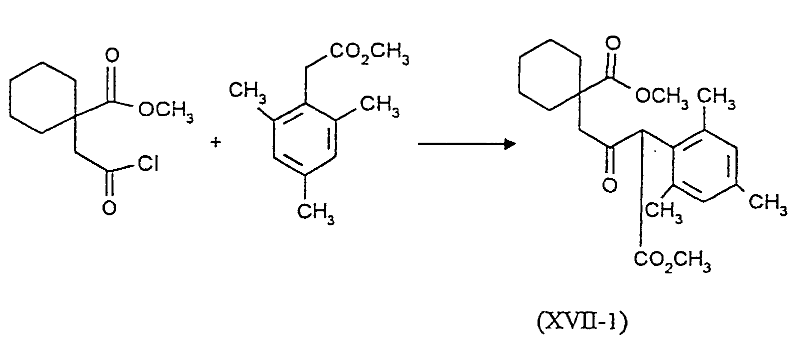

Zu einer Lösung von Lithiumdiisopropylamid (80 mmol) in THF (100 ml) gibt man bei -40°C 14,4 g 2,4,6-Trimethylphenylessigsäuremethylester. Nach 30 Minuten bei Raumtemperatur gibt man 10 g 2,2-Pentamethylen-bernsteinsäuremethylesterchlorid zu, und rührt bei Raumtemperatur (1 Stunde). Dann gibt man 100 ml Wasser und 30 g Ammoniumchlorid zu. Das Produkt wird mit Ethylether extrahiert. Nach Einengen wird der Rückstand an Kieselgel chromatographiert (Laufmittel: CH₂Cl₂). Man erhält 6,2 g (32 %) Feststoff vom Fp. 89°C.

Beispiel XVII-1 ist nicht Gegenstand der Ansprüche, dient aber zur Veranschaltung des Herstellungsprozesses.

Analog wurden die in Tabelle 19 aufgeführten Verbindungen hergestellt:

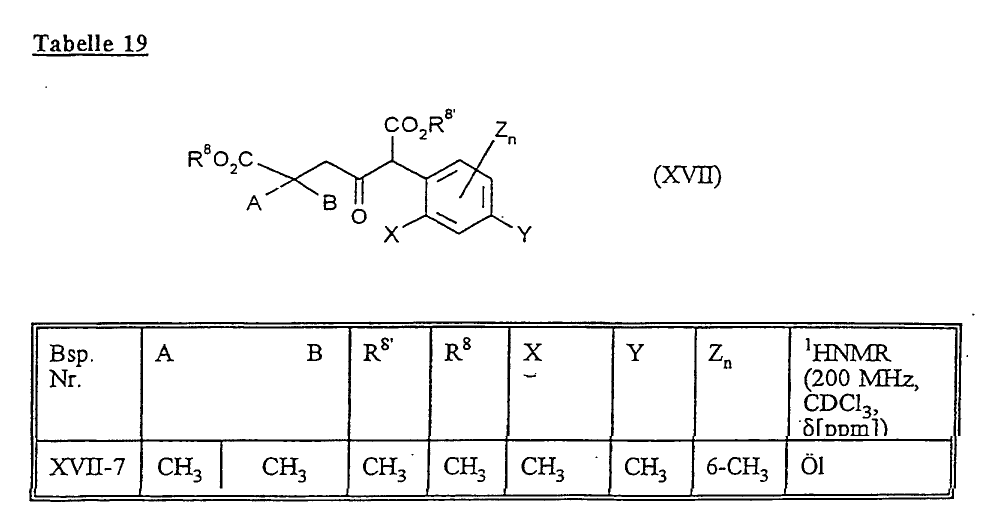

Die als Öle anfallenden Verbindungen der Formeln (XVII) und (XIV) wurden nicht weiter charakterisiert, sondern als Rohprodukte in die Umsetzungen zur Herstellung von Verbindungen der Formel (II) eingesetzt.

### Anwendungsbeispiele

### Beispiel A

| Tetranychus-Test (resistent) | | |
|---|---|---|
| Lösungsmittel | 3 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 1OO %, daß alle Spinnmilben abgetötet wurden; O % bedeutet, daß keine Spinnmilben abgetötet wurden.

In diesem Test bewirkten z.B. die Verbindungen gemäß den Herstellungsbeispielen Ia-3 bei einer beispielhaften Wirkstoffkonzentration von 0,01 % eine Abtötung von mindestens 80 % nach 13 Tagen.

### Beispiel B

| Phaedon-Larven-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

In diesem Test bewirkten z.B. die Verbindungen gemäß den Herstellungsbeispielen Ia-3, Ib-7, Ib-8, Ib-9, Ic-4 und Ic-5 bei einer beispielhaften Wirkstoffkonzentration von 0,1 % eine Abtötung von 100 % nach 7 Tagen.

### Beispiel C

| Plutella-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

In diesem Test bewirkten z.B. die Verbindungen gemäß den Herstellungsbeispielen Ia-3, Ib-7, Ib-8, Ib-9, Ic-4 und Ic-5 bei einer beispielhaften Wirkstoffkonzentration von 0,1 % eine Abtötung von 100 % nach 7 Tagen.

### Beispiel F

| Myzus-Test | | |
|---|---|---|
| Lösungsmittel | 7 Gewichtsteile | Dimethylformamid |
| Emulgator | 1 Gewichtsteil | Alkylarylpolyglykolether |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

In diesem Test bewirkte z.B. die Verbindung gemäß Herstellungsbeispiel Ib-7 bei einer beispielhaften Wirkstoffkonzentration von 0,1 % eine Abtötung von 98 % nach 6 Tagen.

### Beispiel H

| Test mit Boophilus microplus resistent / SP resistenter Parkhurst-Stamm | |
|---|---|
| Testtiere | Adulte gesogene Weibchen |
| Lösungsmittel | Dimethylsulfoxid |

20 mg Wirkstoff werden in 1 ml Dimethylsulfoxid gelöst, geringere Konzentrationen werden durch Verdünnen mit dem gleichen Lösungsmittel hergestellt.

Der Test wird in 5-fach-Bestimmung durchgeführt. 1 µl der Lösungen wird in das Abdomen injiziert, die Tiere in Schalen überführt und in einem klimatisierten Raum aufbewahrt. Die Wirkung wird über die Hemmung der Eiablage bestimmt. Dabei bedeutet 100 %, daß keine Zecke gelegt hat.

In diesem Test zeigten z.B. die Verbindungen gemäß den Herstellungsbeispielen Ib-6, Ic-4 und Ic-5 bei einer beispielhaften Wirkstoffkonzentration von 20 µg/Tier jeweils eine Wirkung von 100 %.

## Patentansprüche

1. Verbindungen der Formel (I) 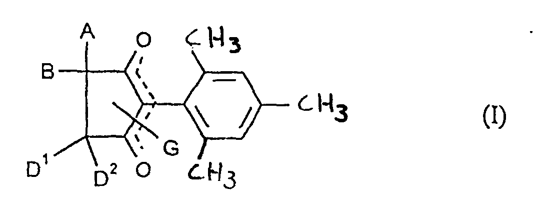 in welcher
A und B unabhängig voneinander für jeweils gegebenenfalls einfach oder mehrfach gleich oder verschieden durch Halogen substituiertes Alkyl,
D¹ und D² unabhängig voneinander für Wasserstoff, Halogen, gegebenenfalls durch Halogen substituiertes Alkyl oder gegebenenfalls substituiertes Phenyl stehen,
G für Wasserstoff (a) oder für eine der Gruppen
-CO-R¹ (b) 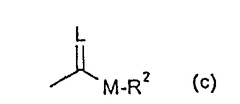
-SO₂-R³ (d) 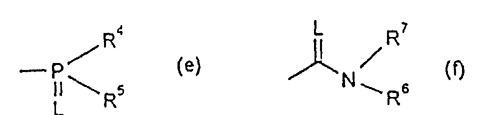
oder
E (g)
E für ein Metallionäquivalent oder ein Ammoniumion steht,
L für Sauerstoff oder Schwefel steht,
M für Sauerstoff oder Schwefel steht,
R¹ für jeweils gegebenenfalls einfach oder mehrfach gleich oder verschieden durch Halogen substituiertes Alkyl, Alkenyl, Alkoxyalkyl, Alkylthioalkyl, Polyalkoxyalkyl oder gegebenenfalls substituiertes, gesättigtes oder ungesättigtes Cycloalkyl steht, das durch mindestens ein Heteroatom unterbrochen sein kann oder für jeweils gegebenenfalls substituiertes Phenyl, Phenylalkyl, Hetaryl, Phenoxyalkyl oder Hetaryloxyalkyl steht,
R² für jeweils gegebenenfalls einfach oder mehrfach gleich oder verschieden durch Halogen substituiertes Alkyl, Alkenyl, Alkoxyalkyl, Polyalkoxyalkyl oder jeweils gegebenenfalls substituiertes Cycloalkyl, Phenyl oder Benzyl steht,
R³ für jeweils gegebenenfalls einfach oder mehrfach, gleich oder verschieden durch Halogen substituiertes Alkyl oder für jeweils gegebenenfalls substituiertes Phenyl oder Phenylalkyl steht,
R⁴ und R⁵ unabhängig voneinander für jeweils gegebenenfalls einfach oder mehrfach gleich oder verschieden durch Halogen substituiertes Alkyl, Alkoxy, Alkylamino, Alkenylamino, Dialkylamino, Dialkenylamino, Alkylthio, Alkenylthio, Alkinylthio, Cycloalkylthio oder für jeweils gegebenenfalls substituiertes Phenyl, Phenoxy oder Phenylthio stehen,
R⁶ und R⁷ unabhängig voneinander für Wasserstoff, jeweils gegebenenfalls einfach oder mehrfach gleich oder verschieden durch Halogen substituiertes Alkyl, Alkenyl, Alkoxy, Alkoxyalkyl, für gegebenenfalls substituiertes Phenyl oder für gegebenenfalls substituiertes Benzyl stehen,
oder
R⁶ und R⁷ zusammen für einen gegebenenfalls durch Sauerstoff oder Schwefel unterbrochenen Alkylenrest stehen,
sowie die enantiomerenreinen Formen von Verbindungen der Formel (I)

2. Verbindungen der Formel (I) gemäß Anspruch 1, welche unter Einbeziehung der verschiedenen Bedeutungen (a), (b), (c), (d), (e), (f) und (g) der Gruppe G folgende Strukturen (Ia) bis (Ig) besitzen: 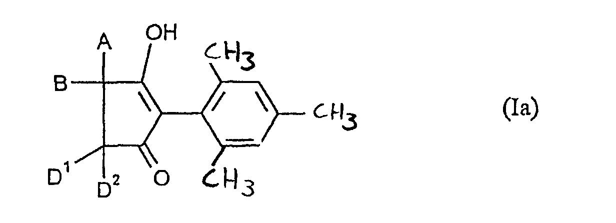 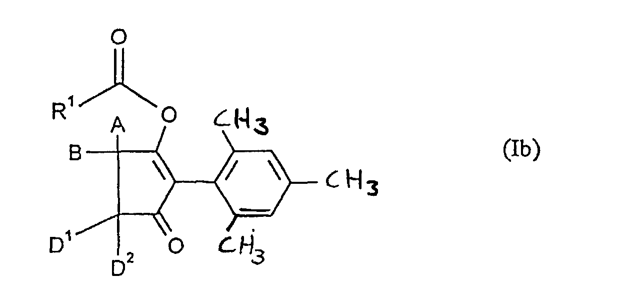 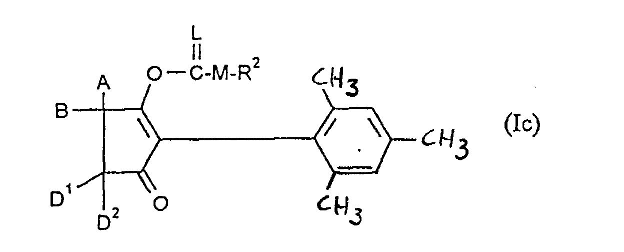 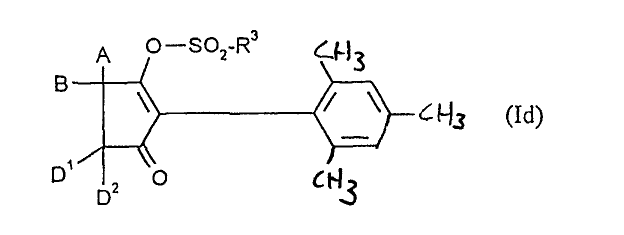 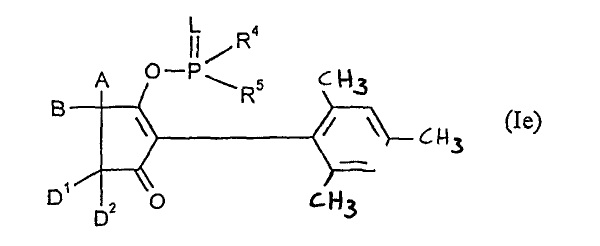 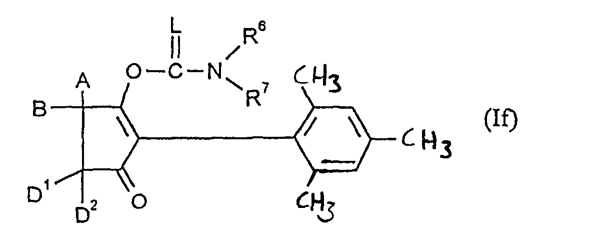 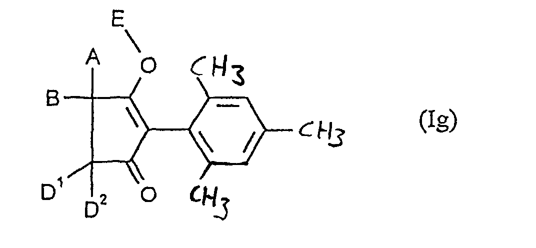 worin
A, B, D¹, D², E, L, M, R¹, R², R³, R⁴, R⁵, R⁶, R⁷ die in Anspruch 1 angegebenen Bedeutungen besitzen.

3. Verbindungen der Formel (I) gemäß Anspruch 1, in welcher
A und B unabhängig voneinander für jeweils gegebenenfalls einfach oder mehrfach, gleich oder verschieden durch Halogen substituiertes C₁-C₁₂-Alkyl,
D¹ und D² unabhängig voneinander für Wasserstoff, Halogen, gegebenenfalls einfach oder mehrfach, gleich oder verschieden durch Halogen substituiertes C₁-C₆-Alkyl oder für gegebenenfalls einfach oder mehrfach, gleich oder verschieden durch Halogen, C₁-C₆-Alkyl, C₁-C₆-Alkoxy, Nitro, C₁-C₆-Halogenalkyl oder C₁-C₆-Halogenalkoxy substituiertes Phenyl stehen,
G für Wasserstoff (a) oder für eine der Gruppen
-CO-R¹ ( (b) 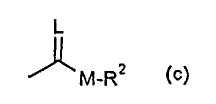
-SO₂-R³ (d) 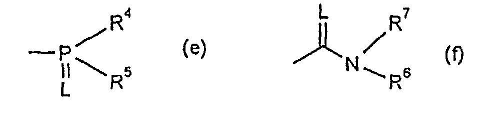
oder
E (g)
steht,
in welchen
E für ein Metallionäquivalent oder ein Ammoniumion steht,
L für Sauerstoff oder Schwefel steht,
M für Sauerstoff oder Schwefel steht,
R¹ für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₁-C₈-alkyl, C₁-C₈-Alkylthio-C₁-C₈-alkyl, PolyC₁-C₈-alkoxy-C₂-C₈-alkyl oder gegebenenfalls einfach oder mehrfach, gleich oder verschieden durch Halogen, C₁-C₆-Alkyl oder C₁-C₆-Alkoxy substituiertes, gesättigtes oder ungesättigtes Cycloalkyl mit 3 bis 8 Ringatomen, in welchem mindestens eine Methylengruppe durch ein Sauerstoff- und/oder Schwefelatom ersetzt sein kann,
für gegebenenfalls einfach oder mehrfach, gleich oder verschieden durch Halogen, Nitro, Cyano, C₁-C₆-Alkyl, C₁-C₆-Alkoxy, C₁-C₆-Halogenalkyl oder C₁-C₆-Halogenalkoxy substituiertes Phenyl,
für gegebenenfalls einfach oder mehrfach, gleich oder verschieden durch Halogen, C₁-C₆-Alkyl, C₁-C₆-Alkoxy, C₁-C₆-Halogenalkyl oder C₁-C₆-Halogenalkoxy substituiertes Phenyl-C₁-C₆-alkyl,
für gegebenenfalls einfach oder mehrfach, gleich oder verschieden durch Halogen oder C₁-C₆-Alkyl substituiertes fünf- oder sechsgliedriges Hetaryl,
für gegebenenfalls einfach oder mehrfach, gleich oder verschieden durch Halogen oder C₁-C₆-Alkyl substituiertes Phenoxy-C₁-C₆-alkyl oder
für gegebenenfalls einfach oder mehrfach, gleich oder verschieden durch Halogen, Amino oder C₁-C₆-Alkyl substituiertes fünf- oder sechsgliedriges Hetaryloxy-C₁-C₆-alkyl steht,
R² für jeweils gegebenenfalls einfach oder mehrfach, gleich oder verschieden durch Halogen substituiertes C₁-C₂₀-Alkyl, C₂-C₂₀-Alkenyl, C₁-C₈-Alkoxy-C₂-C₈-alkyl oder Poly-C₁-C₈-alkoxy-C₂-C₈-alkyl oder für gegebenenfalls einfach oder mehrfach, gleich oder verschieden durch Halogen, C₁-C₆-Alkyl oder C₁-C₆-Alkoxy substituiertes C₃-C₈-Cycloalkyl oder
für jeweils gegebenenfalls einfach oder mehrfach, gleich oder verschieden durch Halogen, Nitro, Cyano, C₁-C₆-Alkyl, C₁-C₆-Alkoxy oder C₁-C₆-Halogenalkyl substituiertes Phenyl oder Benzyl steht,
R³ für jeweils gegebenenfalls einfach oder mehrfach, gleich oder verschieden durch Halogen substituiertes C₁-C₁₂-Alkyl oder für jeweils gegebenenfalls einfach oder mehrfach, gleich oder verschieden durch Halogen, C₁-C₆-Alkyl, C₁-C₆-Alkoxy, C₁-C₃-Halogenalkyl, C₁-C₃-Halogenalkoxy, Cyano oder Nitro substituiertes Phenyl oder Phenyl-C₁-C₄-alkyl steht,
R⁴ und R⁵ unabhängig voneinander für jeweils gegebenenfalls einfach oder mehrfach, gleich oder verschieden durch Halogen substituiertes C₁-C₈-Alkyl, C₁-C₈-Alkoxy, C₁-C₈-Alkylamino, C₃-C₈-Alkenylamino, Di-(C₁-C₈-alkyl)-amino, Di-(C₃-C₈-alkenyl)-amino, C₁-C₈-Alkylthio, C₃-C₅-Alkenylthio, C₃-C₅-Alkinylthio, C₃-C₇-Cycloalkylthio oder für jeweils gegebenenfalls einfach oder mehrfach, gleich oder verschieden durch Halogen, Nitro, Cyano, C₁-C₄-Alkoxy, C₁-C₄-Halogenalkoxy, C₁-C₄-Alkylthio, C₁-C₄-Halogenalkylthio, C₁-C₄-Alkyl oder C₁-C₄-Halogenalkyl substituiertes Phenyl, Phenoxy oder Phenylthio stehen und
R⁶ und R⁷ unabhängig voneinander für Wasserstoff oder für jeweils gegebenenfalls einfach oder mehrfach, gleich oder verschieden durch Halogen substituiertes C₁-C₁₀-Alkyl, C₁-C₁₀-Alkoxy, C₃-C₈-Alkenyl oder C₁-C₁₀-Alkoxy-C₁-C₁₀-alkyl, für gegebenenfalls einfach oder mehrfach, gleich oder verschieden durch Halogen, C₁-C₃-Halogenalkyl, C₁-C₈-Alkyl oder C₁-C₈-Alkoxy substituiertes Phenyl, für gegebenenfalls einfach oder mehrfach, gleich oder verschieden durch Halogen, C₁-C₈-Alkyl, C₁-C₈-Halogenalkyl oder C₁-C₈-Alkoxy substituiertes Benzyl oder zusammen für einen C₂-C₆-Alkylenrest stehen, in welchem eine Methylengruppe durch Sauerstoff oder Schwefel ersetzt sein kann,
sowie die enantiomerenreinen Formen von Verbindungen der Formel (I).

4. Verbindungen der Formel (I) gemäß Anspruch 1, in welcher
A und B unabhängig voneinander für jeweils gegebenenfalls einfach bis fünffach, gleich oder verschieden durch Fluor oder Chlor substituiertes C₁-C₁₀-Alkyl,
D¹ und D² unabhängig voneinander für Wasserstoff, Fluor, Chlor, gegebenenfalls einfach bis fünffach, gleich oder verschieden durch Fluor oder Chlor substituiertes C₁-C₄-Alkyl oder für gegebenenfalls einfach bis dreifach, gleich oder verschieden durch Fluor, Chlor, Brom, C₁-C₄-Alkyl, C₁-C₄-Alkoxy, Nitro, C₁-C₂-Halogenalkyl oder C₁-C₂-Halogenalkoxy substituiertes Phenyl stehen,
G für Wasserstoff (a) oder für eine der Gruppen
-CO-R¹ (b) 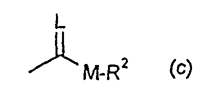
- SO₂-R³ (d) 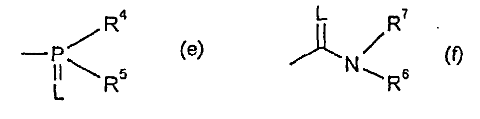
oder
E (g)
steht,
in welchen
E für ein Metallionäquivalent oder ein Ammoniumion steht,
L für Sauerstoff oder Schwefel steht,
M für Sauerstoff oder Schwefel steht,
R¹ für jeweils gegebenenfalls einfach bis neunfach, gleich oder verschieden durch Halogen substituiertes C₁-C₁₆-Alkyl, C₂-C₁₆-Alkenyl, C₁-C₆-Alkoxy-C₁-C₆-alkyl, C₁-C₆-Alkylthio-C₁-C₆-alkyl, Poly-C₁-C₆-alkoxy-C₂-C₆-alkyl oder gegebenenfalls einfach bis sechsfach, gleich oder verschieden durch Fluor, Chlor, C₁-C₄-Alkyl oder C₁-C₄-Alkoxy substituiertes, gesättigtes oder ungesättigtes Cycloalkyl mit 3 bis 7 Ringatomen, in welchem gegebenenfalls eine oder zwei Methylengruppen durch Sauerstoff- und/oder Schwefelatome ersetzt sind,
für gegebenenfalls einfach bis dreifach gleich oder verschieden durch Fluor, Chlor, Brom, Nitro, Cyano, C₁-C₄-Alkyl, C₁-C₄-Alkoxy, C₁-C₃-Halogenalkyl oder C₁-C₃-Halogenalkoxy substituiertes Phenyl,
für gegebenenfalls einfach bis dreifach, gleich oder verschieden durch Fluor, Chlor, Brom, C₁-C₄-Alkyl, C₁-C₄-Alkoxy, C₁-C₃-Halogenalkyl oder C₁-C₃-Halogenalkoxy substituiertes Phenyl-C₁-C₄-alkyl,
für jeweils gegebenenfalls einfach oder zweifach, gleich oder verschieden durch Fluor, Chlor oder C₁-C₆-Alkyl substituiertes Pyridyl, Thienyl, Furanyl, Pyrimidyl, Thiazolyl oder Pyrazolyl,
für gegebenenfalls einfach bis dreifach, gleich oder verschieden durch Fluor, Chlor, Brom oder C₁-C₄-Alkyl substituiertes Phenoxy-C₁-C₅-alkyl oder
für jeweils gegebenenfalls einfach oder zweifach, gleich oder verschieden durch Fluor, Chlor, Brom, Amino oder C₁-C₄-Alkyl substituiertes Pyridyloxy-, Pyrimidyloxy- oder Thiazolyloxy-C₁-C₅-alkyl steht,
R² für jeweils gegebenenfalls einfach bis neunfach, gleich oder verschieden durch Fluor oder Chlor substituiertes C₁-C₁₆-Alkyl, C₂-C₁₆-Alkenyl, C₁-C₆-Alkoxy-C₂-C₆-alkyl oder Poly-C₁-C₆-alkoxy-C₂-C₆-alkyl, für gegebenenfalls einfach bis fünffach, gleich oder verschieden durch Fluor, Chlor, C₁-C₄-Alkyl oder C₁-C₄-Alkoxy substituiertes C₃-C₇-Cycloalkyl oder
für jeweils gegebenenfalls einfach bis dreifach, gleich oder verschieden durch Fluor, Chlor, Brom, Nitro, Cyano, C₁-C₄-Alkyl, C₁-C₃-Alkoxy oder C₁-C₃-Halogenalkyl substituiertes Phenyl oder Benzyl steht,
R³ für jeweils gegebenenfalls einfach bis fünffach, gleich oder verschieden durch Fluor oder Chlor substituiertes C₁-C₈-Alkyl oder für jeweils gegebenenfalls einfach bis dreifach, gleich oder verschieden durch Fluor, Chlor, Brom, C₁-C₄-Alkyl, C₁-C₄-Alkoxy, C₁-C₂-Halogenalkyl, C₁-C₂-Halogenalkoxy, Cyano oder Nitro substituiertes Phenyl oder Phenyl-C₁-C₂-alkyl steht,
R⁴ und R⁵ unabhängig voneinander für jeweils gegebenenfalls einfach bis fünffach, gleich oder verschieden durch Fluor oder Chlor substituiertes C₁-C₆-Alkyl, C₁-C₆-Alkoxy, C₁-C₆-Alkylamino, C₃-C₆-Alkenylamino, Di-(C₁-C₆-alkyl)-amino, Di-(C₃-C₆-alkenyl)-amino, C₁-C₆-Alkylthio, C₃-C₄-Alkenylthio, C₃-C₄-Alkinylthio, C₃-C₆-Cycloalkylthio oder für jeweils gegebenenfalls einfach bis dreifach, gleich oder verschieden durch Fluor, Chlor, Brom, Nitro, Cyano, C₁-C₃-Alkoxy, C₁-C₃-Halogenalkoxy, C₁-C₃-Alkylthio, C₁-C₃-Halogenalkylthio, C₁-C₃-Alkyl oder C₁-C₃-Halogenalkyl substituiertes Phenyl, Phenoxy oder Phenylthio stehen und
R⁶ und R⁷ unabhängig voneinander für Wasserstoff oder für jeweils gegebenenfalls einfach bis fünffach, gleich oder verschieden durch Fluor oder Chlor substituiertes C₁-C₆-Alkyl, C₁-C₆-Alkoxy, C₃-C₆-Alkenyl oder C₁-C₆-Alkoxy-C₁-C₆-alkyl, für gegebenenfalls einfach bis dreifach, gleich oder verschieden durch Fluor, Chlor, C₁-C₃-Halogenalkyl, C₁-C₃-Alkyl oder C₁-C₃-Alkoxy substituiertes Phenyl, für gegebenenfalls einfach bis fünffach, gleich oder verschieden durch Fluor, Chlor, C₁-C₃-Alkyl, C₁-C₃-Halogenalkyl oder C₁-C₃-Alkoxy substituiertes Benzyl oder zusammen für einen C₄-C₆-Alkylenrest stehen, in welchem eine Methylengruppe durch Sauerstoff oder Schwefel ersetzt sein kann,
sowie die enantiomerenreinen Formen von Verbindungen der Formel (I).

5. Verbindungen der Formel (I) gemäß Anspruch 1, in welcher
A und B unabhängig voneinander für jeweils gegebenenfalls einfach bis dreifach, gleich oder verschieden durch Fluor oder Chlor substituiertes C₁-C₈-Alkyl,
D¹ und D² unabhängig voneinander für Wasserstoff, Fluor, Chlor, Methyl, Ethyl oder für gegebenenfalls einfach oder zweifach, gleich oder verschieden durch Fluor, Chlor, Methyl, Methoxy oder Trifluormethyl substituiertes Phenyl stehen,
G für Wasserstoff (a) oder für eine der Gruppen
-CO-R¹ (b) 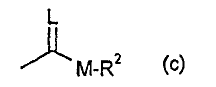
-SO₂-R³ (d) 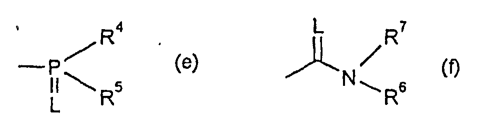
oder
E (g)
steht,
in welchen
E für ein Metallionäquivalent oder ein Ammoniumion steht,
L für Sauerstoff oder Schwefel steht,
M für Sauerstoff oder Schwefel steht,
R¹ für jeweils gegebenenfalls einfach bis dreifach, gleich oder verschieden durch Fluor oder Chlor substituiertes C₁-C₁₄-Alkyl, C₂-C₁₄-Alkenyl, C₁-C₄-Alkoxy-C₁-C₆-alkyl, C₁-C₄-Alkylthio-C₁-C₆-alkyl, Poly-C₁-C₄-alkoxy-C₂-C₄-alkyl oder gegebenenfalls einfach oder zweifach, gleich oder verschieden durch Fluor, Chlor, Methyl, Ethyl, Methoxy oder Ethoxy substituiertes Cycloalkyl mit 3 bis 6 Ringatomen, in welchem gegebenenfalls eine oder zwei Methylengruppen durch Sauerstoff- und/oder Schwefelatome ersetzt sind,
für gegebenenfalls einfach oder-zweifach, gleich oder verschieden durch Fluor, Chlor, Brom, Methyl, Ethyl, Propyl, i-Propyl, Methoxy, Ethoxy, Trifluormethyl, Trifluormethoxy, Cyano oder Nitro substituiertes Phenyl,
für gegebenenfalls einfach oder zweifach, gleich oder verschieden durch Fluor, Chlor, Brom, Methyl, Ethyl, Propyl, i-Propyl, Methoxy, Ethoxy, Trifluormethyl oder Trifluormethoxy substituiertes Pherlyl-C₁-C₃-alkyl, insbesondere Benzyl,
für jeweils gegebenenfalls einfach oder zweifach, gleich oder verschieden durch Fluor, Chlor, Brom, Methyl oder Ethyl substituiertes Pyridyl, Thienyl oder Furanyl,
für gegebenenfalls einfach oder zweifach, gleich oder verschieden durch Fluor, Chlor, Methyl oder Ethyl substituiertes PhenoxyC₁-C₄-alkyl oder
für jeweils gegebenenfalls einfach oder zweifach, gleich oder verschieden durch Fluor, Chlor, Amino, Methyl oder Ethyl substituiertes Pyridyloxy-C₁-C₃-alkyl, Pyrimidyloxy-C₁-C₃-alkyl oder Thiazolyloxy-C₁-C₃-alkyl steht,
R² für jeweils gegebenenfalls einfach bis fünffach, gleich oder verschieden durch Fluor oder Chlor substituiertes C₁-C₁₄-Alkyl, C₂-C₁₄-Alkenyl, C₁-C₄-Alkoxy-C₂-C₆-alkyl oder Poly-C₁-C₄-alkoxy-C₂-C₆-alkyl, für gegebenenfalls einfach bis dreifach, gleich oder verschieden durch Fluor, Chlor, Methyl, Ethyl, Methoxy oder Ethoxy substituiertes C₃-C₆-Cycloalkyl,
oder für jeweils gegebenenfalls einfach oder zweifach, gleich oder verschieden durch Fluor, Chlor, Nitro, Cyano, Methyl, Ethyl, Propyl, i-Propyl, Methoxy, Ethoxy oder Trifluormethyl substituiertes Phenyl oder Benzyl steht,
R³ für gegebenenfalls einfach bis dreifach, gleich oder verschieden durch Fluor oder Chlor substituiertes C₁-C₆-Alkyl oder für jeweils gegebenenfalls einfach oder zweifach, gleich oder verschieden durch Fluor, Chlor, Brom, Methyl, Methoxy, Trifluormethyl, Trifluormethoxy, Cyano oder Nitro substituiertes Phenyl oder Benzyl steht,
R⁴ und R⁵ unabhängig voneinander für jeweils gegebenenfalls einfach bis dreifach, gleich oder verschieden durch Fluor oder Chlor substituiertes C₁-C₄-Alkyl, C₁-C₄-Alkoxy, C₁-C₄-Alkylamino, C₃-C₄-Alkenylamino, Di-(C₁-C₄-alkyl)-amino, Di-(C₃-C₄-alkenyl)-amino, C₁-C₄-Alkylthio, für jeweils gegebenenfalls einfach oder zweifach, gleich oder verschieden durch Fluor, Chlor, Brom, Nitro, Cyano, C₁-C₂-Alkoxy, C₁-C₂-Fluoralkoxy, C₁-C₂-Chloralkoxy, C₁-C₂-Alkylthio, C₁-C₂-Fluoralkylthio, C₁-C₂-Chloralkylthio oder C₁-C₃-Alkyl substituiertes Phenyl, Phenoxy oder Phenylthio stehen und
R⁶ und R⁷ unabhängig voneinander für Wasserstoff oder für jeweils gegebenenfalls einfach bis dreifach, gleich oder verschieden durch Fluor oder Chlor substituiertes C₁-C₄-Alkyl, C₁-C₄-Alkoxy oder C₁-C₄-Alkoxy-C₁-C₄-alkyl, für gegebenenfalls einfach oder zweifach, gleich oder verschieden durch Fluor, Chlor, C₁-C₂-Halogenalkyl, C₁-C₂-Alkyl oder C₁-C₂-Alkoxy substituiertes Phenyl, für gegebenenfalls einfach oder zweifach, gleich oder verschieden durch Fluor, Chlor, Brom, C₁-C₂-Alkyl, C₁-C₂-Halogenalkyl oder C₁-C₂-Alkoxy substituiertes Benzyl oder zusammen für einen C₄-C₆-Alkylenrest stehen, in welchem eine Methylengruppe durch Sauerstoff oder Schwefel ersetzt sein kann,
sowie die enantiomerenreinen Formen von Verbindungen der Formel (I).

6. Verfahren zur Herstellung von Verbindungen der Formel (I) gemäß Anspruch 1, **dadurch gekennzeichnet, daß** man
(A)
zur Herstellung von Verbindungen der Formel (Ia) 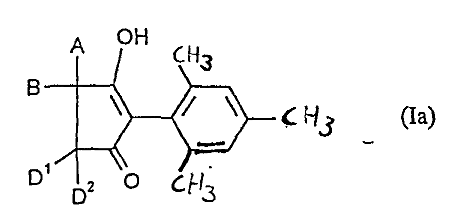 in welcher
A, B, D¹, D² die in Anspruch 1 angegebene Bedeutung haben,
Ketocarbonsäureester der Formel (II) 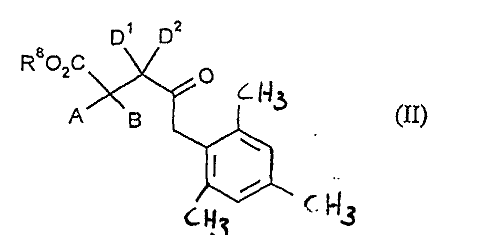 in welcher
A, B, D¹, D² die oben angegebene Bedeutung haben,
und
R⁸ für Alkyl steht,
gegebenenfalls in Gegenwart eines Verdünnungsmittels und in Gegenwart einer Base intramolekular cyclisiert und
(B)
zur Herstellung von Verbindungen der Formel (Ib) 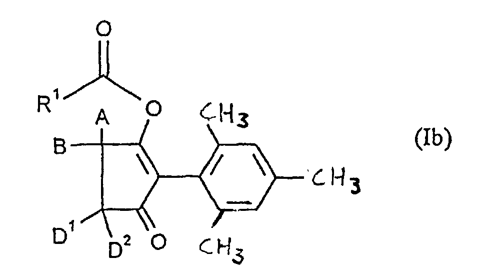
in welcher
A, B, D¹, D², R¹ die in Anspruch 1 angegebene Bedeutung haben,
Verbindungen der Formel (Ia) 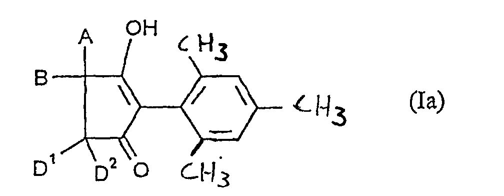 in welcher
A, B, D¹, D² die oben angegebene Bedeutung haben,
α) mit Säurehalogeniden der Formel (III) 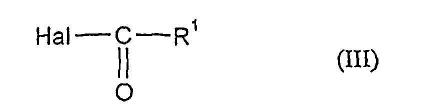 in welcher
R¹ die oben angegebene Bedeutung hat
und
Hal für Halogen steht,
gegebenenfalls in Gegenwart eines Verdünnungsmittels und gegebenenfalls in Gegenwart eines Säurebindemittels umsetzt
oder
β) mit Carbonsäureanhydriden der Formel (IV)
R¹-CO-O-CO-R¹- (IV)
in welcher
R¹ die oben angegebene Bedeutung hat,
gegebenenfalls in Gegenwart eines Verdünnungsmittels und gegebenenfalls in Gegenwart eines Säurebindemittels,
umsetzt, und
(C)
zur Herstellung von Verbindungen der Formel (Ic) 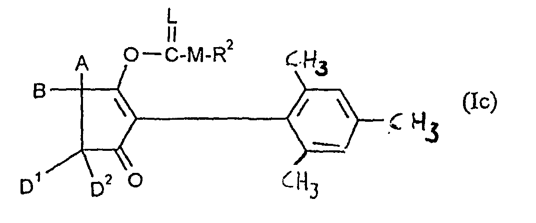 in welcher
A, B, D¹, D², R² die in Anspruch 1 angegebene Bedeutung haben,
L für Sauerstoff und
M für Sauerstoff oder Schwefel steht,
Verbindungen der Formel (Ia) 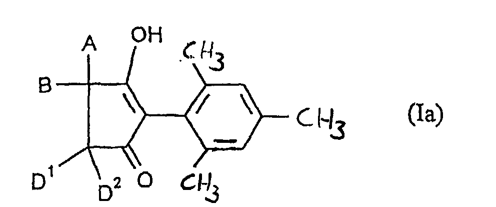 in welcher
A, B, D¹, D² die oben angegebene Bedeutung haben,
mit einem Chlorameisensäureester oder Chlorameisensäurethiolester der Formel (V)
R²-M-CO-Cl (V)
in welcher
R² und M die oben angegebene Bedeutung haben,
gegebenenfalls in Gegenwart eines Verdünnungsmittels und gegebenenfalls in Gegenwart eines Säurebindemittels umsetzt, und
(D)
zur Herstellung von Verbindungen der Formel (Ic) 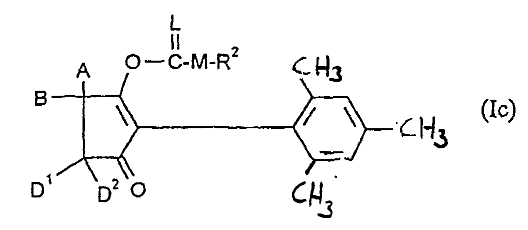 in welcher
A, B, D¹, D², R² die oben angegebene Bedeutung haben,
L für Schwefel und
M für Sauerstoff oder Schwefel steht,
Verbindungen der Formel (Ia) 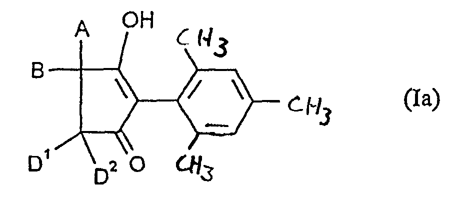 in welcher
A, B, D¹, D² die oben angegebene Bedeutung haben,
α) mit einem Chlormonothioameisensäureester oder Chlordithioameisensäureester der Formel (VI) 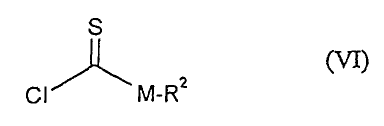 in welcher
M und R² die oben angegebene Bedeutung haben,
gegebenenfalls in Gegenwart eines Verdünnungsmittels und gegebenenfalls in Gegenwart eines Säurebindemittels umsetzt, oder
β) mit Schwefelkohlenstoff und anschließend mit Alkylhalogeniden der Formel (VII)
R²-Hal (VII)
in welcher
R² die oben angegebene Bedeutung hat
und
Hal für Chlor, Brom oder Iod steht,
gegebenenfalls in Gegenwart eines Verdünnungsmittels und gegebenenfalls in Gegenwart einer Base umsetzt und
(E)
zur Herstellung von Verbindungen der Formel (Id) 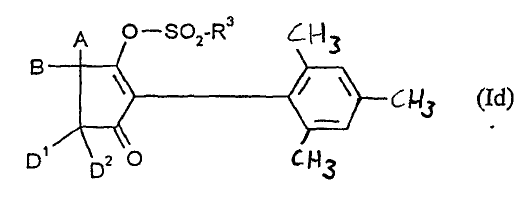 in welcher
A, B, D¹, D², R³ die in Anspruch 1 angegebene Bedeutung haben,
Verbindungen der Formel (Ia) 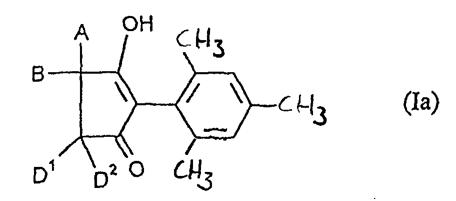 in welcher
A, B, D¹, D², die oben angegebene Bedeutung haben,
mit Sulfonsäurechloriden der Formel (VIII)
R³-SO₂-Cl (VIII)
in welcher
R³ die oben angegebene Bedeutung hat,
gegebenenfalls in Gegenwart eines Verdünnungsmittels und gegebenenfalls in Gegenwart eines Säurebindemittels umsetzt, und
(F)
zur Herstellung von Verbindungen der Formel (Ie) 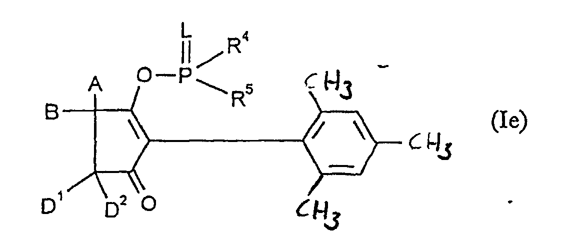 in welcher
A, B, D¹, D², L, R⁴, R⁵ die in Anspruch 1 angegebene Bedeutung haben,
Verbindungen der Formel (Ia) 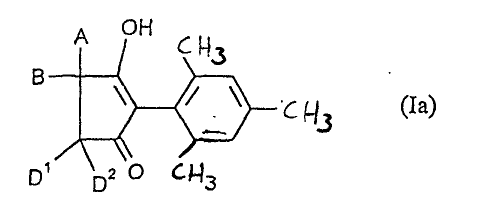 in welcher
A, B, D¹, D², die oben angegebene Bedeutung haben,
mit Phosphorverbindungen der Formel (IX) 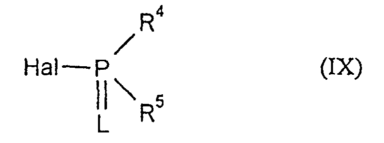 in welcher
L, R⁴ und R⁵ die oben angegebene Bedeutung haben und
Hal für Halogen steht,
gegebenenfalls in Gegenwart eines Verdünnungsmittels und gegebenenfalls in Gegenwart eines Säurebindemittels umsetzt, und
(G)
zur Herstellung von Verbindungen der Formel (If) 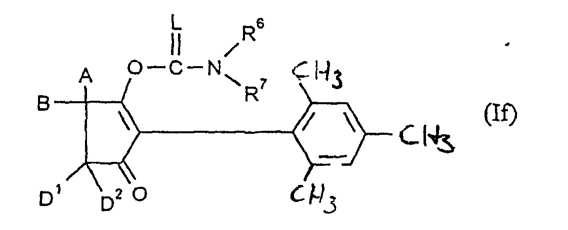 in welcher
A, B, D¹, D², L, R⁶, R⁷ die in Anspruch 1 angegebene Bedeutung haben,
Verbindungen der Formel (Ia) 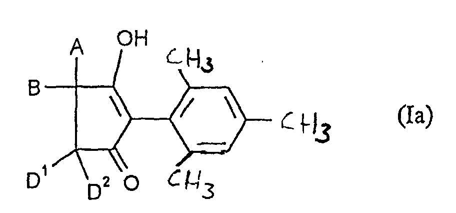 in welcher
A, B, D¹, D², X, Y, Z und n die oben angegebene Bedeutung haben,
α) mit Isocyanaten oder Isothiocyanaten der Formel (X)
R⁶-N=C=L (X)
in welcher
R⁶ und L die oben angegebene Bedeutung haben,
gegebenenfalls in Gegenwart eines Verdünnungsmittels und gegebenenfalls in Gegenwart eines Katalysators umsetzt, oder
β) mit Carbamidsäurechloriden oder Thiocarbamidsäurechloriden der Formel (XI) 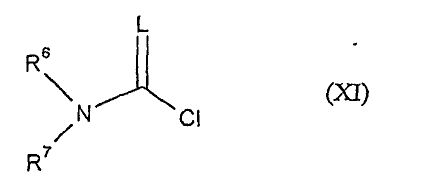
in welcher
L, R⁶ und R⁷ die oben angegebene Bedeutung haben,
gegebenenfalls in Gegenwart eines Verdünnungsmittels und gegebenenfalls in Gegenwart eines Säurebindemittels umsetzt, und
(H)
zur Herstellung von Verbindungen der Formel (Ig) 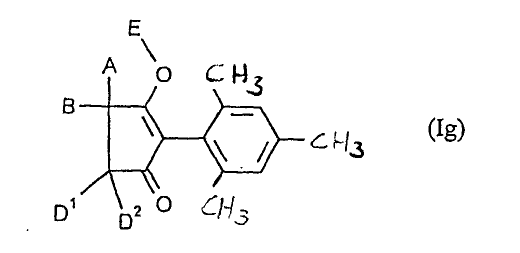 in welcher
D¹, D², A, B die oben angegebene Bedeutung haben,
und
E für ein Metallionäquivalent oder ein Ammoniumion steht,
Verbindungen der Formel (Ia) 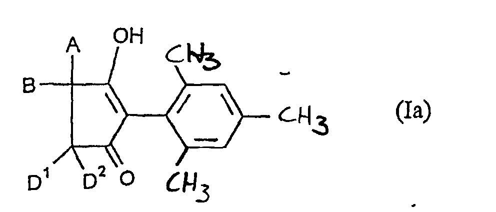 in welcher
A, B, D¹, D² die oben angegebene Bedeutung haben,
mit Metallverbindungen der Formel (XII) oder Aminen der Formel (XIII)
MeR⁹ₜ (XII) 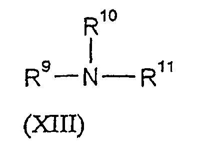
in welchen
Me für ein- oder zweiwertige Metallionen und
t für die Zahl 1 oder 2 steht und
R⁹, R¹⁰ und R¹¹ unabhängig voneinander für Wasserstoff, Alkyl, Alkoxy oder Hydroxy stehen,
gegebenenfalls in Gegenwart eines Verdünnungsmittels, umsetzt.

7. Verbindungen der Formel (II) 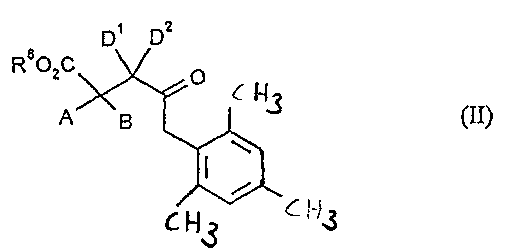 in welcher
A, B, D¹, D², die in Anspruch 1 angegebene Bedeutung haben und
R⁸ für Alkyl steht.

8. Verfahren zur Herstellung von Verbindungen der Formel (II) gemäß Anspruch 7, **dadurch gekennzeichnet, daß** man Verbindungen der Formel (XIV) 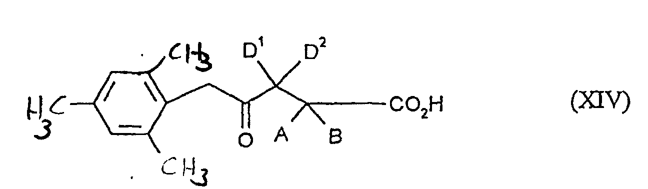 in welcher
A, B, D¹, D² die in Anspruch 1 angegebene Bedeutung haben,
verestert.

9. Verbindungen der Formel (XIV) 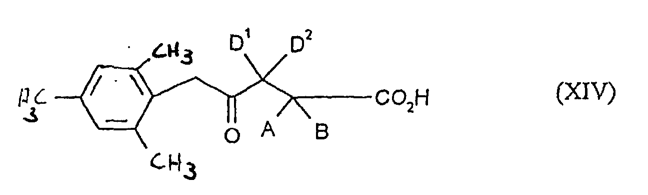 in welcher
A, B, D¹, D² die in Anspruch 1 angegebene Bedeutung haben.

10. Verfahren zur Herstellung von Verbindungen der Formel (XIV) gemäß Anspruch 9, **dadurch gekennzeichnet, daß** man entweder Carbonsäureanhydride der Formel (XV) 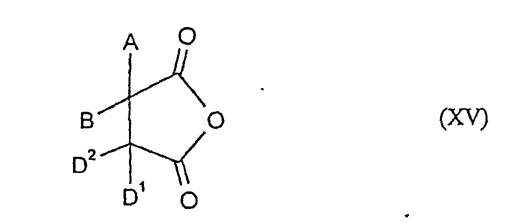 in welcher
A, B und D die oben angegebene Bedeutung haben,
mit metallorganischen Verbindungen der Formel (XVI) 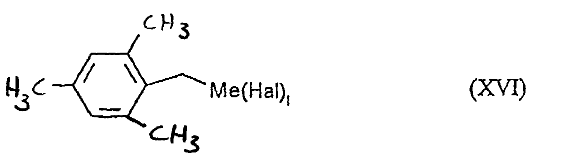 in welcher
Me für ein- oder zweiwertige Metallionen,
Hal für Chlor oder Brom
und
l für die Zahl 0 oder 1 steht,
in Gegenwart eines Verdünnungsmittels umsetzt,
oder
Verbindungen der Formel (XVII) 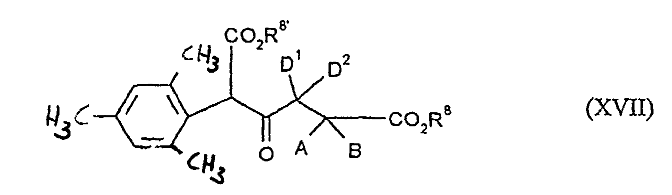 in welcher
A, B, D¹, D², die oben angegebene Bedeutung haben und
R⁸ und R^{8'} für Alkyl stehen,
gegebenenfalls in Gegenwart eines Verdünnungsmittels und gegebenenfalls in Gegenwart einer Base oder Säure decarboxyliert.

11. Schädlingsbekämpfungsmittel und Herbizide, **gekennzeichnet durch** einen Gehalt an mindestens einer Verbindung der Formel (I) gemäß Anspruch 1.

12. Verwendung von Verbindungen der Formel (I) gemäß Anspruch 1 zur Bekämpfung von tierischen Schädlingen und unerwünschtem Pflanzenbewuchs.

13. Verfahren zur Bekämpfung von tierischen Schädlingen und unerwünschtem Bewuchs, **dadurch gekennzeichnet, daß** man Verbindungen der Formel (I) gemäß Anspruch 1 auf die Schädlinge, Pflanzen und/oder deren Lebensraum ausbringt.

14. Verfahren zur Herstellung von Schädlingsbekämpfungsmitteln und Herbiziden, **dadurch gekennzeichnet, daß** man Verbindungen der Formel (I) gemäß Anspruch 1 mit Streckmitteln und/oder oberflächenaktiven Stoffen vermischt.

15. Verwendung von Verbindungen der Formel (I) gemäß Anspruch 1 zur Herstellung von Schädlingsbekämpfungsmitteln und Herbiziden.

## Claims

1. Compounds of the formula (I) 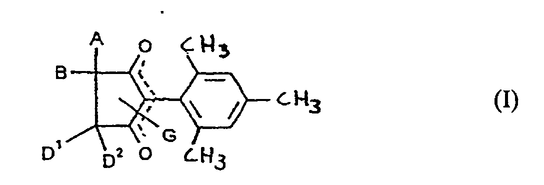 in which
A and B independently of one another represent alkyl in each case optionally substituted once or several times in an identical or different manner by halogen,
D¹ and D² independently of one another represent hydrogen, halogen or alkyl which is optionally substituted by halogen or optionally substituted phenyl,
G represents hydrogen (a), or represents one of the groups
-CO-R¹ (b) 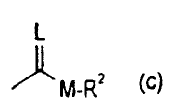
-SO₂-R³ (d) 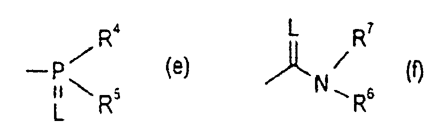
or
E (g)
E represents a metal ion equivalent or an ammonium ion,
L represents oxygen or sulphur,
M represents oxygen or sulphur,
R¹ represents alkyl, alkenyl, alkoxyalkyl, alkylthioalkyl or polyalkoxyalkyl, in each case optionally substituted once or several times in an identical or different manner by halogen, or optionally substituted, saturated or unsaturated cycloalkyl, which can be interrupted by at least one heteroatom, or represents in each case optionally substituted phenyl, phenylalkyl, hetaryl, phenoxyalkyl or hetaryloxyalkyl,
R² represents alkyl, alkenyl, alkoxyalkyl or polyalkoxyalkyl, in each case optionally substituted once or several times in an identical or different manner by halogen, or in each case optionally substituted cycloalkyl, phenyl or benzyl,
R³ represents alkyl which is in each case optionally substituted once or several times in an identical or different manner by halogen, or represents in each case optionally substituted phenyl or phenylalkyl,
R⁴ and R⁵ independently of one another represent alkyl, alkoxy, alkylamino, alkenylamino, dialkylamino, dialkenylamino, alkylthio, alkenylthio, alkinylthio or cycloalkylthio, in each case optionally substituted once or several times in an identical or different manner by halogen, or represent in each case optionally substituted phenyl, phenoxy or phenylthio,
R⁶ and R⁷ independently of one another represent hydrogen or alkyl, alkenyl, alkoxy or alkoxyalkyl, in each case optionally substituted once or several times in an identical or different manner by halogen, or represent optionally substituted phenyl or represent optionally substituted benzyl,
or
R⁶ and R⁷ together represent an alkylene radical which is optionally interrupted by oxygen or sulphur,
and the enantiomerically pure forms of compounds of the formula (I).

2. Compounds ofthe formula (I) according to Claim 1, which, incorporating the various meanings (a), (b), (c), (d), (e), (f) and (g) ofthe group G, have the following structures (Ia) to (Ig): 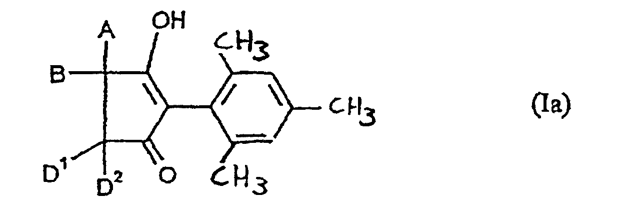 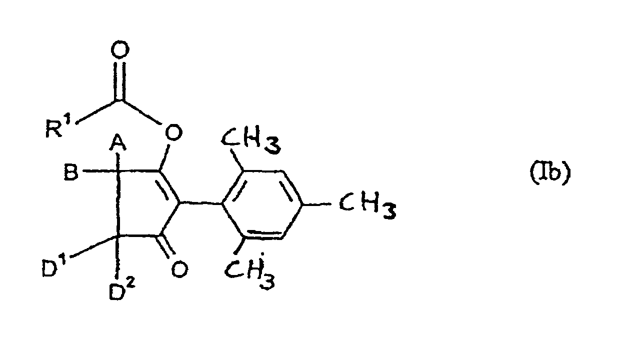 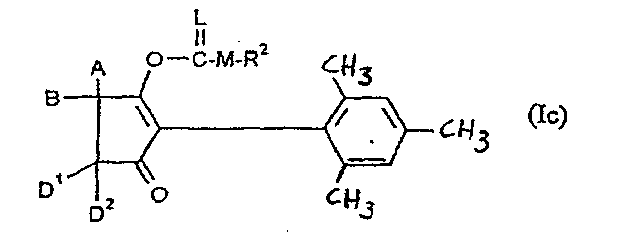 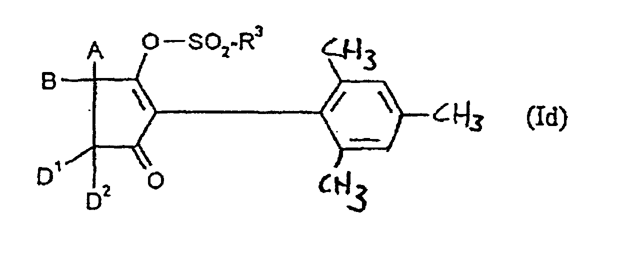 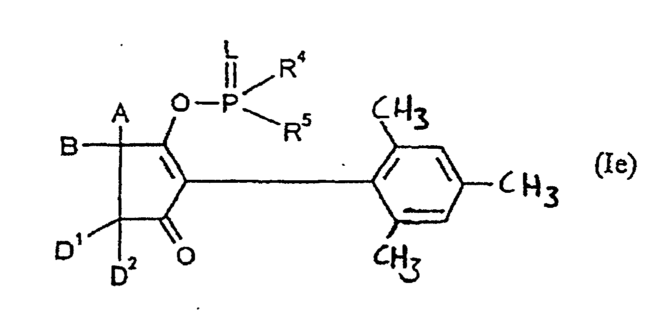 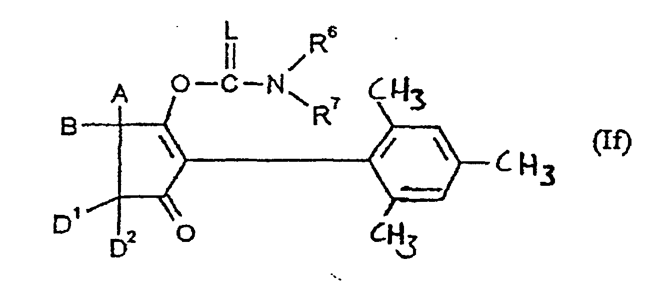 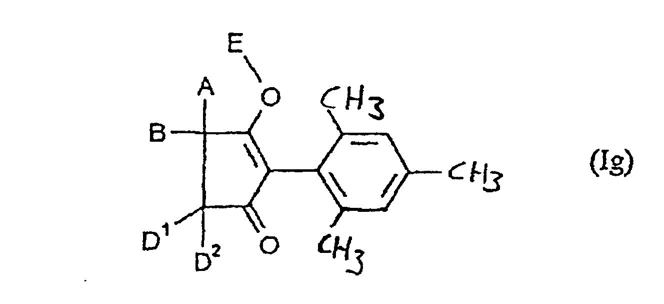 wherein
A, B, D¹, D², E, L, M, R¹, R², R³, R⁴, R⁵, R⁶, R⁷ have the meanings given in Claim 1.

3. Compounds of the formula (I) according to Claim 1, in which
A and B independently of one another represent C₁-C₁₂-alkyl in each case optionally substituted once or several times in an identical or different manner by halogen,
D¹ and D² independently of one another represent hydrogen, halogen or C₁-C₆-alkyl which is optionally substituted once or several times in an identical or different manner by halogen, or represent phenyl which is optionally substituted once or several times in an identical or different manner by halogen, C₁-C₆-alkyl, C₁-C₆-alkoxy, nitro, C₁-C₆-halogenoalkyl or C₁-C₆-halogenoalkoxy,
G represents hydrogen (a), or represents one of the groups
-CO-R¹ (b) 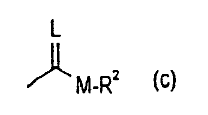
-SO₂-R³ (d) 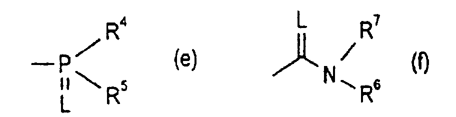
or
E (g)
in which
E represents a metal ion equivalent or an ammonium ion,
L represents oxygen or sulphur,
M represents oxygen or sulphur,
R¹ represents C₁-C₂₀-alkyl, C₂-C₂₀-alkenyl, C₁-C₈-alkoxy-C₁-C₈-alkyl, C₁-C₈-alkylthio-C₁-C₈-alkyl or poly-C₁-C₈-alkoxy-C₂-C₈-alkyl, in each case optionally substituted once or several times in an identical or different manner by halogen, or saturated or unsaturated cycloalkyl having 3 to 8 ring atoms, which is optionally substituted once or several times in an identical or different manner by halogen, C₁-C₆-alkyl or C₁-C₆-alkoxy and in which at least one methylene group can be replaced by an oxygen and/or sulphur atom,
or represents phenyl which is optionally substituted once or several times in an identical or different mannerby halogen, nitro, cyano, C₁-C₆-alkyl, C₁-C₆-alkoxy, C₁-C₆-halogenoalkyl or C₁-C₆-halogenoalkoxy,
or represents phenyl-C₁-C₆-alkyl which is optionally substituted once or several times in an identical or different manner by halogen, C₁-C₆-alkyl, C₁-C₆-alkoxy, C₁-C₆-halogenoalkyl or C₁-C₆-halogenoalkoxy,
or represents hetaryl which has 5 or 6 ring atoms and is optionally substituted once or several times in an identical or different manner by halogen or C₁-C₆-alkyl,
or represents phenoxy-C₁-C₆-alkyl which is optionally substituted once or several times in an identical or different manner by halogen or C₁-C₆-alkyl,
or represents hetaryloxy-C₁-C₆-alkyl which has 5 or 6 ring atoms and is optionally substituted once or several times in an identical or different manner by halogen, amino or C₁-C₆-alkyl,
R² represents C₁-C₂₀-alkyl, C₂-C₂₀-alkenyl, C₁-C₈-alkoxy-C₂-C₈-alkyl or poly-C₁-C₈-alkoxy-C₂-C₈-alkyl, in each case optionally substituted once or several times in an identical or different manner by halogen, or represents C₃-C₈-cycloalkyl, which is optionally substituted once or several times in an identical or different manner by halogen, C₁-C₆-alkyl or C₁-C₆-alkoxy, or represents phenyl or benzyl, in each case optionally substituted once or several times in an identical or different manner by halogen, nitro, cyano, C₁-C₆-alkyl, C₁-C₆-alkoxy or C₁-C₆-halogenoalkyl,
R³ represents C₁-C₁₂-alkyl which is in each case optionally substituted once or several times in an identical or different manner by halogen, or represents phenyl or phenyl-C₁-C₄-alkyl, in each case optionally substituted once or several times in an identical or different manner by halogen, C₁-C₆-alkyl, C₁-C₆-alkoxy, C₁-C₃-halogenoalkyl, C₁-C₃-halogenoalkoxy, cyano or nitro,
R⁴ and R⁵ independently ofone another represent C₁-C₈-alkyl, C₁-C₈-alkoxy, C₁-C₈-alkylamino, C₃-C₈-alkenylamino, di-(C₁-C₈-alkyl)-amino, di-(C₃-C₈-alkenyl)-amino, C₁-C₈-alkylthio, C₃-C₅-alkenylthio, C₃-C₅-alkinylthio or C₃-C₇-cycloalkylthio, in each case optionally substituted once or several times in an identical or different manner by halogen, or represent phenyl, phenoxy or phenylthio, in each case optionally substituted once or several times in an identical or different manner by halogen, nitro, cyano, C₁-C₄-alkoxy, C₁-C₄-halogenoalkoxy, C₁-C₄-alkylthio, C₁-C₄-halogenoalkylthio, C₁-C₄-alkyl or C₁-C₄-halogenoalkyl and
R⁶ and R⁷ independently ofone another represent hydrogen, or represent C₁-C₁₀-alkyl, C₁-C₁₀-alkoxy, C₃-C₈-alkenyl or C₁-C₁₀-alkoxy-C₁-C₁₀-alkyl, in each case optionally substituted once or several times in an identical or different manner by halogen, or represent phenyl which is optionally substituted once or several times in an identical or different manner by halogen, C₁-C₃-halogenoalkyl, C₁-C₈-alkyl or C₁-C₈-alkoxy, or represent benzyl which is optionally substituted once or several times in an identical or different manner by halogen, C₁-C₈-alkyl, C₁-C₈-halogenoalkyl or C₁-C₈-alkoxy, or together represent a C₂-C₆-alkylene radical, in which a methylene group can be replaced by oxygen or sulphur and the enantiomerically pure forms of compounds of the formula (I).

4. Compounds of the formula (I) according to Claim 1, in which
A and B independently ofone another represent C₁-C₁₀-alkyl in each case optionally substituted once to five times in an identical or different manner by fluorine or chlorine,
D¹ and D² independently of one another represent hydrogen, fluorine, chlorine or C₁-C₄-alkylwhich is optionally substituted once to five times in an identical or different manner by fluorine or chlorine, or represent phenyl which is optionally substituted once to three times in an identical or different manner by fluorine, chlorine, bromine, C₁-C₄-alkyl, C₁-C₄-alkoxy, nitro, C₁-C₂-halogenoalkyl or C₁-C₂-halogenoalkoxy,
G represents hydrogen (a), or represents one of the groups
-CO-R¹ (b) 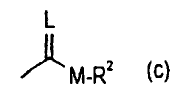
-SO₂-R³ (d) 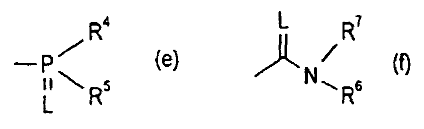
or
E (g)
in which
E represents a metal ion equivalent or an ammonium ion,
L represents oxygen or sulphur,
M represents oxygen or sulphur,
R¹ represents C₁-C₁₆-alkyl, C₂-C₁₆-alkenyl, C₁-C₆-alkoxy-C₁-C₆-alkyl, C₁-C₆-alkylthio-C₁-C₆-alkyl or poly-C₁-C₆-alkoxy-C₂-C₆-alkyl, in each case optionally substituted once to nine times in an identical or different manner by halogen, or cycloalkyl which has 3 to 7 ring atoms, is optionally substituted once or six times in an identical or different manner by fluorine, chlorine, C₁-C₄-alkyl or C₁-C₄-alkoxy, and in which one or two methylene groups are optionally replaced by oxygen and/or sulphur atoms,
or represents phenyl which is optionally substituted once to three times in an identical or different manner by fluorine, chlorine, bromine, nitro, cyano, C₁-C₄-alkyl, C₁-C₄-alkoxy, C₁-C₃-halogenoalkyl or C₁-C₃-halogenoalkoxy,
or represents phenyl-C₁-C₄-alkyl which is optionally substituted once to three times in an identical or different manner by fluorine, chlorine, bromine, C₁-C₄-alkyl, C₁-C₄-alkoxy, C₁-C₃-halogenoalkyl or C₁-C₃-halogenoalkoxy,
or represents pyridyl, thienyl, furanyl, pyrimidyl, thiazolyl or pyrazolyl, in each case optionally substituted once or twice in an identical or different manner by fluorine, chlorine or C₁-C₆-alkyl,
or represents phenoxy-C₁-C₅-alkyl which is optionally substituted once to three times in an identical or different manner by fluorine, chlorine, bromine or C₁-C₄-alkyl,
or represents pyridyloxy-C₁-C₅-alkyl, pyrimidyloxy-C₁-C₅-alkyl or thiazolyloxy-C₁-C₅-alkyl, in each case optionally substituted once or twice in an identical or different manner by fluorine, chlorine, bromine, amino or C₁-C₄-alkyl,
R² represents C₁-C₆-alkyl, C₂-C₁₆-alkenyl, C₁-C₆-alkoxy-C₂-C₆-alkyl or poly-C₁-C₆-alkoxy-C₂-C₆-alkyl, in each case optionally substituted once to nine times in an identical or different manner by fluorine or chlorine, or represents C₃-C₇-cycloalkyl which is optionally substituted once to five times in an identical or different manner by fluorine, chlorine, C₁-C₄-alkyl or C₁-C₄-alkoxy,
or represents phenyl or benzyl, in each case optionally substituted once to three times in an identical or different manner by fluorine, chlorine, bromine, nitro, cyano, C₁-C₄-alkyl, C₁-C₃-alkoxy or C₁-C₃-halogenoalkyl,
R³ represents C₁-C₈-alkyl which is in each case optionally substituted once to five times in an identical or different manner by fluorine or chlorine, or represents phenyl orphenyl-C₁-C₂-alkyl, in each case optionally substituted once to three times in an identical or different manner by fluorine, chlorine, bromine, C₁-C₄-alkyl, C₁-C₄-alkoxy, C₁-C₂-halogenoalkyl, C₁-C₂-halogenoalkoxy, cyano or nitro,
R⁴ and R⁵ independently ofone another represent C₁-C₆-alkyl, C₁-C₆-alkoxy, C₁-C₆-alkylamino, C₃-C₆-alkenylamino, di-(C₁-C₆-alkyl)-amino, di-(C₃-C₆-alkenyl)-amino, C₁-C₆-alkylthio, C₃-C₄-alkenylthio, C₃-C₄-alkinylthio or C₃-C₆-cycloalkylthio, in each case optionally substituted once to five times in an identical or different manner by fluorine or chlorine, or represent phenyl, phenoxy or phenylthio, in each case optionally substituted once to three times in an identical or different manner by fluorine, chlorine, bromine, nitro, cyano, C₁-C₃-alkoxy, C₁-C₃-halogenoalkoxy, C₁-C₃-alkylthio, C₁-C₃-halogenoalkylthio, C₁-C₃-alkyl or C₁-C₃-halogenoalkyl and
R⁶ and R⁷ independently of one another represent hydrogen, or represent C₁-C₆-alkyl, C₁-C₆-alkoxy, C₃-C₆-alkenyl or C₁-C₆-alkoxy-C₁-C₆-alkyl, in each case optionally substituted once to five times in an identical or different manner by fluorine or chlorine, or represent phenyl which is optionally substituted once to three times in an identical or different manner by fluorine, chlorine, C₁-C₃-halogenoalkyl, C₁-C₃-alkyl or C₁-C₃-alkoxy, or represent benzyl which is optionally substituted once to five times in an identical or different manner by fluorine, chlorine, C₁-C₃-alkyl, C₁-C₃-halogenoalkyl or C₁-C₃-alkoxy, or together represent a C₄-C₆-alkylene radical, in which amethylene group can be replaced by oxygen or sulphur,
and the enantiomerically pure forms of compounds of the formula (I).

5. Compounds of the formula (I) according to Claim 1, in which
A and B independently of one another represent C₁-C₈-alkyl in each case optionally substituted once to three times in an identical or different manner by fluorine or chlorine,
D¹ and D² independently of one another represent hydrogen, fluorine, chlorine, methyl or ethyl, or represent phenyl which is optionally substituted once or twice in an identical or different manner by fluorine, chlorine, methyl, methoxy or trifluoromethyl,
G represents hydrogen (a), or represents one of the groups
-CO-R¹ (b) 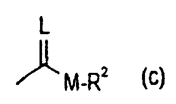
-SO₂-R³ (d) 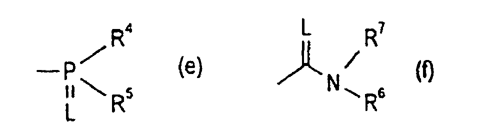
or
E (g)
in which
E represents a metal ion equivalent or an ammonium ion,
L represents oxygen or sulphur,
M represents oxygen or sulphur,
R¹ represents C₁-C₁₄-alkyl, C₂-C₁₄-alkenyl, C₁-C₄-alkoxy-C₁-C₆-alkyl, C₁-C₄-alkylthio-C₁-C₆-alkyl or poly-C₁-C₄-alkoxy-C₂-C₄-alkyl, in each case optionally substituted once to three times in an identical or different manner by fluorine or chlorine, or cycloalkyl which has 3 to 6 ring atoms, is optionally substituted once or twice in an identical or different manner by fluorine, chlorine, methyl, ethyl, methoxy or ethoxy, and in which one or two methylene groups are optionally replaced by oxygen and/or sulphur atoms,
orrepresents phenylwhich is optionally substituted once or twice in an identical or different manner by fluorine, chlorine, bromine, methyl, ethyl, propyl, i-propyl, methoxy, ethoxy, trifluoromethyl, trifluoromethoxy, cyano or nitro,
or represents phenyl-C₁-C₃-alkyl, in particular benzyl, which is optionally substituted once or twice in an identical or different manner by fluorine, chlorine, bromine, methyl, ethyl, propyl, i-propyl, methoxy, ethoxy, trifluoromethyl or trifluoromethoxy,
or represents pyridyl, thienyl or furanyl, in each case optionally substituted once or twice in an identical or different manner by fluorine, chlorine, bromine, methyl or ethyl,
or represents phenoxy-C₁-C₄-alkyl which is optionally substituted once or twice in an identical or different manner by fluorine, chlorine, methyl or ethyl,
or represents pyridyloxy-C₁-C₃-alkyl, pyrimidyloxy-C₁-C₃-alkyl or thiazolyloxy-C₁-C₃-alkyl, in each case optionally substituted once or twice in an identical or different manner by fluorine, chlorine, amino, methyl or ethyl,
R² represents C₁-C₁₄-alkyl, C₂-C₁₄-alkenyl, C₁-C₄-alkoxy-C₂-C₆-alkyl or poly-C₁-C₄-alkoxy-C₂-C₆-alkyl, in each case optionally substituted once to five times in an identical or different manner by fluorine or chlorine, or represents C₃-C₆-cycloalkyl which is optionally substituted once to three times in an identical or different manner by fluorine, chlorine, methyl, ethyl, methoxy or ethoxy,
orrepresents phenyl or benzyl, in each case optionally substituted once ortwice in an identical or different manner by fluorine, chlorine, nitro, cyano, methyl, ethyl, propyl, i-propyl, methoxy, ethoxy or trifluoromethyl,
R³ represents C₁-C₆-alkyl which is optionally substituted once to three times in an identical or different manner by fluorine or chlorine, or represents phenyl or benzyl, in each case optionally substituted once or twice in an identical or different manner by fluorine, chlorine, bromine, methyl, methoxy, trifluoromethyl, trifluoromethoxy, cyano or nitro,
R⁴ and R⁵ independently of one another represent C₁-C₄-alkyl, C₁-C₄-alkoxy, C₁-C₄-alkylamino, C₃-C₄-alkenylamino, di-(C₁-C₄-alkyl)-amino, di-(C₃-C₄-alkenyl)-amino, C₁-C₄-alkylthio, in each case optionally substituted once to three times in an identical or different manner by fluorine or chlorine, or represent phenyl, phenoxy or phenylthio, in each case optionally substituted once or twice in an identical or different manner by fluorine, chlorine, bromine, nitro, cyano, C₁-C₂-alkoxy, C₁-C₂-fluoroalkoxy, C₁-C₂-chloroalkoxy, C₁-C₂-alkylthio, C₁-C₂-fluoroalkylthio, C₁-C₂-chloroalkylthio or C₁-C₃-alkyl, and
R⁶ and R⁷ independently of one another represent hydrogen, or represent C₁-C₄-alkyl, C₁-C₄-alkoxy or C₁-C₄-alkoxy-C₁-C₄-alkyl, in each case optionally substituted once to three times in an identical or different manner by fluorine or chlorine, or represent phenyl which is optionally substituted once or twice in an identical or different manner by fluorine, chlorine, C₁-C₂-halogenoalkyl, C₁-C₂-alkyl or C₁-C₂-alkoxy, or represent benzyl which is optionally substituted once or twice in an identical or different manner by fluorine, chlorine, bromine, C₁-C₂-alkyl, C₁-C₂-halogenoalkyl or C₁-C₂-alkoxy, or together represent a C₄-C₆-alkylene radical, in which a methylene group can be replaced by oxygen or sulphur,
and the enantiomerically pure forms of compounds of the formula (I).

6. Process for the preparation of compounds of the formula (I) according to Claim 1, **characterized in that**
(A)
to prepare compounds of the formula (Ia)
in which 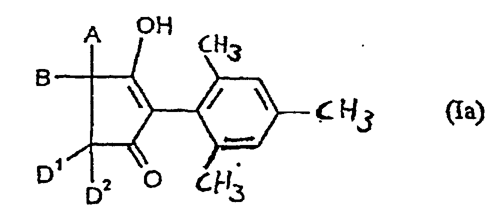
A, B, D¹, D² have the meaning given in Claim 1,
ketocarboxylic acid esters of the formula (II) 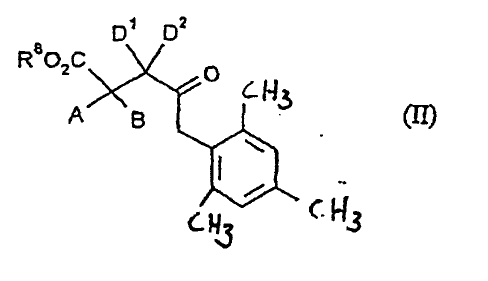 in which
A, B, D¹, D² have the abovementioned meaning
and
R⁸ represents alkyl,
are subjected to intermolecular cyclization, if appropriate in the presence of a diluent and in the presence of a base, and
(B)
to prepare compounds of the formula (Ib) 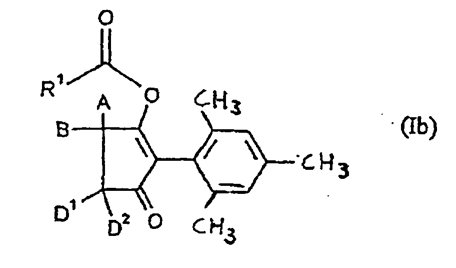 in which
A, B, D¹, D², R¹ have the meaning given in Claim 1,
compounds of the formula (Ia) 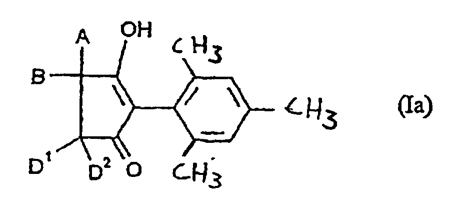 in which
A, B, D¹, D² have the abovementioned meaning,
α) are reacted with acid halides of the formula (III) 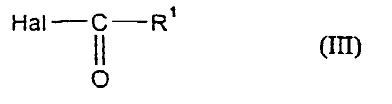 in which
R¹ has the abovementioned meaning
and
Hal represents halogen,
if appropriate in the presence of a diluent and if appropriate in the presence of an acid-binding agent,
or
β) are reacted with carboxylic acid anhydrides of the formula (IV)
R¹-CO-O-CO-R¹ (IV)
in which
R¹ has the abovementioned meaning,
if appropriate in the presence ofa diluent and if appropriate in the presence of an acid-binding agent,
and
(C)
to prepare compounds of the formula (Ic) 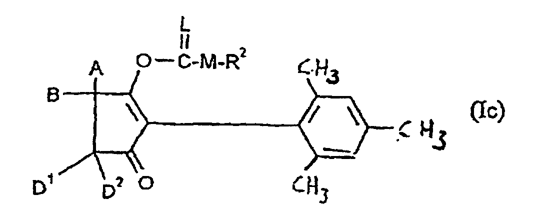 in which
A, B, D¹, D², R² have the meaning given in Claim 1,
L represents oxygen and
M represents oxygen or sulphur,
compounds of the formula (Ia) 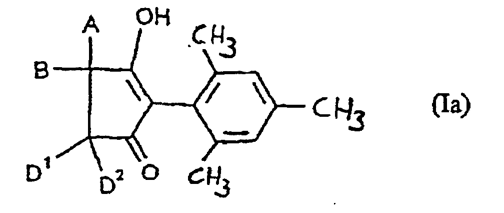 in which
A, B, D¹, D² have the abovementioned meaning,
are reacted with a chloroformic acid ester or chloroformic acid thiol ester of the formula (V)
R²-M-CO-Cl (V)
in which
R² and M have the abovementioned meaning,
if appropriate in the presence of a diluent and if appropriate in the presence of an acid-binding agent, and
(D)
to prepare compounds of the formula (Ic) 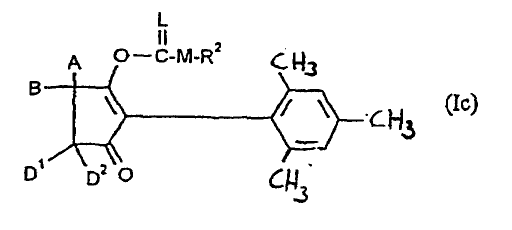 in which
A, B, D¹, D², R² have the abovementioned meaning,
L represents sulphur and
M represents oxygen or sulphur,
compounds of the formula (Ia) 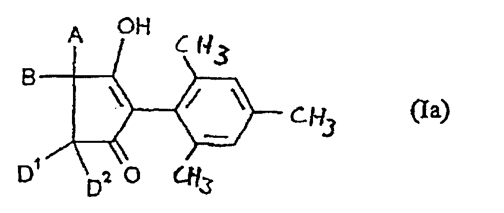 in which
A, B, D¹, D² have the abovementioned meaning,
α) are reacted with a chloromonothioformic acid ester or chlorodithioformic acid ester of the formula (VI) 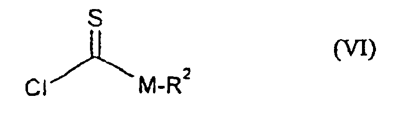 in which
M and R² have the abovementioned meaning,
if appropriate in the presence of a diluent and if appropriate in the presence of an acid-binding agent, or
β) are reacted with carbon disulphide and then with alkyl halides of the formula (VII)
R²-Hal (VII)
in which
R² has the abovementioned meaning
and
Hal represents chlorine, bromine or iodine,
if appropriate in the presence of a diluent and if appropriate in the presence of a base, and
(E)
to prepare compounds of the formula (Id) 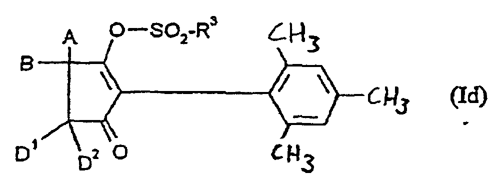 in which
A, B, D¹, D², R³ have the meaning given in Claim 1,
compounds of the formula (Ia) 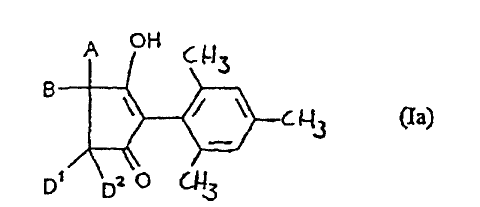 in which
A, B, D¹, D² have the abovementioned meaning,
are reacted with sulphonic acid chlorides of the formula (VIII)
R³-SO₂-Cl (VIII)
in which
R³ has the abovementioned meaning,
if appropriate in the presence of a diluent and if appropriate inthe presence of an acid-binding agent, and
(F)
to prepare compounds of the formula (Ie) 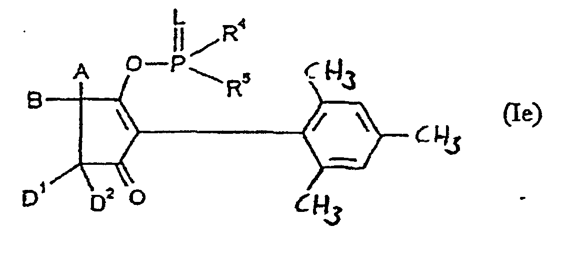 in which
A, B, D¹, D², L, R⁴, R⁵ have the meaning given in Claim 1,
compounds of the formula (Ia) 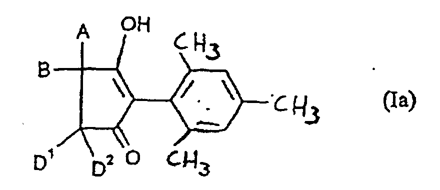 in which
A, B, D¹, D² have the abovementioned meaning,
are reacted with phosphorus compounds of the formula (IX) 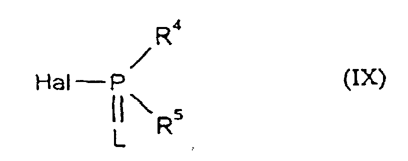 in which
L,R⁴ and R⁵ have the abovementioned meaning and
Hal represents halogen,
if appropriate in the presence ofa diluent and if appropriate in the presence ofan acid-binding agent, and
(G)
to prepare compounds of the formula (If) 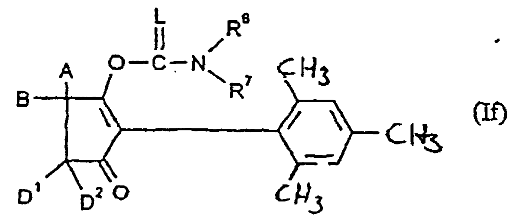 in which
A, B, D¹, D², L, R⁶, R⁷ have the meaning given in Claim 1,
compounds of the formula (Ia) 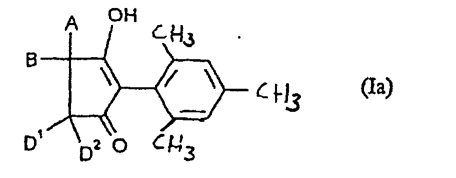 in which
A, B, D¹, D² have the abovementioned meaning,
α) are reacted with isocyanates or isothiocyanates of the formula (X)
R⁶-N=C=L (X)
in which
R⁶ and L have the abovementioned meaning,
if appropriate in the presence of a diluent and if appropriate in the presence of a catalyst, or
β) are reacted with carbamic acid chlorides or thiocarbamic acid chlorides of the formula (XI) 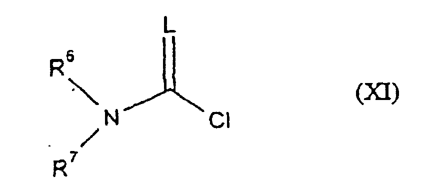
in which
L, R⁶ and R⁷ have the abovementioned meaning,
if appropriate in the presence of a diluent and if appropriate in the presence of an acid-binding agent, and
(H)
to prepare compounds of the formula (Ig) 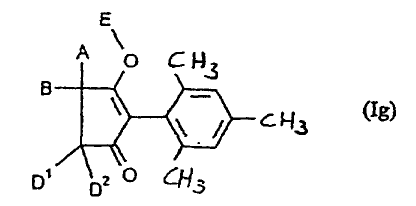 in which
D¹, D², A, B have the abovementioned meaning
and
E represents a metal ion equivalent or an ammonium ion,
compounds of the formula (Ia) 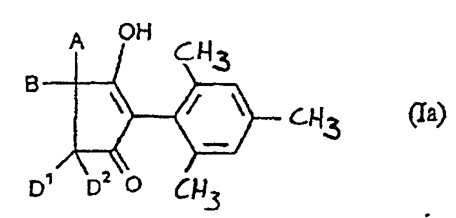 in which
A, B, D¹, D² have the abovementioned meaning,
are reacted with metal compounds of the formula (XII) or amines of the formula (XIII)
MeR⁹ₜ (XII) 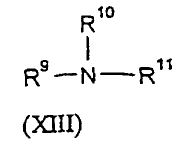
in which
Me represents mono- or divalent metal ions and
t represents the number 1 or 2 and
R⁹, R¹⁰ and R¹¹ independently of one another represent hydrogen, alkyl, alkoxy or hydroxyl,
if appropriate in the presence of a diluent.

7. Compounds of the formula (II) 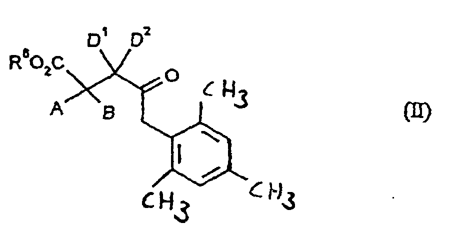 in which
A, B, D¹, D² have the meaning given in Claim 1 and
R⁸ represents alkyl.

8. Process for the preparation of compounds of the formula (II) according to Claim 7, **characterized in that** compounds of the formula (XIV) 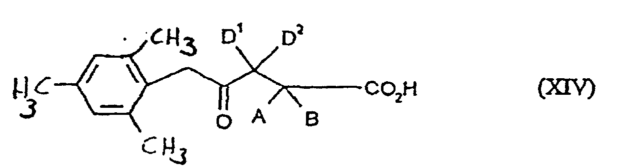 in which
A, B, D¹, D² have the meaning given in Claim 1,
are esterified.

9. Compounds of the formula (XIV) 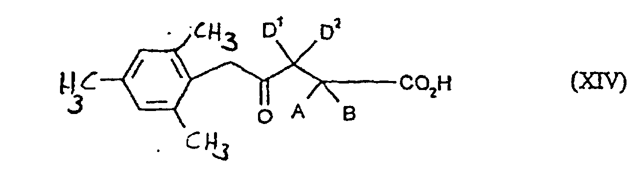 in which
A, B, D¹, D² have the meaning given in Claim 1.

10. Process for the preparation of compounds ofthe formula (XIV) according to Claim 9, **characterized in that** either carboxylic acid anhydrides of the formula (XV) 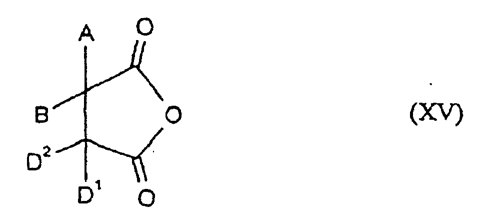 in which
A, B and D have the abovementioned meaning,
are reacted with organometallic compounds of the formula (XVI) 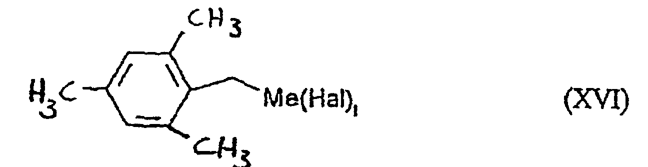 in which
Me represents mono- or divalent metal ions,
Hal represents chlorine or bromine
and
l represents the number 0 or 1,
in the presence of a diluent,
or
compounds of the formula (XVII) 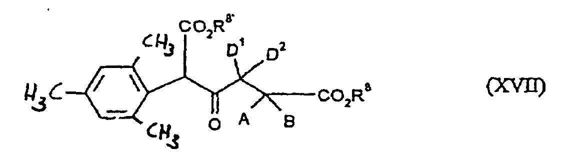 in which
A, B, D¹, D² have the abovementioned meaning and
R⁸ and R^{8'} represent alkyl,
are decarboxylated, if appropriate in the presence of a diluent and if appropriate in the presence of a base or acid.

11. Compositions for controlling pests, andherbicides, **characterized in that** they comprise at least one compound of the formula (I) according to Claim 1.

12. Use of compounds ofthe formula (I) according to Claim 1 for controlling animal pests and unwanted plant growth.

13. Method of controlling animal pests and unwanted vegetation, **characterized in that** compounds of the formula (I) according to Claim 1 are applied to the pests, plants and/or their environment.

14. Process for the preparation of compositions for controlling pests, and herbicides, **characterized in that** compounds ofthe formula (I) according to Claim 1 are mixed with extenders and/or surface-active substances.

15. Use of compounds of the formula (I) according to Claim 1 for the preparation of compositions for controlling pests, and herbicides.

## Revendications

1. Composés de formule (I) 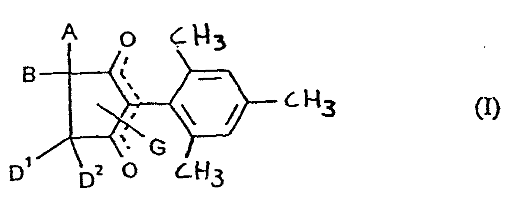 dans laquelle
A et B représentent, indépendamment l'un de l'autre, un reste alkyle éventuellement substitué dans chaque cas une ou plusieurs fois identiques ou différentes par un halogène,
D¹ et D² représentent indépendamment l'un de l'autre l'hydrogène, un halogène, un reste phényle ou alkyle éventuellement substitué par un halogène,
G représente l'hydrogène (a) ou l'un des groupes
-CO-R¹ (b) 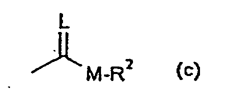
-SO₂-R³ (d) 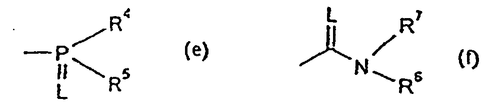
ou
E (g)
E est un équivalent d'ion métallique ou un ion ammonium,
L représente l'oxygène ou le soufre,
M représente l'oxygène ou le soufre,
R¹ représente un reste alkyle, alcényle, alkoxyalkyle, alkylthioalkyle, polyalkoxyalkyle dont chacun est éventuellement substitué une ou plusieurs fois identiques ou différentes par un halogène, ou un reste cycloalkyle saturé ou non saturé, éventuellement substitué, qui peut être interrompu par au moins un hétéroatome, ou un reste phényle, phénylalkyle, hétaryle, phénoxyalkyle ou hétaryloxyalkyle dont chacun est éventuellement substitué,
R² est un reste alkyle, alcényle, alkoxyalkyle, polyalkoxyalkyle dont chacun est éventuellement substitué une ou plusieurs fois identiques ou différentes par un halogène, ou un reste cycloalkyle, phényle ou benzyle dont chacun est éventuellement substitué,
R³ est un reste alkyle éventuellement substitué dans chaque cas une ou plusieurs fois identiques ou différentes par un halogène, ou un reste phényle ou phénylalkyle dont chacun est éventuellement substitué,
R⁴ et R⁵ représentent indépendamment l'un de l'autre un reste alkyle, alkoxy, alkylamino, alcénylamino, dialkylamino, dialcénylamino, alkylthio, alcénylthio, alcynylthio, cycloalkylthio dont chacun est éventuellement substitué une ou plusieurs fois identiques ou différentes par un halogène, ou un reste phényle, phénoxy ou phénylthio dont chacun est éventuellement substitué,
R⁶ et R⁷ désignent indépendamment l'un de l'autre l'hydrogène, un reste alkyle, alcényle, alkoxy, alkoxyalkyle dont chacun. est éventuellement substitué une ou plusieurs fois identiques ou différentes par un halogène, un reste phényle éventuellement substitué ou un reste benzyle éventuellement substitué,
ou bien
R⁶ et R⁷ forment ensemble un reste alkylène éventuellement interrompu par de l'oxygène ou du soufre,
ainsi que les formes énantiomériquement pures de composés de formule (I).

2. Composés de formule (I) suivant la revendication 1, qui possèdent, en incorporant les diverses définitions (a), (b), (c), (d), (e), (f) et (g) du groupe G, les structures (Ia) à (Ig) suivantes : 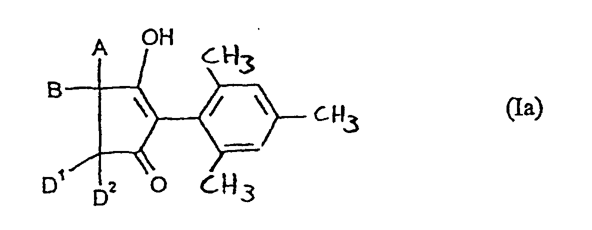 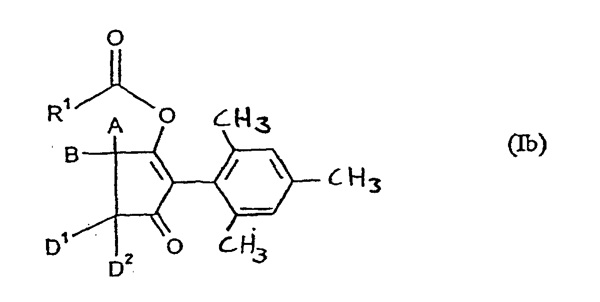 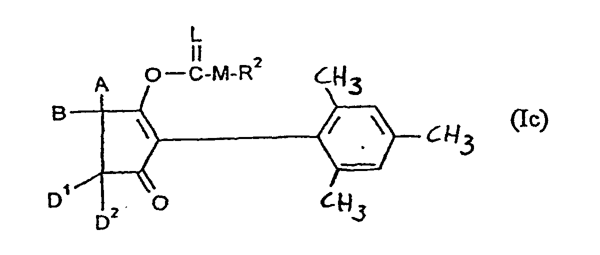 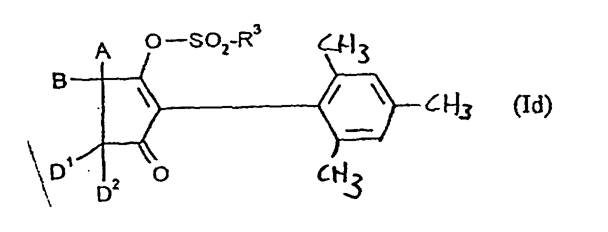 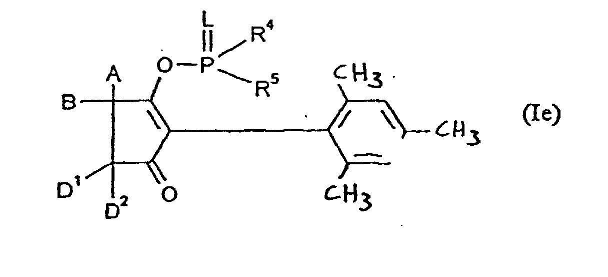 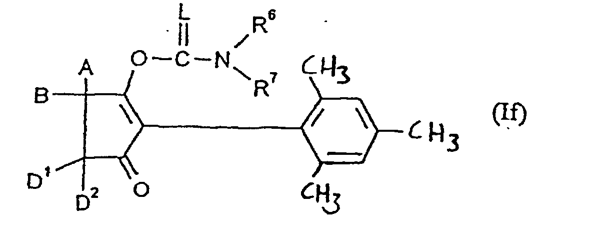 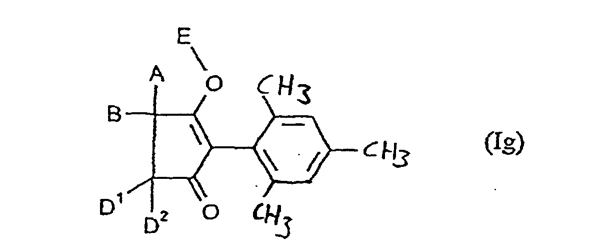 où
A, B, D¹, D², E, L, M, R¹, R², R³, R⁴, R⁵, R⁶ et R⁷ ont les définitions indiquées dans la revendication 1.

3. Composés de formule (I) suivant la revendication 1, formule dans laquelle
A et B représentent, indépendamment l'un de l'autre, un reste alkyle en C₁ à C₁₂ éventuellement substitué dans chaque cas une ou plusieurs fois identiques ou différentes par un halogène,
D¹ et D² représentent, indépendamment l'un de l'autre, l'hydrogène, un halogène, un reste alkyle en C₁ à C₆ éventuellement substitué une ou plusieurs fois identiques ou différentes par un halogène, ou un reste phényle éventuellement substitué une ou plusieurs fois identiques ou différentes par un halogène, un radical alkyle en C₁ à C₆, alkoxy en C₁ à C₆, nitro, halogénalkyle en C₁ à C₆ ou halogénalkoxy en C₁ à C₆,
G représente l'hydrogène (a) ou l'un des groupes
-CO-R¹ (b) 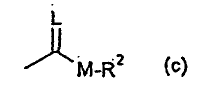
-SO₂-R³ (d) 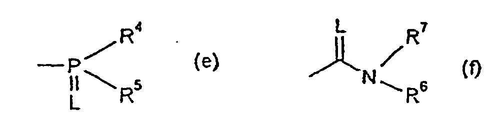
ou
E (g)
dans lesquels
E est un équivalent d'ion métallique ou un ion ammonium,
L représente l'oxygène ou le soufre,
M représente l'oxygène ou le soufre,
R¹ représente un reste alkyle en C₁ à C₂₀, alcényle en C₂ à C₂₀, (alkoxy en C₁ à C₈)-(alkyle en C₁ à C₈), (alkylthio en C₁ à C₈)-(alkyle en C₁ à C₈), poly(alkoxy en C₁ à C₈)-(alkyle en C₂ à C₈) dont chacun est éventuellement substitué une ou plusieurs fois identiques ou différentes par un halogène, ou un reste cycloalkyle à noyau de 3 à 8 atomes, saturé ou non saturé, éventuellement substitué une ou plusieurs fois identiques ou différentes par un halogène, un radical alkyle en C₁ à C₆ ou alkoxy en C₁ à C₆ et dont au moins un groupe méthylène peut être remplacé par un atome d'oxygène et/ou de soufre,
un reste phényle éventuellement substitué une ou plusieurs fois identiques ou différentes par un halogène, un radical nitro, cyano, alkyle en C₁ à C₆, alkoxy en C₁ à C₆, halogénalkyle en C₁ à C₆ ou halogénalkoxy en C₁ à C₆,
un reste phényl(alkyle en C₁ à C₆) éventuellement substitué une ou plusieurs fois identiques ou différentes par un halogène, un radical alkyle en C₁ à C₆, alkoxy en C₁ à C₆, halogénalkyle en C₁ à C₆ ou halogénalkoxy en Ci à C₆,
un reste hétaryle pentagonal ou hexagonal éventuellement substitué une ou plusieurs fois identiques ou différentes par un halogène ou un radical alkyle en C₁ à C₆,
un reste phénoxy(alkyle en C₁ à C₆) éventuellement substitué une ou plusieurs fois identiques ou différentes par un halogène ou un radical alkyle en C₁ à C₆,
ou bien
un reste hétaryloxy(alkyle en C₁ à C₆) pentagonal ou hexagonal éventuellement substitué une ou plusieurs fois identiques ou différentes par un halogène, un radical amino ou alkyle en C₁ à C₆,
R² représente un reste alkyle en C₁ à C₂₀, alcényle en C₂ à C₂₀, (alkoxy en C₁ à C₈)-(alkyle en C₂ à C₈) ou poly(alkoxy en C₁ à C₈)-(alkyle en C₂ à C₈) dont chacun est éventuellement substitué une ou plusieurs fois identiques ou différentes par un halogène, ou un reste cycloalkyle en C₃ à C₈ éventuellement substitué une ou plusieurs fois identiques ou différentes par un halogène, un radical alkyle en C₁ à C₆ ou alkoxy en C₁ à C₆, ou bien
un reste phényle ou benzyle dont chacun est éventuellement substitué une ou plusieurs fois identiques ou différentes par un halogène, un radical nitro, cyano, alkyle en C₁ à C₆, alkoxy en C₁ à C₆ ou halogénalkyle en C₁ à C₆,
R³ est un reste alkyle en C₁ à C₁₂ éventuellement substitué dans chaque cas une ou plusieurs fois identiques ou différentes par un halogène, ou un reste phényle ou phényl (alkyle en C₁ à C₄) dont chacun est éventuellement substitué une ou plusieurs fois identiques ou différentes par un halogène, un radical alkyle en C₁ à C₆, alkoxy en C₁ à C₆, halogénalkyle en C₁ à C₃, halogénalkoxy en C₁ à C₃, cyano ou nitro,
R⁴ et R⁵ représentent indépendamment l'un de l'autre un reste alkyle en C₁ à C₈, alkoxy en C₁ à C₈, alkylamino en C₁ à C₈, alcénylamino en C₃ à C₈, di (alkyle en C₁ à C₈)amino, di(alcényle en C₃ à C₈)amino, alkylthio en C₁ à C₈, alcénylthio en C₃ à C₅, alcynylthio en C₃ à C₅, cycloalkylthio en C₃ à C₇ dont chacun est éventuellement substitué une ou plusieurs fois identiques ou différentes par un halogène, ou un reste phényle, phénoxy ou phénylthio dont chacun est éventuellement substitué une ou plusieurs fois identiques ou différentes par un halogène, un radical nitro, cyano, alkoxy en C₁ à C₄, halogénalkoxy en C₁ à C₄, alkylthio en C₁ à C₄, halogénalkylthio en C₁ à C₄, alkyle en C₁ à C₄ ou halogénalkyle en C₁ à C₄, et
R⁶ et R⁷ représentent indépendamment l'un de l'autre l'hydrogène ou un reste alkyle en C₁ à C₁₀, alkoxy en C₁ à C₁₀, alcényle en C₃ à C₈ ou (alkoxy en C₁ à C₁₀)-(alkyle en C₁ à C₁₀) dont chacun est éventuellement substitué une ou plusieurs fois identiques ou différentes par un halogène, un reste phényle éventuellement substitué une ou plusieurs fois identiques ou différentes par un halogène, un radical halogénalkyle en C₁ à C₃, alkyle en Ci à C₈ ou alkoxy en C₁ à C₈, un reste benzyle éventuellement substitué une ou plusieurs fois identiques ou différentes par un halogène, un radical alkyle en C₁ à C₈, halogénalkyle en C₁ à C₈ ou alkoxy en C₁ à C₈, ou forment ensemble un reste alkylène en C₂ à C₆ dans lequel un groupe méthylène peut être remplacé par de l'oxygène ou du soufre,
ainsi que les formes énantiomériquement pures de composés de formule (I).

4. Composés de formule (I) suivant la revendication 1, formule dans laquelle
A et B représentent, indépendamment l'un de l'autre, un reste alkyle en C₁ à C₁₀ éventuellement substitué,dans chaque cas une à cinq fois identiques ou différentes par du fluor ou du chlore,
D¹ et D² représentent indépendamment l'un de l'autre l'hydrogène, le fluor, le chlore, un reste alkyle en C₁ à C₄ éventuellement substitué une à cinq fois identiques ou différentes par du fluor ou du. chlore, ou un reste phényle éventuellement substitué une à trois fois identiques ou différentes par du fluor, du chlore, du brome, un radical alkyle en C₁ à C₄, alkoxy en C₁ à C₄, nitro, halogénalkyle en C₁ ou C₂ ou halogénalkoxy en C₁ ou C₂,
G représente l'hydrogène (a) ou l'un des groupes
-CO-R¹ (b) 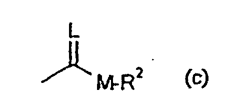
-SO₂-R³ (d) 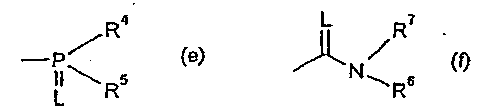
ou
E (g)
dans lesquels
E est un équivalent d'ion métallique ou un ion ammonium,
L représente l'oxygène ou le soufre,
M représente l'oxygène ou le soufre,
R¹ représente un reste alkyle en C₁ à C₁₆, alcényle en C₂ à C₁₆, (alkoxy en C₁ à C₆) - (alkyle en C₁ à C₆), (alkylthio en C₁ à C₆) - (alkyle en C₁ à C₆), poly(alkoxy en C₁ à C₆)-(alkyle en C₂ à C₆) dont chacun est éventuellement substitué une à neuf fois identiques ou différentes par un halogène, ou un reste cycloalkyle à noyau de 3 à 7 atomes, saturé ou non saturé, éventuellement substitué une à six fois identiques ou différentes par du fluor, du chlore, un radical alkyle en C₁ à C₄ ou alkoxy en C₁ à C₄ et dans lequel un ou deux groupes méthylène sont remplacés par des atomes d'oxygène et/ou de soufre,
un reste phényle éventuellement substitué une à trois fois identiques ou différentes par du fluor, du chlore, du brome, un radical nitro, cyano, alkyle en C₁ à C₄, alkoxy en C₁ à C₄, halogénalkyle en C₁ à C₃ ou halogénalkoxy en C₁ à C₃,
un reste phényl(alkyle en C₁ à C₄) éventuellement substitué une à trois fois identiques ou différentes par du fluor, du chlore, du brome, un radical alkyle en C₁ à C₄, alkoxy en C₁ à C₄, halogénalkyle en C₁ à C₃ ou halogénalkoxy en C₁ à C₃,
un reste pyridyle, thiényle, furannyle, pyrimidyle, thiazolyle ou pyrazolyle dont chacun est éventuellement substitué une ou deux fois identiques ou différentes par du fluor, du chlore, ou un radical alkyle en C₁ à C₆,
un reste phénoxy(alkyle en C₁ à C₅) éventuellement substitué une à trois fois identiques ou différentes par du fluor, du chlore, du brome ou un radical alkyle en Ci à C₄, ou bien
un reste pyridyloxy-, pyrimidyloxy- ou thiazolyloxy-(alkyle en C₁ à C₅) dont chacun est éventuellement substitué une ou deux fois identiques ou différentes par du fluor, du chlore, du brome, un radical amino ou alkyle en C₁ à C₄,
R² représente un reste alkyle en C₁ à C₁₆, alcényle en C₂ à C₁₆, (alkoxy en C₁ à C₆)-(alkyle en C₂ à C₆) ou poly(alkoxy en C₁ à C₆)-(alkyle en C₂ à C₆) dont chacun est éventuellement substitué une à neuf fois identiques ou différentes par du fluor ou du chlore, ou bien un reste cycloalkyle en C₃ à C₇ éventuellement substitué une à cinq fois identiques ou différentes par du fluor, du chlore, un radical alkyle en C₁ à C₄ ou alkoxy en C₁ à C₄, ou bien
un reste phényle ou benzyle éventuellement substitué une à trois fois identiques ou différentes par du fluor, du chlore, du brome, un radical nitro, cyano, alkyle en C₁ à C₄, alkoxy en C₁ à C₃ ou halogénalkyle en C₁ à C₃,
R³ est un reste alkyle en C₁ à C₈ éventuellement substitué dans chaque cas une à cinq fois identiques ou différentes par du fluor ou du chlore, ou un reste phényle ou phényl(alkyle en C₁ ou C₂) dont chacun est éventuellement substitué une à trois fois identiques ou différentes par du fluor, du chlore, du brome, un radical alkyle en C₁ à C₄, alkoxy en C₁ à C₄, halogénalkyle en C₁ ou C₂, halogénalkoxy en C₁ ou C₂, cyano ou nitro,
R⁴ et R⁵ représentent indépendamment l'un de l'autre un reste alkyle en C₁ à C₆, alkoxy en C₁ à C₆, alkylamino en C₁ à C₆, alcénylamino en C₃ à C₆, di (alkyle en C₁ à C₆)amino, di(alcényle en C₃ à C₆)amino, alkylthio en C₁ à C₆, alcénylthio en C₃ ou C₄, alcynylthio en C₃ ou C₄, cycloalkylthio en C₃ à C₆ dont chacun est éventuellement substitué une à cinq fois identiques ou différentes par du fluor ou du chlore, ou un reste phényle, phénoxy ou phénylthio dont chacun est éventuellement substitué une à trois fois identiques ou différentes par du fluor, du chlore, du brome, un radical nitro, cyano, alkoxy en C₁ à C₃, halogénalkoxy en C₁ à C₃, alkylthio en C₁ à C₃, halogénalkylthio en C₁ à C₃, alkyle en C₁ à C₃ ou halogénalkyle en C₁ à C₃, et
R⁶ et R⁷ représentent indépendamment l'un de l'autre l'hydrogène ou un reste alkyle en C₁ à C₆, alkoxy en C₁ à C₆, alcényle en C₃ à C₆ ou (alkoxy en C₁ à C₆)-(alkyle en C₁ à C₆) dont chacun est éventuellement substitué une à cinq fois identiques ou différentes par du fluor ou du chlore, un reste phényle éventuellement substitué une à trois identiques ou différentes par du fluor, du chlore, un radical halogénalkyle en C₁ à C₃, alkyle en C₁ à C₃ ou alkoxy en C₁ à C₃, un reste benzyle éventuellement substitué une à cinq fois identiques ou différentes par du fluor, du chlore, un radical alkyle en C₁ à C₃, halogénalkyle en C₁ à C₃ ou alkoxy en C₁ à C₃, ou forment ensemble un reste alkylène en C₄ à C₆ dans lequel un groupe méthylène peut être remplacé par de l'oxygène ou du soufre,
ainsi que les formes énantiomériquement pures de composés de formule (I).

5. Composés de formule (I) suivant la revendication 1, formule dans laquelle
A et B représentent, indépendamment l'un de l'autre, un reste alkyle en C₁ à C₈ éventuellement substitué dans chaque cas une à trois fois identiques ou différentes par du fluor ou du chlore,
D¹ et D² représentent indépendamment l'un de l'autre l'hydrogène, le fluor, le chlore, un reste méthyle, éthyle ou un reste phényle éventuellement substitué une ou deux fois identiques ou différentes par du fluor, du chlore, un radical méthyle, méthoxy ou trifluorométhyle,
G représente l'hydrogène (a) ou l'un des groupes
-CO-R¹ (b) 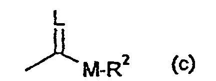
-SO₂-R³ (d) 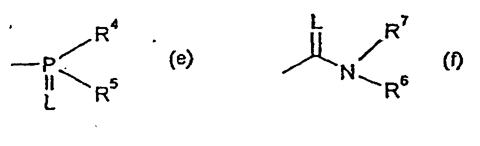
ou
E (g)
dans lesquels
E est un équivalent d'ion métallique ou un ion ammonium,
L représente l'oxygène ou le soufre,
M représente l'oxygène ou le soufre,
R¹ est un reste alkyle en C₁ à C₁₄, alcényle en C₂ à C₁₄, (alkoxy en C₁ à C₄)-(alkyle en C₁ à C₆), (alkylthio en C₁ à C₄)-(alkyle en C₁ à C₆), poly(alkoxy en C₁ à C₄)-(alkyle en C₂ à C₄) dont chacun est éventuellement substitué une à trois fois identiques ou différentes par du fluor ou du chlore, ou un reste cycloalkyle à noyau de 3 à 6 atomes éventuellement substitué une ou deux fois identiques ou différentes par du fluor, du chlore, un radical méthyle, éthyle, méthoxy ou éthoxy, dans lequel le cas échéant un ou deux groupes méthylène peuvent être remplacés par des atomes d'oxygène et/ou de soufre,
un reste phényle éventuellement substitué une ou deux fois identiques ou différentes par du fluor, du chlore, du brome, un radical méthyle, éthyle, propyle, isopropyle, méthoxy, éthoxy, trifluorométhyle, trifluorométhoxy, cyano ou nitro,
un reste phényl(alkyle en C₁ à C₃), en particulier benzyle, éventuellement substitué une ou deux fois identiques ou différentes par du fluor, du chlore, du brome, un radical méthyle, éthyle, propyle, isopropyle, méthoxy, éthoxy, trifluorométhyle ou trifluorométhoxy,
un reste pyridyle, thiényle ou furranyle dont chacun est éventuellement substitué une ou deux fois identiques ou différentes par du fluor, du chlore, du brome, un radical méthyle ou éthyle,
un reste phénoxy(alkyle en C₁ à C₄) éventuellement substitué une ou deux fois identiques ou différentes par du fluor, du chlore, un radical méthyle ou éthyle,
ou bien
un reste pyridyloxy(alkyle en C₁ à C₃), pyrimidyloxy(alkyle en C₁ à C₃) ou thiazolyloxy(alkyle en C₁ à C₃) dont chacun est éventuellement substitué une ou deux fois identiques ou différentes par du fluor, du chlore, un radical amino, méthyle ou éthyle,
R² est un reste alkyle en C₁ à C₁₄, alcényle en C₂ à C₁₄, (alkoxy en C₁ à C₄)-(alkyle en C₂ à C₆) ou poly(alkoxy en C₁ à C₄)-(alkyle en C₂ à C₆) dont chacun est éventuellement substitué une à cinq fois identiques ou différentes par du fluor ou du chlore, un reste cycloalkyle en C₃ à C₆ éventuellement substitué une à trois fois identiques ou différentes par du fluor, du chlore, un radical méthyle, éthyle, méthoxy ou éthoxy, où bien un reste phényle ou benzyle dont chacun est éventuellement substitué une ou deux fois identiques ou différentes par du fluor, du chlore, un radical nitro, cyano, méthyle, éthyle, propyle, isopropyle, méthoxy, éthoxy ou trifluorométhyle,
R³ est un reste alkyle en C₁ à C₆ éventuellement substitué une à trois fois identiques ou différentes par du fluor ou du chlore ou un reste phényle ou benzyle dont chacun est éventuellement substitué une ou deux fois identiques ou différentes par du fluor, du chlore, du brome, un radical méthyle, méthoxy, trifluorométhyle, trifluorométhoxy, cyano ou nitro,
R⁴ et R⁵ représentent indépendamment l'un de l'autre un reste alkyle en C₁ à C₄, alkoxy en C₁ à C₄, alkylamino en C₁ à C₄, alcénylamino en C₃ ou C₄, di (alkyle en C₁ à C₄)amino, di(alcényle en C₃ ou C₄)amino, alkylthio en C₁ à C₄ dont chacun est éventuellement substitué une à trois fois identiques ou différentes par du fluor ou du chlore, un reste phényle, phénoxy ou phénylthio dont chacun est éventuellement substitué une ou deux fois identiques ou différentes par du fluor, du chlore, du brome, un radical nitro, cyano, alkoxy en C₁ ou C₂, fluoralkoxy en C₁ ou C₂, chloralkoxy en C₁ ou C₂, alkylthio en C₁ ou C₂, fluoralkylthio en C₁ ou C₂, chloralkylthio en C₁ ou C₂ ou alkyle en C₁ à C₃ et
R⁶ et R⁷ représentent indépendamment l'un de l'autre l'hydrogène ou un reste alkyle en C₁ à C₄, alkoxy en C₁ à C₄ ou (alkoxy en C₁ à C₄)-(alkyle en C₁ à C₄) dont chacun est éventuellement substitué une à trois fois identiques ou différentes par du fluor ou du chlore, un reste phényle éventuellement substitué une ou deux fois identiques ou différentes par du fluor, du chlore, un radical halogénalkyle en C₁ ou C₂, alkyle en C₁ ou C₂ ou alkoxy en C₁ ou C₂, un reste benzyle éventuellement substitué une ou deux fois identiques ou différentes par du fluor, du chlore, du brome, un radical alkyle en C₁ ou C₂, halogénalkyle en C₁ ou C₂ ou alkoxy en C₁ ou C₂, ou forment ensemble un reste alkylène en C₄ à C₆ dans lequel un groupe méthylène peut être remplacé par de l'oxygène ou du soufre,
ainsi que les formes énantiomériquement pures de composés de formule (I).

6. Procédé de production de composés de formule (I) suivant la revendication 1, **caractérisé en ce que**
(A)
pour la production de composés de formule (Ia) 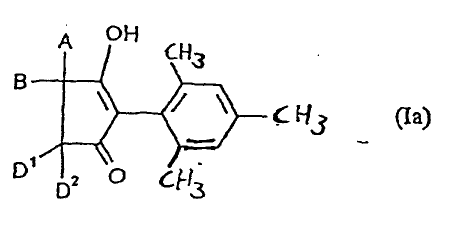 dans laquelle
A, B, D¹, D² ont la définition indiquée dans la revendication 1,
on effectue la cyclisation intramoléculaire d'esters d'acide cétocarboxylique de formule (II) 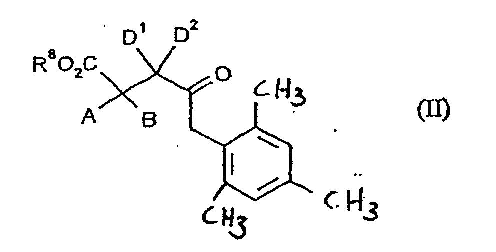 dans laquelle
A, B, D¹, D² ont la définition indiquée ci-dessus
et
R⁸ est un reste alkyle,
éventuellement en présence d'un diluant et, le cas échéant, en présence d'une base et
(B)
pour la production de composés de formule (Ib) 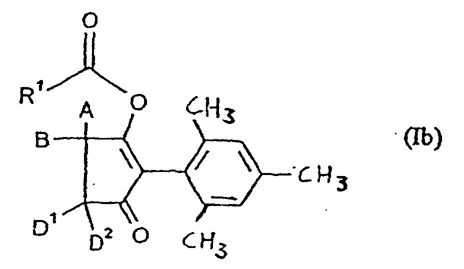 dans laquelle
A, B, D¹, D², R¹ ont la définition indiquée dans la revendication 1,
on fait réagir des composés de formule (Ia) 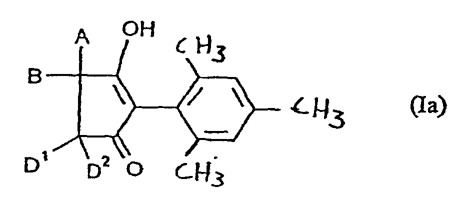 dans laquelle
A, B, D¹, D² ont la définition indiquée ci-dessus,
α) avec des halogénures d'acide de formule (III) 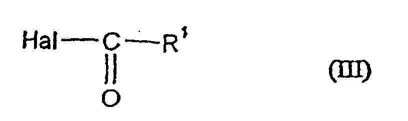 dans laquelle
R¹ a la définition indiquée ci-dessus,
et
Hal représente un halogène,
éventuellement en présence d'un diluant et, le cas échéant, en présence d'un accepteur d'acide,
ou bien
β) avec des anhydrides d'acide carboxylique de formule (IV)
R¹-CO-O-CO-R¹ (IV)
dans laquelle
R¹ a la définition indiquée ci-dessus,
éventuellement en présence d'un diluant et, le cas échéant, en présence d'un accepteur d'acide,
et
(C)
pour la production de composés de formule (Ic) 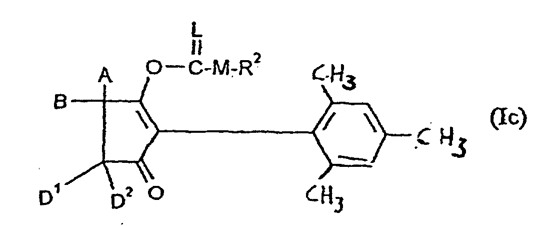 dans laquelle
A, B, D¹, D², R² ont la définition indiquée dans la revendication 1,
L représente l'oxygène et
M représente l'oxygène ou le soufre,
on fait réagir des composés de formule (Ia) 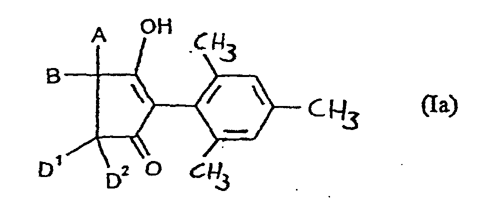 dans laquelle
A, B, D¹, D² ont la définition indiquée ci-dessus,
avec un ester d'acide chloroformique ou un thioester d'acide chloroformique de formule (V)
R²-M-CO-Cl (V)
dans laquelle
R² et M ont la définition indiquée ci-dessus,
éventuellement en présence d'un diluant et, le cas éché en présence d'un accepteur d'acide, et
(D)
pour la production de composés de formule (Ic) 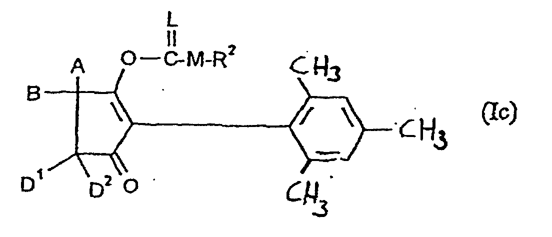 dans laquelle
A, B, D¹, D², R² ont la définition indiquée ci-dessus,
L représente le soufre et
M est l'oxygène ou le soufre,
on fait réagir des composés de formule (Ia) 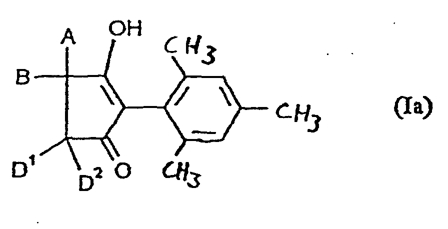 dans laquelle
A, B, D¹, D² ont la définition indiquée ci-dessus,
α) avec un ester d'acide chloromonothioformique ou un ester d'acide chlorodithioformique de formule (VI) 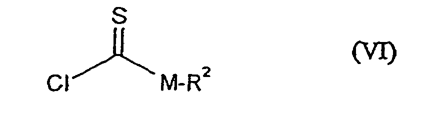 dans laquelle
M et R² ont la définition indiquée ci-dessus,
éventuellement en présence d'un diluant et, le cas échéant, en présence d'un accepteur d'acide, ou bien
β) avec le sulfure de carbone puis avec des halogénures d'alkyle de formule (VII)
R²-Hal (VII)
dans laquelle
R² a la définition indiquée ci-dessus
et
Hal représente le chlore, le brome ou l'iode,
éventuellement en présence d'un diluant et, le cas échéant, en présence d'une base, et
(E)
pour la production de composés de formule (Id) 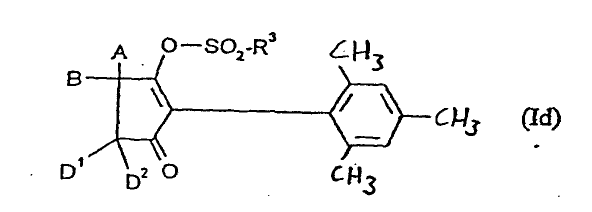 dans laquelle
A, B, D¹, D², R³ ont la définition indiquée ci-dessus,
on fait réagir des composés de formule (Ia) 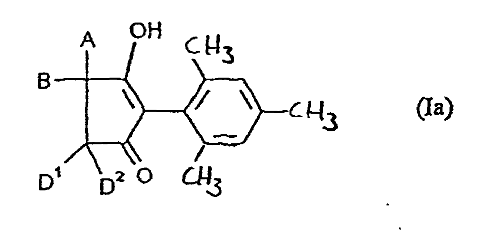 dans laquelle
A, B, D¹, D² ont la définition indiquée ci-dessus,
avec des chlorures d'acide sulfonique de formule (VIII)
R³-SO₂-Cl (VIII)
dans laquelle
R³ a la définition indiquée ci-dessus,
éventuellement en présence d'un diluant et, le cas échéant, en présence d'un accepteur d'acide; et
(F)
pour la production de composés de formule (Ie) 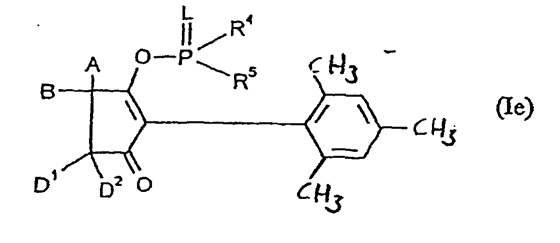 dans laquelle
A, B, D¹, D², L, R⁴, R⁵ ont la définition indiquée dans la revendication 1,
on fait réagir des composés de formule (Ia) 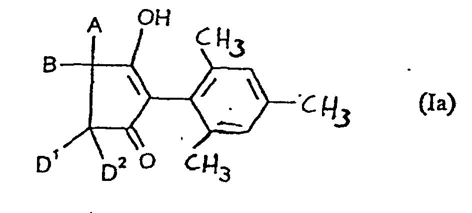 dans laquelle
A, B, D¹, D² ont la définition indiquée ci-dessus,
avec des composés de phosphore de formule (IX) 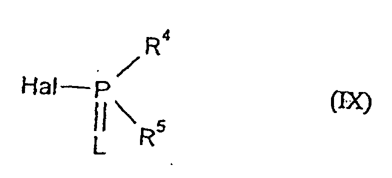 dans laquelle
L, R⁴ et R⁵ ont la définition indiquée ci-dessus, et
Hal représente un halogène,
éventuellement en présence d'un diluant et, le cas échéant, en présence d'un accepteur d'acide, et
(G)
pour la production de composés de formule (If) 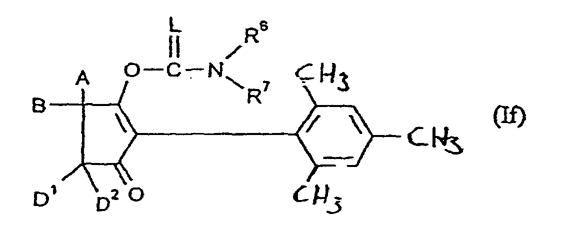 dans laquelle
A, B, D¹, D², L, R⁶, R⁷ ont la définition indiquée dans la revendication 1,
on fait réagir des composés de formule (Ia) 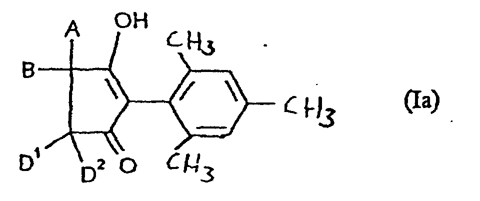 dans laquelle
A, B, D¹, D² ont la définition indiquée ci-dessus,
α) avec des isocyanates ou des isothiocyanates de formule (X)
R⁶-N=C=L (X)
dans laquelle
R⁶ et L ont la définition indiquée ci-dessus,
éventuellement en présence d'un diluant et, le cas échéant, en présence d'un catalyseur, ou bien
β) avec des chlorures d'acide carbamique ou des chlorures d'acide thiocarbamique de formule (XI) 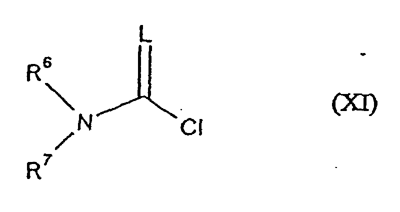 dans laquelle
L, R⁶ et R⁷ ont la définition indiquée ci-dessus,
éventuellement en présence d'un diluant et, le cas échéant, en présence d'un accepteur d'acide, et
(H)
pour la production de composés de formule (Ig) 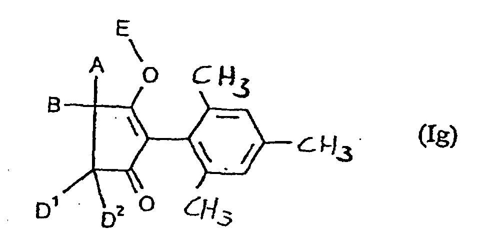
dans laquelle
D¹, D², A, B ont la définition indiquée ci-dessus,
et
E représente un équivalent d'ion métallique ou un ion ammonium,
on fait réagir des composés de formule (Ia) 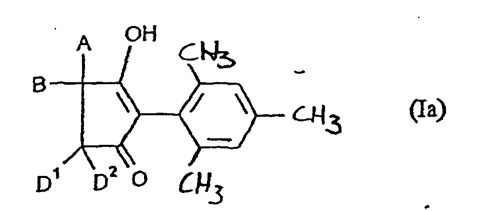 dans laquelle
A, B, D¹, D² ont la définition indiquée ci-dessus,
avec des composés métalliques de formule (XII) ou des amines de formule (XIII)
MeR⁹ₜ (XII) 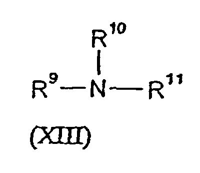
formules dans lesquelles
Me représente des ions métalliques monovalents ou divalents, et
t représente le nombre 1 ou 2, et
R⁹, R¹⁰ et R¹¹ représentent indépendamment l'un de l'autre l'hydrogène, un reste alkyle, alkoxy ou hydroxy,
éventuellement en présence d'un diluant.

7. Composés de formule (II) 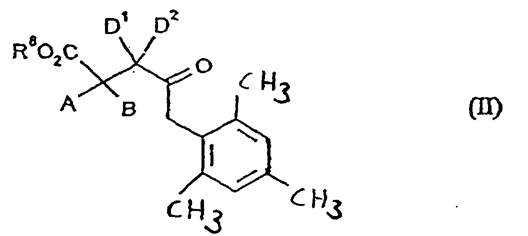 dans laquelle
A, B, D¹, D² ont la définition indiquée dans la revendication 1, et
R⁸ est un reste alkyle.

8. Procédé de production de composés de formule (II) suivant la revendication 7, **caractérisé en ce qu'**on estérifie des composés de formule (XIV) 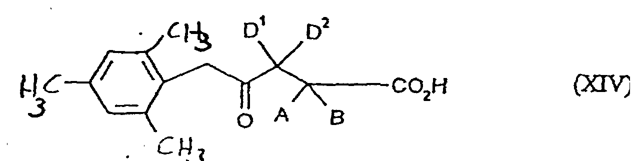 dans laquelle
A, B, D¹, D² ont la définition indiquée dans la revendication 1.

9. Composés de formule (XIV) 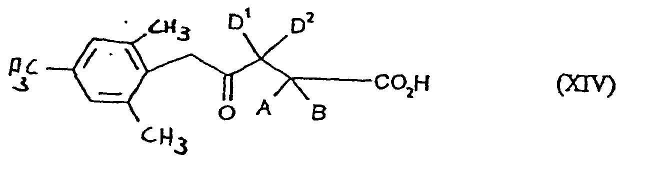 dans laquelle
A, B, D¹, D² ont la définition indiquée dans la revendication 1.

10. Procédé de production de composés de formule (XIV) suivant la revendication 9, **caractérisé en ce qu'**on fait réagir des anhydrides d'acide carboxylique de formule (XV) 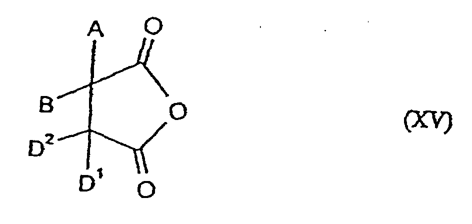 dans laquelle
A, B et D ont la définition indiquée ci-dessus,
avec des composés organométalliques de formule (XVI) 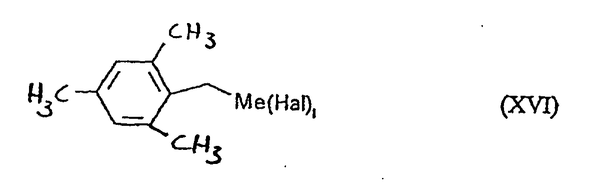 dans laquelle
Me représente des ions métalliques monovalents ou divalents,
Hal représente le chlore ou le brome,
et
l représente le nombre 0 ou 1,
en présence d'un diluant,
ou bien
on décarboxyle des composés de formule (XVII) 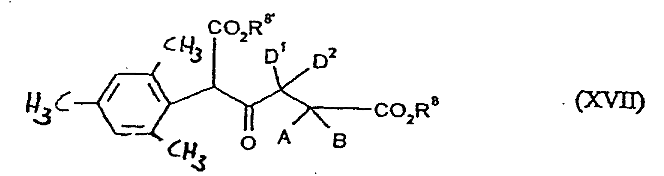 dans laquelle
A, B, D¹, D² ont la définition indiquée ci-dessus et
R⁸ et R^{8'} représente un reste alkyle,
éventuellement en présence d'un diluant et, le cas échéant, en présence d'une base ou d'un acide.

11. Pesticides et herbicides, **caractérisés par** une teneur en au moins un composé de formule (I) suivant la revendication 1.

12. Utilisation de composés de formule (I) suivant la revendication 1 pour combattre des parasites animaux et une végétation indésirable.

13. Procédé de lutte contre des parasites animaux et une végétation indésirable, **caractérisé en ce qu'**on fait agir des composés de formule (I) suivant la revendication 1 sur les parasites, les végétaux et/ou leur milieu.

14. Procédé de préparation de pesticides et d'herbicides, **caractérisé en ce qu'**on mélange des composés de formule (I) suivant la revendication 1 avec des diluants et/ou des agents tensioactifs.

15. Utilisation de composés de formule (I) suivant la revendication 1 pour la préparation de pesticides et d'herbicides.
